# EUROPEAN PATENT SPECIFICATION

(11) **EP 2 473 022 B1**
(45) Date of publication and mention of the grant of the patent: **07.12.2016**
(21) Application number: 10814446.0
(22) Date of filing: 01.09.2010
(51) Int. Cl.: C12N 9/00, C12N 15/82, A01H 5/12

(54) **HERBICIDE-TOLERANT PLANTS**
HERBIZIDTOLERANTE PFLANZEN
VÉGÉTAUX TOLÉRANT LES HERBICIDES

(30) Priority: 16.07.2010 US 365298 P; 01.09.2009 US 238906 P
(43) Date of publication of application: 11.07.2012
(73) Proprietor: BASF Agrochemical Products, B.V., 6835 EA Arnhem (NL)
(72) Inventor: MANKIN, Scots, L., Raleigh, NC 27612 (US); SCHOFL, Ulrich, Apex, NC 27523 (US); HONG, Haiping, Cary, NC 27519 (US); WENCK, Allan, R., Durham, NC 27705 (US); NEUTEBOOM, Leon, Youngsville, NC 27596 (US); WHITT, Sherry, R., Raleigh, NC 27614 (US); CARLSON, Dale, R., Apex, NC 27502 (US)
(74) Representative: Reitstötter Kinzebach
(86) International application number: PCT/US2010/047571
(87) International publication number: WO 2011/028832

(56) References cited:
- WO-A1-2005/123946
- WO-A2-2008/089061
- US-A1- 2013 023 416
- LIU WENJIE ET AL: "Single-site mutations in the carboxyltransferase domain of plastid acetyl-CoA carboxylase confer resistance to grass-specific herbicides", PROCEEDINGS OF THE NATIONAL ACADEMY OF SCIENCES OF THE UNITED STATES OF AMERICA, vol. 104, no. 9, February 2007 (2007-02), pages 3627-3632, XP002697538, ISSN: 0027-8424
- DELYE CHRISTOPHE: "Weed resistance to acetyl coenzyme A carboxylase inhibitors: an update", WEED SCIENCE, vol. 53, no. 5, September 2005 (2005-09), pages 728-746, XP002697539, ISSN: 0043-1745
- DELYE CHRISTOPHE ET AL: "Cross-resistance patterns to ACCase-inhibiting herbicides conferred by mutant ACCase isoforms in Alopecurus myosuroides Huds. (black-grass), re-examined at the recommended herbicide field rate", PEST MANAGEMENT SCIENCE, vol. 64, no. 11, November 2008 (2008-11), pages 1179-1186, XP002697540, ISSN: 1526-498X
- YU QIN ET AL: "Diversity of acetyl-coenzyme a carboxylase mutations in resistant Lolium populations: Evaluation using clethodim", PLANT PHYSIOLOGY (ROCKVILLE), vol. 145, no. 2, October 2007 (2007-10), pages 547-558, XP002697541, ISSN: 0032-0889
- DELYE C ET AL: "An isoleucine residue within the carboxyl-transferase domain of multidomain acetyl-coenzyme A carboxylase is a major determinant of sensitivity to aryloxyphenoxypropionate but not to cyclohexanedione inhibitors", PLANT PHYSIOLOGY, AMERICAN SOCIETY OF PLANT PHYSIOLOGISTS, ROCKVILLE, MD, US, vol. 132, no. 3, 1 July 2003 (2003-07-01), pages 1716-1723, XP002344970, ISSN: 0032-0889, DOI: 10.1104/PP.103.021139
- DELYE CHRISTOPHE ET AL: "Molecular bases for sensitivity to acetyl-coenzyme a carboxylase inhibitors in black-grass", PLANT PHYSIOLOGY (ROCKVILLE), vol. 137, no. 3, March 2005 (2005-03), pages 794-806, XP002688872, ISSN: 0032-0889
- DELYE CHRISTOPHE ET AL: "PCR-based detection of resistance to acetyl-CoA carboxylase-inhibiting herbicides in black-grass (Alopecurus myosuroides Huds) and ryegrass (Lolium rigidum Gaud)", PEST MANAGEMENT SCIENCE, vol. 58, no. 5, May 2002 (2002-05), pages 474-478, XP002344971, ISSN: 1526-498X
- ZAGNITKO O ET AL: "An isoleucine/leucine residue in the carboxyltransferase domain of acetyl-CoA carboxylase is critical for interaction with aryloxyphenoxypropionate and cyclohexanedione inhibitors", PROCEEDINGS OF THE NATIONAL ACADEMY OF SCIENCES, NATIONAL ACADEMY OF SCIENCES, US, vol. 98, no. 12, 5 June 2001 (2001-06-05), pages 6617-6622, XP002298286, ISSN: 0027-8424, DOI: 10.1073/PNAS.121172798
- DELYE C ET AL: "An isoleucine-leucine substitution in chloroplastic acetyl-CoA carboxylase from green foxtail (Setaria viridis L. Beauv.) is responsible for resistance to the cyclohexanedione herbicide sethoxydim", PLANTA, SPRINGER VERLAG, DE, vol. 214, no. 3, 1 January 2002 (2002-01-01), pages 421-427, XP002344972, ISSN: 0032-0935, DOI: 10.1007/S004250100633
- DELYE C ET AL: "SNP markers for black-grass (Alopecurus myosuroides Huds.) genotypes resistant to acetyl CoA-carboxylase inhibiting herbicides", THEORETICAL AND APPLIED GENETICS, SPRINGER, BERLIN, DE, vol. 104, no. 6-7, 1 May 2002 (2002-05-01) , pages 1114-1120, XP002344973, ISSN: 0040-5752, DOI: 10.1007/S00122-001-0852-6
- CHRISTOFFERS M J ET AL: "AN ISOLEUCINE TO LEUCINE MUTATION IN ACETYL-COA CARBOXYLASE CONFERS HERBICIDE RESISTANCE IN WILD OAT", GENOME, NATIONAL RESEARCH COUNCIL CANADA, OTTAWA; CA, vol. 45, no. 6, 1 January 2002 (2002-01-01), pages 1049-1056, XP001155164, ISSN: 0831-2796, DOI: 10.1139/G02-080
- MENCHARI Y ET AL: "Fitness costs associated with three mutant acetyl-coenzyme A carboxylase alleles endowing herbicide resistance in black-grass Alopecurus myosuroides", JOURNAL OF APPLIED ECOLOGY, vol. 45, no. 3, June 2008 (2008-06), pages 939-947, XP002697542, ISSN: 0021-8901
- 'Targeting of the arabidopsis homomeric acetyl-coenzyme a carboxylase to plas tids of rapeseeds' PLANT PHYSIOL. vol. 113, 1997, pages 75 - 81, XP000891347
- 'Computational simulations of the interactions between acetyl-coenzyme a carb ocylase and clodinafop: resistance mechanism due to active and nonactive sit e mutations' J.CHEM.INF.MODEL. vol. 49, 13 July 2009, pages 1936 - 1943, XP008155534
- PLANT CELL PHYSIOL. vol. 43, no. 12, 2002, pages 1518 - 1525, XP009097628

## Description

Rice is one of the most important food crops in the world, particularly in Asia. Rice is a cereal grain produced by plants in the genus *Oryza.* The two most frequently cultivated species are *Oryza sativa* and *Oryza glaberrima,* with *O*. *sativa* being the most frequently cultivated domestic rice. In addition to the two domestic species, the genus *Oryza* contains more than 20 wild species. One of these wild species, *Oryza rufipogon* ("red rice" also referred to as *Oryza sativa* subsp. *rufipogon*) presents a major problem in commercial cultivation. Red rice produces red coated seeds. After harvest, rice seeds are milled to remove their hull. After milling, domestic rice is white while wild red rice appears discolored. The presence of discolored seeds reduces the value of the rice crop. Since red rice belongs to the same species as cultivated rice (*Oryza sativa*), their genetic makeup is very similar. This genetic similarity has made herbicidal control of red rice difficult.

Domestic rice tolerant to imidazolinone herbicides have been developed and are currently marketed under the tradename CLEARFIELD^{®}. Imidazolinone herbicides inhibit a plant's acetohydroxyacid synthase (AHAS) enzyme. When cultivating CLEARFIELD^{®} rice, it is possible to control red rice and other weeds by application of imidazolinone herbicides. Unfortunately, imidazolinone herbicide-tolerant red rice and weeds have developed.

Acetyl-Coenzyme A carboxylase (ACCase; EC 6.4.1.2) enzymes synthesize malonyl-CoA as the start of the de novo fatty acid synthesis pathway in plant chloroplasts. ACCase in grass chloroplasts is a multifunctional, nuclear-genome-encoded, very large, single polypeptide, transported into the plastid via an N-terminal transit peptide. The active form in grass chloroplasts is a homomeric protein, likely a homodimer.

ACCase enzymes in grasses are inhibited by three classes of herbicidal active ingredients. The two most prevalent classes are aryloxyphenoxypropanoates ("FOPs") and cyclohexanediones ("DIMs"). In addition to these two classes, a third class phenylpyrazolines ("DENs") has been described.

A number of ACCase-inhibitor-tolerance (AIT) mutations have been found in monocot weed species exhibiting tolerance toward one or more DIM or FOP herbicides. Further, an AIT maize has been marketed by BASF. All such mutations are found in the carboxyltransferase domain of the ACCase enzyme, and these appear to be located in a substrate binding pocket, altering access to the catalytic site.

DIMs and FOPs are important herbicides and it would be advantageous if rice could be provided that exhibits tolerance to these classes of herbicide. Currently, these classes of herbicide are of limited value in rice agriculture. In some cases, herbicide-tolerance-inducing mutations create a severe fitness penalty in the tolerant plant. Therefore, there remains a need in the art for an AIT rice that also exhibits no fitness penalty. This need and others are met by the present invention.

### BRIEF SUMMARY OF THE INVENTION

The present invention relates to herbicide-tolerant rice plants, rice seeds and rice cells, and methods of producing and treating herbicide-tolerant plants. The rice plant, rice seed and rice cell of the present invention comprise a mutagenized acetyl-Coenzyme A carboxylase (ACCase) nucleic acid having a sequence obtained by an induced, random mutagenesis method and encoding a rice plastidic ACCase having, as a result of said mutagenesis, an isoleucine-to-leucine substitution at an amino acid position corresponding to position 1,781 of the *Alopecurus myosuroides* (*Am*) plastidic ACCase, the rice plastidic ACCase conferring to a rice plant tolerance to 100 g ai/ha cycloxydim. In one embodiment, the present invention provides a rice plant tolerant to at least one herbicide that inhibits acetyl-Coenzyme A carboxylase activity at levels of herbicide that would normally inhibit the growth of a rice plant. The herbicide-tolerant rice plant of the invention expresses an acetyl-Coenzyme A carboxylase (ACCase) in which the amino acid sequence differs from an amino acid sequence of an acetyl-Coenzyme A carboxylase of a wild-type rice plant. By convention, mutations within monocot ACCase amino acid residues are typically referred to in reference to their position in the *Alopecurus myosuroides* (blackgrass) plastidic monomeric ACCase sequence (Genbank CAC84161.1) and denoted with an *(Am).* Examples of additional amino acid positions at which an acetyl-Coenzyme A carboxylase of an herbicide-tolerant plant of the invention differs from the acetyl-Coenzyme A carboxylase of the corresponding wild-type plant include, but are not limited to, one or more of the following positions: 1,785*(Am)*, 1,786*(Am)*, *1,811(Am),* 1,824*(Am), 1,864(Am),* 1,999*(Am),* 2,027*(Am),* 2,039*(Am)*, 2,041*(Am),* 2,049*(Am),* 2,059*(Am),* 2,074*(Am),* 2,075*(Am),* 2,078*(Am), 2,079(Am),* 2,080*(Am),* 2,081 *(Am),* 2,088*(Am)*, 2,095*(Am),* 2,096*(Am)*, or 2,098*(Am)*. Examples of differences at these amino acid positions include, but are not limited to, one or more of the following: the amino acid at position 1,785*(Am)* is other than alanine; the amino acid at position 1,786*(Am)* is other than alanine; the amino acid at position 1,811*(Am)* is other than isoleucine; the amino acid position 1,824*(Am)* is other than glutamine; the amino acid position 1,864*(Am)* is other than valine; the amino acid at position 1,999*(Am)* is other than tryptophan; the amino acid at position 2,027*(Am)* is other than tryptophan; the amino acid position 2,039*(Am)* is other than glutamic acid; the amino acid at position 2,041 *(Am)* is other than isoleucine; the amino acid at position 2,049*(Am)* is other than valine; the amino acid position 2,059*(Am)* is other than an alanine; the amino acid at position 2,074*(Am)* is other than tryptophan; the amino acid at position 2,075*(Am)* is other than valine; the amino acid at position 2,078*(Am)* is other than aspartate; the amino acid position at position 2,079*(Am)* is other than serine; the amino acid at position 2,080*(Am)* is other than lysine; the amino acid position at position 2,081*(Am)* is other than isoleucine; the amino acid at position 2,088*(Am)* is other than cysteine; the amino acid at position 2,095*(Am)* is other than lysine; the amino acid at position 2,096*(Am)* is other than glycine; or the amino acid at position 2,098*(Am)* is other than valine. In some embodiments, the present invention provides a rice plant expressing an acetyl-Coenzyme A carboxylase enzyme comprising an amino acid sequence that comprises an isoleucine-to-leucine substitution at position 1,781*(Am)* and optionally one or more of the following: the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine; the amino acid at position 2,039*(Am)* is glycine; the amino acid at position 2,041*(Am)* is asparagine; the amino acid at position 2,049*(Am)* is phenylalanine; the amino acid at position 2,059*(Am)* is valine; the amino acid at position 2,074*(Am)* is leucine; the amino acid at position 2,075*(Am)* is leucine, isoleucine or methionine; the amino acid at position 2,078*(Am)* is glycine, or threonine; the amino acid at position 2,079*(Am)* is phenylalanine; the amino acid at position 2,080*(Am)* is glutamic acid; the amino acid at position 2,080*(Am)* is deleted; the amino acid at position 2,081 *(Am)* is deleted; the amino acid at position 2,088(*Am*) is arginine, or tryptophan; the amino acid at position 2,095*(Am)* is glutamic acid; the amino acid at position 2,09*6(Am)* is alanine, or
serine; or the amino acid at position 2,098*(Am)* is alanine, glycine, proline, histidine, or serine.

Also described herein are methods of producing herbicide-tolerant plants and plants produced by such methods. An example of a plant produced by the methods described herein is an herbicide-tolerant rice plant which is tolerant to at least one herbicide that inhibits acetyl-Coenzyme A carboxylase activity at levels of herbicide that would normally inhibit the growth of said plant, wherein the herbicide-tolerant plant is produced by: a) obtaining cells from a plant that is not tolerant to the herbicide; b) contacting the cells with a medium comprising one or more acetyl-Coenzyme A carboxylase inhibitors; and c) generating an herbicide-tolerant plant from the cells. Herbicide-tolerant plants produced by methods described herein include, but are not limited to, herbicide-tolerant plants generated by performing a), b) and c) above and progeny of a plant generated by performing a), b), and c) above. Cells used to practice methods of this type can be in the form of a callus.

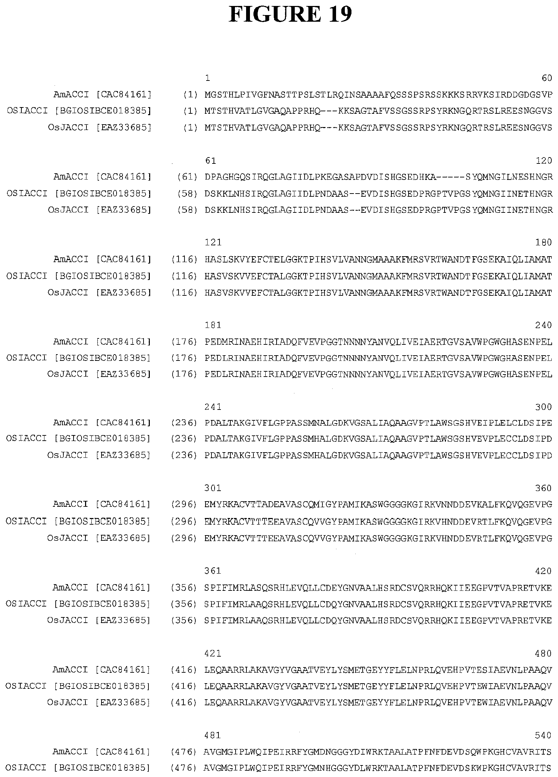

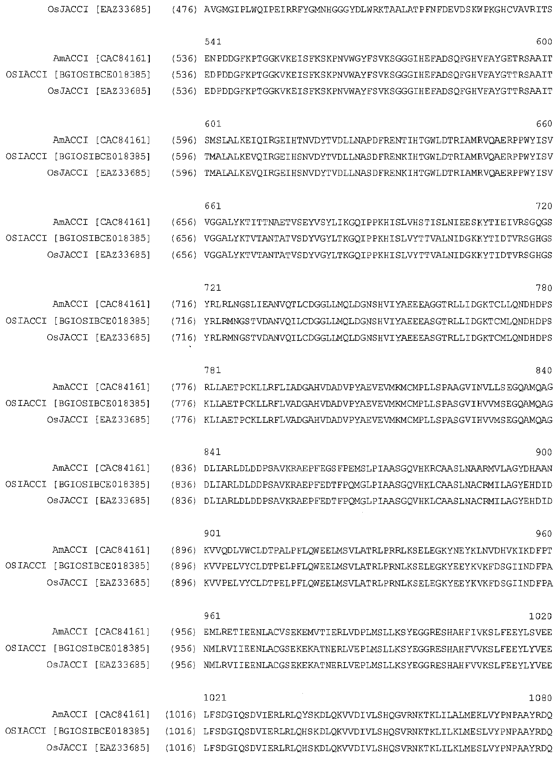

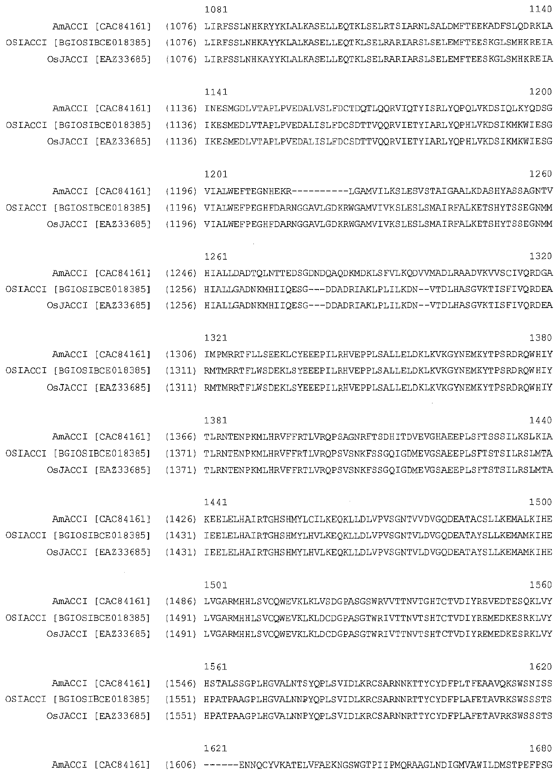

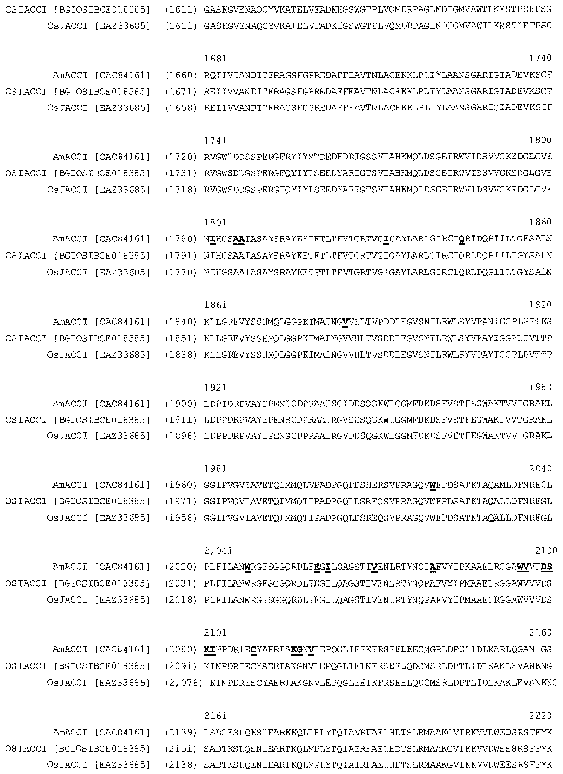

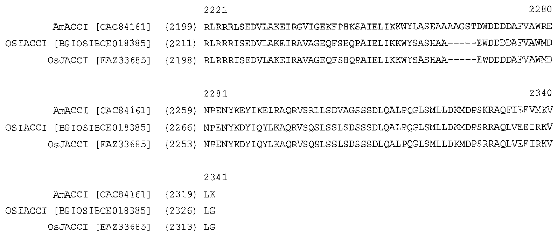

The present invention provides rice plants expressing acetyl-Coenzyme A carboxylase enzymes comprising defined amino acid sequences. For example, the present invention provides a rice plant, wherein one or more of the genomes of said rice plant encode a protein comprising a modified version of one or both of SEQ ID NOs:2 and 3, wherein the sequence is modified such that the encoded protein comprises an isoleucine-to-leucine substitution at position 1,781 *(Am)* and optionally one or more of the following: the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine; the amino acid at position 2,039*(Am)* is glycine; the amino acid at position 2,041 *(Am)* is asparagine; the amino acid at position 2,049*(Am)* is phenylalanine; the amino acid at position 2,059*(Am)* is valine; the amino acid at position 2,074*(Am)* is leucine; the amino acid at position 2,075*(Am)* is leucine, isoleucine or methionine; the amino acid at position 2,078*(Am)* is glycine, or threonine; the amino acid at position 2,079*(Am)* is phenylalanine; the amino acid at position 2,080*(Am)* is glutamic acid; the amino acid at position 2,080*(Am)* is deleted; the amino acid at position 2,081*(Am)* is deleted; the amino acid at position 2,088*(Am)* is arginine, or tryptophan; the amino acid at position 2,095*(Am)* is glutamic acid; the amino acid at position 2,096*(Am)* is alanine, or serine; or the amino acid at position 2,098*(Am)* is alanine, glycine, proline, histidine, or serine. Figure 19 below provides an alignment of the *Alopecurus myosuroides* acetyl-Coenzyme A carboxylase sequence (SEQ ID NO:1), the *Oryza sativa* Indical acetyl-Coenzyme A carboxylase sequence (SEQ ID NO:2.) and the *Oryza sativa* Japonica acetyl-Coenzyme A carboxylase sequence (SEQ ID NO:3) with examples of positions where the wild type sequences may differ with sequences of the invention indicated.

In another embodiment, the present invention comprises seeds deposited in an acceptable depository in accordance with the Budapest Treaty, cells derived from such seeds, plants grown from such seeds and cells derived from such plants, progeny of plants grown from such seed and cells derived from such progeny. The growth of plants produced from deposited seed and progeny of such plants will typically be tolerant to acetyl-Coenzyme A carboxylase-inhibiting herbicides at levels of herbicide that would normally inhibit the growth of a corresponding wild-type plant. In one embodiment, the present invention provides a rice plant grown from a seed produced from a plant of any one of lines OsHPHI2, OsARWI1, OsARWI3, OsARWI8, or OsHPHN1, a representative sample of seed of each line having been deposited with American Type Culture Collection (ATCC) under Patent Deposit Designation Number PTA-10267, PTA-10568, PTA-10569, PTA-10570, or PTA-10571, respectively. The present invention also encompasses mutants, recombinants, and/or genetically engineered derivatives prepared from a plant of any one of lines OsHPHI2, OsARWI1, OsARWI3, OsARWI8, or OsHPHN1, a representative sample of seed of each line having been deposited with ATCC under Patent Deposit Designation Number PTA-10267, PTA-10568, PTA-10569, PTA-10570, or PTA-10571, respectively, as well as any progeny of the plant grown or bred from a plant of any one of lines OsHPHI2, OsARWI1, OsARWI3, OsARWI8, or OsHPHN1, a representative sample of seed of each line having been deposited with ATCC under Patent Deposit Designation Number PTA-10267, PTA-10568, PTA-10569, PTA-10570, or PTA-10571, respectively, so long as such plants or progeny have the herbicide tolerance characteristics of the plant grown from a plant of any one of lines OsHPHI2, OsARWI1 OsARWI3, OsARWI8, or OsHPHN1, a representative sample of seed of each line having been deposited with ATCC under Patent Deposit Designation Number PTA-10267, PTA-10568, PTA-10569, PTA-10570, or PTA-10571, respectively. The present invention also encompasses cells cultured from such seeds and plants and their progeny produced from the cultured cells.

An herbicide-tolerant plant of the invention may be a member of the species *O*. *sativa.* Herbicide-tolerant plants of the invention are typically tolerant to aryloxyphenoxypropionate herbicides, cyclohexanedione herbicides, phenylpyrazoline herbicides or combinations thereof at levels of herbicide that would normally inhibit the growth of a corresponding wild-type plant, for example, a rice plant. In some embodiments, an herbicide-tolerant plant of the invention is not a GMO-plant. The present invention also provides an herbicide-tolerant rice plant that is mutagenized. The present invention also encompasses cells derived from the plants and seeds of the herbicide-tolerant plants described above.

The present invention can be used in methods for controlling growth of weeds. In particular, the present invention can be used in a method of controlling growth of weeds in vicinity to the rice plants of the invention. Such methods may comprise applying to the weeds and rice plants an amount of an acetyl-Coenzyme A carboxylase-inhibiting herbicide that inhibits naturally occurring acetyl-Coenzyme A carboxylase activity, wherein said rice plants comprise altered acetyl-Coenzyme A carboxylase activity such that said rice plants are tolerant to the applied amount of herbicide. The methods described herein may be practiced with any herbicide that interferes with acetyl-Coenzyme A carboxylase activity including, but not limited to, aryloxyphenoxypropionate herbicides, cyclohexanedione herbicides, phenylpyrazoline herbicides or combinations thereof.

One example of a method for controlling growth of weeds in vicinity to rice plants may comprise applying one or more herbicides to the weeds and to the rice plants at levels of herbicide that would normally inhibit the growth of a rice plant, wherein at least one herbicide inhibits acetyl-Coenzyme A carboxylase activity. Such methods may be practiced with any herbicide that inhibits acetyl-Coenzyme A carboxylase activity. Suitable examples of herbicides that may be used in the practice of methods of controlling weeds include, but are not limited to, aryloxyphenoxypropionate herbicides, cyclohexanedione herbicides, phenylpyrazoline herbicides or combinations thereof.

One example of a method for controlling growth of weeds may comprise (a) crossing an herbicide-tolerant rice plant with other rice germplasm, and harvesting the resulting hybrid rice seed; (b) planting the hybrid rice seed; and (c) applying one or more acetyl-Coenzyme A carboxylase-inhibiting herbicides to the hybrid rice and to the weeds in vicinity to the hybrid rice at levels of herbicide that would normally inhibit the growth of a rice plant. Such methods may be practiced with any herbicide that inhibits acetyl-Coenzyme A carboxylase activity. Suitable examples of herbicides that may be used in the practice of methods of controlling weeds include, but are not limited to, aryloxyphenoxypropionate herbicides, cyclohexanedione herbicides, phenylpyrazoline herbicides or combinations thereof.

Further, a method for selecting herbicide-tolerant rice plants is described herein. One example of such methods may comprise (a) crossing an herbicide-tolerant rice plant with other rice germplasm, and harvesting the resulting hybrid rice seed; (b) planting the hybrid rice seed; (c) applying one or more herbicides to the hybrid rice at levels of herbicide that would normally inhibit the growth of a rice plant, wherein at least one of the herbicides inhibits acetyl-Coenzyme A carboxylase; and (d) harvesting seeds from the rice plants to which herbicide has been applied. Such methods maybe practiced with any herbicide that inhibits acetyl-Coenzyme A carboxylase activity. Suitable examples of herbicides that may be used in the practice of methods of controlling weeds include, but are not limited to, aryloxyphenoxypropionate herbicides, cyclohexanedione herbicides, phenylpyrazoline herbicides or combinations thereof.

The present invention also encompasses a method for growing herbicide-tolerant rice plants of the invention and applying to the plants and the vicinity thereof an acetyl-Coenzyme A carboxylase-inhibiting herbicide in an amount that inhibits growth of a corresponding wild type plant. One example of such a method comprises (a) planting rice seeds; (b) allowing the rice seeds to sprout; (c) applying one or more herbicides to the rice sprouts at levels of herbicide that would normally inhibit the growth of a rice plant, wherein at least one of the herbicides inhibits acetyl-Coenzyme A carboxylase. Such methods may be practiced with any herbicide that inhibits acetyl-Coenzyme A carboxylase activity. Suitable examples of herbicides that may be used in the practice of methods of controlling weeds include, but are not limited to, aryloxyphenoxypropionate herbicides, cyclohexanedione herbicides, phenylpyrazoline herbicides or combinations thereof.

Seed of the present invention may be used to grow herbicide-tolerant rice plants, wherein a plant grown from the seed is tolerant to at least one herbicide that inhibits acetyl-Coenzyme A carboxylase activity at levels of herbicide that would normally inhibit the growth of a rice plant. Examples of herbicides to which plants grown from seeds of the invention would be tolerant include but are not limited to, aryloxyphenoxypropionate herbicides, cyclohexanedione herbicides, phenylpyrazoline herbicides or combinations thereof.

In another embodiment, the present invention provides a seed of a rice plant, wherein a plant grown from the seed expresses an acetyl-Coenzyme A carboxylase (ACCase) in which the amino acid sequence differs from an amino acid sequence of an acetyl-Coenzyme A carboxylase of a wild-type rice plant at position 1,781*(Am)* (by an isoleucine-to-leucine substitution) and optionally at one or more of the following positions: 1,785*(Am),* 1,786*(Am),* 1,811*(Am),* 1,824*(Am),* 1,864*(Am), 1,999(Am),* 2,027*(Am),* 2,039*(Am)*, 2,041*(Am)*, *2,049(Am),* 2,059*(Am),* 2,074*(Am),* 2,075*(Am)*, 2,078*(Am),* 2,079*(Am),* 2,080*(Am),* 2,081*(Am), 2,088(Am),* 2,095*(Am),* 2,096*(Am),* or 2,098*(Am).* Examples of differences at these amino acid positions include, but are not limited to, one or more of the following: the amino acid at position 1,785*(Am)* is other than alanine; the amino acid at position 1,786*(Am)* is other than alanine; the amino acid at position 1,811*(Am)* is other than isoleucine; the amino acid position 1,824*(Am)* is other than glutamine; the amino acid position 1,864*(Am)* is other than valine; the amino acid at position 1,999*(Am)* is other than tryptophan; the amino acid at position 2,027*(Am)* is other than tryptophan; the amino acid position 2,039*(Am)* is other than glutamic acid; the amino acid at position 2,041 *(Am)* is other than isoleucine; the amino acid at position 2,049*(Am)* is other than valine; the amino acid position 2,059*(Am)* is other than an alanine; the amino acid at position 2,074*(Am)* is other than tryptophan; the amino acid at position 2,075*(Am)* is other than valine; the amino acid at position 2,078*(Am)* is other than aspartate; the amino acid position at position 2,079*(Am)* is other than serine; the amino acid at position 2,080*(Am)* is other than lysine; the amino acid position at position 2,081*(Am)* is other than isoleucine; the amino acid at position 2,088*(Am)* is other than cysteine; the amino acid at position 2,095*(Am)* is other than lysine; the amino acid at position 2,096*(Am)* is other than glycine; or the amino acid at position 2,098*(Am)* is other than valine. In some embodiments, a plant grown from the seed may express an acetyl-Coenzyme A carboxylase enzyme comprising an amino acid sequence that comprises an isoleucine-to-leucine substitution at position 1,781*(Am)* and optionally one or more of the following: the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine; the amino acid at position 2,039*(Am)* is glycine; the amino acid at position 2,041 *(Am)* is asparagine; the amino acid at position 2,049*(Am)* is phenylalanine; the amino acid at position 2,059*(Am)* is valine; the amino acid at position 2,074*(Am)* is leucine; the amino acid at position 2,075*(Am)* is leucine, isoleucine or methionine; the amino acid at position 2,078*(Am)* is glycine, or threonine; the amino acid at position 2,079*(Am)* is phenylalanine; the amino acid at position 2,080*(Am)* is glutamic acid; the amino acid at position 2,080*(Am)* is deleted; the amino acid at position 2,081 *(Am)* is deleted; the amino acid at position 2,088*(Am)* is arginine, or tryptophan; the amino acid at position 2,095*(Am)* is glutamic acid; the amino acid at position 2,096*(Am)* is alanine, or serine; or the amino acid at position 2,098*(Am)* is alanine, glycine, proline, histidine, or serine.

The present invention encompasses seeds of specific herbicide-tolerant cultivars. One example of such seeds is a seed of rice cultivar Indical, wherein a representative sample of seed of said cultivar was deposited under ATCC Accession No. PTA-10267, PTA-10568, PTA-10569, or PTA-10570. Another example of such seeds are those of an herbicide-tolerant Nipponbare cultivar, wherein a representative sample of seed of said cultivar was deposited under ATCC Accession No. PTA-10571. The present invention also encompasses a rice plant, or a part thereof, produced by growing the seeds as well as a tissue culture of cells produced from the seed. Tissue cultures of cells may be produced from a seed directly or from a part of a plant grown from a seed, for example, from the leaves, pollen, embryos, cotyledons, hypocotyls, meristematic cells, roots, root tips, pistils, anthers, flowers and/or stems. The present invention also includes plants and their progeny that have been generated from tissue cultures of cells. Such plants will typically have all the morphological and physiological characteristics of cultivar Indical .

Also methods for producing rice seed are described herein. Such methods may comprise crossing an herbicide-tolerant rice plant with other rice germplasm; and harvesting the resulting hybrid rice seed, wherein the herbicide-tolerant rice plant is tolerant to aryloxyphenoxypropionate herbicides, cyclohexanedione herbicides, phenylpyrazoline herbicides or combinations thereof at levels of herbicide that would normally inhibit the growth of a rice plant.

Also methods of producing F1 hybrid rice seed are described herein. Such methods may comprise crossing an herbicide-tolerant rice plant with a different rice plant; and harvesting the resultant F1 hybrid rice seed, wherein the herbicide-tolerant rice plant is tolerant to aryloxyphenoxypropionate herbicides, cyclohexanedione herbicides, phenylpyrazoline herbicides or combinations thereof at levels of herbicide that would normally inhibit the growth of a rice plant.

Also methods of producing F1 hybrid plants are described herein. Such methods may comprise crossing an herbicide-tolerant plant with a different plant; and harvesting the resultant F1 hybrid seed and growing the resultant F1 hybrid plant, wherein the herbicide-tolerant plant is tolerant to aryloxyphenoxypropionate herbicides, cyclohexanedione herbicides, phenylpyrazoline herbicides or combinations thereof at levels of herbicide that would normally inhibit the growth of a plant.

Also methods of producing herbicide-tolerant rice plants that may also comprise a transgene are described herein. One example of such a method may comprise transforming a cell of a rice plant with a transgene, wherein the transgene encodes an acetyl-Coenzyme A carboxylase enzyme that confers tolerance to at least one herbicide is selected from the group consisting of aryloxyphenoxypropionate herbicides, cyclohexanedione herbicides, phenylpyrazoline herbicides or combinations thereof. Any suitable cell may be used in the practice of the methods described herein, for example, the cell may be in the form of a callus. The transgene may comprise a nucleic acid sequence encoding an amino acid sequence comprising a modified version of one or both of SEQ ID NOs: 2 and 3, wherein the sequence is modified such that the encoded protein comprises one or more of the following: the amino acid at position 1,781*(Am)* is leucine, threonine, valine, or alanine; the amino acid at position 1,785*(Am)* is glycine; the amino acid at position 1,786*(Am)* is proline; the amino acid at position 1,811 *(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine; the amino acid at position 2,039*(Am)* is glycine; the amino acid at position 2,041 *(Am)* is asparagine; the amino acid at position 2,049*(Am)* is phenylalanine; the amino acid at position 2,059*(Am)* is valine; the amino acid at position 2,074*(Am)* is leucine; the amino acid at position *2,075(Am)* is leucine, isoleucine or methionine; the amino acid at position 2,078*(Am)* is glycine, or threonine; the amino acid at position 2,079*(Am)* is phenylalanine; the amino acid at position 2,080*(Am)* is glutamic acid; the amino acid at position 2,080*(Am)* is deleted; the amino acid at position 2,081 *(Am)* is deleted; the amino acid at position 2,088*(Am)* is arginine, or tryptophan; the amino acid at position 2*,*095(*Am*) is glutamic acid; the amino acid at position 2,09*6(Am)* is alanine, or serine; or the amino acid at position 2,098*(Am)* is alanine, glycine, proline, histidine, or serine. Another example of a method of producing an herbicide-tolerant plant comprising a transgene may comprise transforming a cell of a rice plant with a transgene encoding an enzyme that confers herbicide tolerance, wherein the cell was produced from a rice plant or seed thereof expressing an acetyl-Coenzyme A carboxylase enzyme that confers tolerance to at least one herbicide is selected from the group consisting of aryloxyphenoxypropionate herbicides, cyclohexanedione herbicides, phenylpyrazoline herbicides or combinations thereof. Any suitable cell may be used in the practice of the methods described herein, for example, the cell may be in the form of a callus.

Further, methods of producing recombinant plants are described herein. An example of a method for producing a recombinant rice plant may comprise transforming a cell of a rice plant with a transgene, wherein the cell was produced from a rice plant expressing an acetyl-Coenzyme A carboxylase enzyme that confers tolerance to at least one herbicide is selected from the group consisting of aryloxyphenoxypropionate herbicides, cyclohexanedione herbicides, phenylpyrazoline herbicides or combinations thereof. Any suitable cell may be used in the practice of the methods described herein, for example, the cell may be in the form of a callus. A transgene for use in the methods described herein may comprise any desired nucleic acid sequence, for example, the transgene may encode a protein. In one example, the transgene may encode an enzyme, for example, an enzyme that modifies fatty acid metabolism and/or carbohydrate metabolism. Examples of suitable enzymes include but are not limited to, fructosyltransferase, levansucrase, alpha-amylase, invertase and starch branching enzyme or encoding an antisense of stearyl-ACP desaturase.

The methods described herein may be used to produce a plant, e.g., a rice plant, having any desired traits. An example of such a method may comprise: (a) crossing a rice plant that is tolerant to aryloxyphenoxypropionate herbicides, cyclohexanedione herbicides, phenylpyrazoline herbicides or combinations thereof at levels of herbicide that would normally inhibit the growth of a rice plant with a plant of another rice cultivar that comprises the desired trait to produce progeny plants; (b) selecting one or more progeny plants that have the desired trait to produce selected progeny plants; (c) crossing the selected progeny plants with the herbicide-tolerant plants to produce backcross progeny plants; (d) selecting for backcross progeny plants that have the desired trait and herbicide tolerance; and (e) repeating steps (c) and (d) three or more times in succession to produce selected fourth or higher backcross progeny plants that comprise the desired trait and herbicide tolerance. Any desired trait may be introduced using the methods described herein. Examples of traits that may be desired include, but are not limited to, male sterility, herbicide tolerance, drought tolerance insect resistance, modified fatty acid metabolism, modified carbohydrate metabolism and resistance to bacterial disease, fungal disease or viral disease. An example of a method for producing a male sterile rice plant may comprise transforming a rice plant tolerant to at least one herbicide that inhibits acetyl-Coenzyme A carboxylase activity at levels of herbicide that would normally inhibit the growth of a rice plant with a nucleic acid molecule that confers male sterility.

Further, compositions comprising plant cells, for example, cells from a rice plant are described herein. One example of such a composition comprises one or more cells of a rice plant; and an aqueous medium, wherein the medium comprises a compound that inhibits acetyl-Coenzyme A carboxylase activity. The cells may be derived from a rice plant tolerant to aryloxyphenoxypropionate herbicides, cyclohexanedione herbicides, phenylpyrazoline herbicides or combinations thereof at levels of herbicide that would normally inhibit the growth of a rice plant. Any compound that inhibits acetyl-Coenzyme A carboxylase activity may be used in the compositions described herein, for example, one or more of aryloxyphenoxypropionate herbicides, cyclohexanedione herbicides, phenylpyrazoline herbicides and combinations thereof.

The present invention comprises isolated nucleic acid molecules encoding an acetyl-Coenzyme A carboxylase enzyme as comprised by the plants, seeds or cells of the invention. In some embodiments, the invention comprises an isolated and optionally recombinant and/or synthetic nucleic acid molecule encoding a rice acetyl-Coenzyme A carboxylase (ACCase) in which the amino acid sequence differs from an amino acid sequence of an acetyl-Coenzyme A carboxylase of a wild-type rice plant at position 1,781*(Am)* and optionally at one or more of the following positions: 1,785*(Am)*, 1,786*(Am),* 1,811*(Am), 1,824(Am),* 1,864*(Am),* 1,999*(Am)*, 2,027*(Am),* 2,039*(Am),* 2,041*(Am), 2,049(Am),* 2*,*059(*Am*), *2,074(Am),* 2,075*(Am),* 2,078*(Am),* 2,079*(Am),* 2,080*(Am),* 2,081*(Am),* 2,088*(Am),* 2,095*(Am),* 2,096*(Am)*, or 2,098*(Am)*. Examples of differences at these amino acid positions include, but are not limited to, one or more of the following: the amino acid at position 1,785*(Am)* is other than alanine; the amino acid at position 1,786*(Am)* is other than alanine; the amino acid at position 1,811 *(Am)* is other than isoleucine; the amino acid position 1,824*(Am)* is other than glutamine; the amino acid position 1,864*(Am)* is other than valine; the amino acid at position 1,999*(Am)* is other than tryptophan; the amino acid at position 2,027*(Am)* is other than tryptophan; the amino acid position 2,039*(Am)* is other than glutamic acid; the amino acid at position 2,041 *(Am)* is other than isoleucine; the amino acid at position 2,049*(Am)* is other than valine; the amino acid position 2,059*(Am)* is other than an alanine; the amino acid at position 2,074*(Am)* is other than tryptophan; the amino acid at position 2,075*(Am)* is other than valine; the amino acid at position 2,078*(Am)* is other than aspartate; the amino acid position at position 2,079*(Am)* is other than serine; the amino acid at position 2,080*(Am)* is other than lysine; the amino acid position at position 2,081*(Am)* is other than isoleucine; the amino acid at position 2,088*(Am)* is other than cysteine; the amino acid at position 2,095*(Am)* is other than lysine; the amino acid at position 2,096*(Am)* is other than glycine; or the amino acid at position 2,098*(Am)* is other than valine. In some embodiments, a nucleic acid molecule of the invention may encode an acetyl-Coenzyme A carboxylase enzyme comprising an amino acid sequence that comprises an isoleucine-to-leucine substitution at position 1,781*(Am)* and optionally one or more of the following: the amino acid at position 1,785*(Am)* is glycine; the amino acid at position 1,786*(Am)* is proline; the amino acid at position 1,811 *(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine; the amino acid at position 2,039*(Am)* is glycine; the amino acid at position 2,041*(Am)* is asparagine; the amino acid at position 2,049*(Am)* is phenylalanine; the amino acid at position 2,059*(Am)* is valine; the amino acid at position 2,074*(Am)* is leucine; the amino acid at position 2,075*(Am)* is leucine, isoleucine or methionine; the amino acid at position 2,078*(Am)* is glycine, or threonine; the amino acid at position 2,079*(Am)* is phenylalanine; the amino acid at position 2,080*(Am)* is glutamic acid; the amino acid at position 2,080*(Am)* is deleted; the amino acid at position 2,081*(Am)* is deleted; the amino acid at position 2,088*(Am)* is arginine, or tryptophan; the amino acid at position 2,095*(Am)* is glutamic acid; the amino acid at position 2,096*(Am)* is alanine, or serine; or the amino acid at position 2,098*(Am)* is alanine, glycine, proline, histidine, or serine. In some embodiments, the invention comprises an isolated and optionally recombinant and/or synthetic nucleic acid encoding a protein comprising all or a portion of a modified version of one or both of SEQ ID NOs: 2 and 3, wherein the sequence is modified such that the encoded protein comprises an isoleucine-to-leucine substitution at position 1,781*(Am)* and optionally one or more of the following: the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine; the amino acid at position 2,039*(Am)* is glycine; the amino acid at position 2,041*(Am)* is asparagine; the amino acid at position 2,049*(Am)* is phenylalanine; the amino acid at position 2,059*(Am)* is valine; the amino acid at position 2,074*(Am)* is leucine; the amino acid at position 2,075*(Am)* is leucine, isoleucine or methionine; the amino acid at position 2,078*(Am)* is glycine, or threonine; the amino acid at position 2,079*(Am)* is phenylalanine; the amino acid at position 2,080*(Am)* is glutamic acid; the amino acid at position 2,080*(Am)* is deleted; the amino acid at position 2,081*(Am)* is deleted; the amino acid at position 2,088*(Am)* is arginine, or tryptophan; the amino acid at position 2,095*(Am)* is glutamic acid; the amino acid at position 2,096*(Am)* is alanine, or serine; or the amino acid at position 2,098*(Am)* is alanine, glycine, proline, histidine, or serine.

Further, an herbicide-tolerant, BEP clade plant is described herein. Typically such a plant is one having increased tolerance to an ACCase-inhibitor (ACCI) as compared to a wild-type variety of the plant. Such plants may be produced by a process comprising either:
(I) the steps of
   (a) providing BEP clade plant cells having a first, zero or non-zero level of ACCI tolerance;
   (b) growing the cells in contact with a medium to form a cell culture;
   (c) contacting cells of said culture with an ACCI;
   (d) growing ACCI-contacted cells from step (c) to form a culture containing cells having a level of ACCI tolerance greater than the first level of step (a); and
   (e) generating, from ACCI-tolerant cells of step (d), a plant having a level of ACCI tolerance greater than that of a wild-type variety of the plant; or
(II) the steps of
   (f) providing a first, herbicide-tolerant, BEP clade plant having increased tolerance to an ACCase-inhibitor (ACCI) as compared to a wild-type variety of the plant, said herbicide-tolerant plant having been produced by a process comprising steps (a)-(e); and
   (g) producing from the first plant a second, herbicide-tolerant, BEP clade plant that retains the increased herbicide tolerance characteristics of the first plant;
thereby obtaining an herbicide-tolerant, BEP clade plant.

The herbicide-tolerant BEP clade plant described herein may be a BET subclade plant.

The herbicide-tolerant BET subclade plant described herein may be a BET crop plant.

The herbicide-tolerant plant described herein may be a member of the Bambusoideae - Ehrhartoideae subclade. Any suitable medium for growing plant cells may be used in the process described herein. The medium may comprise a mutagen. Alternatively, the medium may not comprise a mutagen. An herbicide-tolerant plant described herein may be a member of the subfamily Ehrhartoideae. Any suitable cells may be used in the practice of the methods described herein, for example, the cells may be in the form of a callus. The herbicide-tolerant plant of the invention may be a member of the genus *Oryza,* for example, may be a member of the species *O*. *sativa.*

Herbicide-tolerant BEP clade plants produced by the above method may express an acetyl-Coenzyme A carboxylase (ACCase) in which the amino acid sequence differs from an amino acid sequence of an acetyl-Coenzyme A carboxylase of a corresponding wild-type BEP clade plant at one or more of the following positions: 1,781*(Am),* 1,785*(Am),* 1,786*(Am)*, 1,811*(Am)*, 1,824*(Am),* 1,864*(Am), 1,999(Am), 2,027(Am),* 2,039*(Am)*, 2,041*(Am),* 2,049*(Am),* 2,059*(Am)*, *2,074(Am),* 2,075*(Am),* 2,078*(Am),* 2,079*(Am),* 2,080*(Am),* 2,081*(Am),* 2,088*(Am), 2,095(Am),* 2,096*(Am),* or 2,098*(Am)*. Examples of differences at these amino acid positions include, but are not limited to, one or more of the following: the amino acid at position 1,781*(Am)* is other than isoleucine; the amino acid at position 1,785*(Am)* is other than alanine; the amino acid at position 1,786*(Am)* is other than alanine; the amino acid at position 1,811 *(Am)* is other than isoleucine; the amino acid position 1,824*(Am)* is other than glutamine; the amino acid position 1,864*(Am)* is other than valine; the amino acid at position 1,999*(Am)* is other than tryptophan; the amino acid at position 2,027*(Am)* is other than tryptophan; the amino acid position 2,039*(Am)* is other than glutamic acid; the amino acid at position 2,041 *(Am)* is other than isoleucine; the amino acid at position *2,049(Am)* is other than valine; the amino acid position 2,059*(Am)* is other than an alanine; the amino acid at position 2,074*(Am)* is other than tryptophan; the amino acid at position 2,075*(Am)* is other than valine; the amino acid at position *2,078(Am)* is other than aspartate; the amino acid position at position 2,079*(Am)* is other than serine; the amino acid at position 2,080*(Am)* is other than lysine; the amino acid position at position 2,081 *(Am)* is other than isoleucine; the amino acid at position 2,088*(Am)* is other than cysteine; the amino acid at position 2,095*(Am)* is other than lysine; the amino acid at position 2,096*(Am)* is other than glycine; or the amino acid at position 2,098*(Am)* is other than valine. The herbicide-tolerant BEP clade plant described herein may express an acetyl-Coenzyme A carboxylase enzyme comprising an amino acid sequence that comprises one or more of the following: the amino acid at position 1,781*(Am)* is leucine, threonine, valine, or alanine; the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine; the amino acid at position 2,039*(Am)* is glycine; the amino acid at position 2,041 *(Am)* is asparagine; the amino acid at position 2,049*(Am)* is phenylalanine; the amino acid at position 2,059*(Am)* is valine; the amino acid at position 2,074*(Am)* is leucine; the amino acid at position 2,075*(Am)* is leucine, isoleucine or methionine; the amino acid at position 2,078*(Am)* is glycine, or threonine; the amino acid at position 2,079*(Am)* is phenylalanine; the amino acid at position 2,080*(Am)* is glutamic acid; the amino acid at position 2,080*(Am)* is deleted; the amino acid at position 2,081*(Am)* is deleted; the amino acid at position 2,088*(Am)* is arginine, or tryptophan; the amino acid at position 2,095*(Am)* is glutamic acid; the amino acid at position 2,096*(Am)* is alanine, or serine; or the amino acid at position 2,098*(Am)* is alanine, glycine, proline, histidine, or serine.

In one embodiment, the present invention also includes rice plants that are tolerant to ACCase inhibitors by virtue of having only one substitution (an isoleucine-to-leucine substitution at position 1,781*(Am)*) in its plastidic ACCase as compared to the corresponding wild-type ACCase. In yet another embodiment, the invention includes rice plants that are tolerant to ACCase inhibitors by virtue of having one or more additional substitutions in its plastidic ACCase as compared to the corresponding wild-type ACCase.

In one embodiment, the present invention provides rice plants that are tolerant to ACCase inhibitors, by virtue of having two or more substitution in its plastidic ACCase as compared to the corresponding wild-type ACCase, wherein the substitutions are an isoleucine-to-leucine substitution at position 1,781 *(Am)* and one or more additional substitutions at amino acid positions selected from the group consisting of 1,785*(Am), 1,786(Am),* 1,811*(Am), 1,824(Am),* 1,864*(Am),* 1,999*(Am),* 2,027*(Am),* 2,039*(Am)*, 2,041*(Am), 2,049(Am),* 2,059*(Am),* 2,074*(Am),* 2,075*(Am),* 2,078*(Am), 2,079(Am),* 2,080(Am), 2,081*(Am),* 2,088*(Am), 2,095(Am),* 2,096*(Am),* or 2,098*(Am)*.

In one embodiment, the present invention provides rice plants wherein the rice plants comprise plastidic ACCase that is not transgenic. In one embodiment, the present invention provides plants wherein the plants comprise a rice plastidic ACCase that is transgenic.

Further, methods for controlling growth of weeds within the vicinity of a rice plant are described herein, comprising applying to the weeds and rice plants an amount of an acetyl-Coenzyme A carboxylase-inhibiting herbicide that inhibits naturally occurring acetyl-Coenzyme A carboxylase activity, wherein said rice plants comprise altered acetyl-Coenzyme A carboxylase activity such that said rice plants are tolerant to the applied amount of herbicide.

Further, methods for producing seed are described herein comprising: (i) planting seed produced from a plant of the invention, (ii) growing plants from the seed and (ii) harvesting seed from the plants.

Also described are herbicide-tolerant BEP clade plants produced by the process of (a) crossing or back-crossing a plant grown from a seed of an herbicide-tolerant BEP clade plant produced as described above with other germplasm; (b) growing the plants resulting from said crossing or back-crossing in the presence of at least one herbicide that normally inhibits acetyl-Coenzyme A carboxylase, at levels of the herbicide that would normally inhibit the growth of a plant; and (c) selecting for further propagation plants resulting from said crossing or back-crossing, wherein the plants selected are plants that grow without significant injury in the presence of the herbicide.

The present invention also encompasses an isolated and optionally recombinant and/or synthetic nucleic acid molecule comprising a nucleotide sequence encoding a mutagenized acetyl-Coenzyme A carboxylase of a plant in the BEP clade of the Family Poaceae, in which the amino acid sequence of the mutagenized acetyl-Coenzyme A carboxylase differs from an amino acid sequence of an acetyl-Coenzyme A carboxylase of the corresponding wild-type plant at position 1,781 *(Am)* (by an isoleucine-to-leucine substitution) and optionally at one or more of the following positions: 1,785*(Am)*, 1,786*(Am),* 1,811*(Am),* 1,824*(Am),* 1,864*(Am)*, 1,999*(Am)*, *2,027(Am),* 2,039*(Am)*, 2,041(*Am*), *2,049(Am),* 2,059*(Am),* 2*,*014(*Am*), *2,075(Am),* 2,078*(Am),* 2,079*(Am),* 2,080*(Am)*, 2,081*(Am),* 2,088*(Am),* 2,095*(Am),* 2,096*(Am),* or 2,098(*Am*). Such a nucleic acid molecule may be produced by a process comprising either:
(I) the steps of
   (a) providing BEP clade plant cells having a first, zero or non-zero level of ACCase-inhibitor (ACCI) tolerance;
   (b) growing the cells in contact with a medium to form a cell culture;
   (c) contacting cells of said culture with an ACCI;
   (d) growing ACCI-contacted cells from step (c) to form a culture containing cells having a level of ACCI tolerance greater than the first level of step (a); and
   (e) generating, from ACCI-tolerant cells of step (d), a plant having a level of ACCI tolerance greater than that of a wild-type variety of the plant; or
(II) the steps of
   (f) providing a first, herbicide-tolerant, BEP clade plant having increased tolerance to an ACCase-inhibitor (ACCI) as compared to a wild-type variety of the plant, said herbicide-tolerant plant having been produced by a process comprising steps (a)-(e); and
   (g) producing from the first plant a second, herbicide-tolerant, BEP clade plant that retains the increased herbicide tolerance characteristics of the first plant;
thereby obtaining an herbicide-tolerant, BEP clade plant; and isolating a nucleic acid from the herbicide-tolerant BEP clade plant.

Further, methods of screening, isolating, identifying, and/or characterizing herbicide tolerant mutations in monocot plastidic ACCases are described herein. Calli, or plant cell lines can be used. Plant material or cells can be cultured in a tissue culture environment. A nylon membrane can be present in the tissue culture environment. The tissue culture environment can comprise liquid phase media. Alternatively, the environment can comprise semi-solid media. Plant material can be cultured in the presence of herbicide (e.g., cycloxydim) in liquid media followed by culturing in semi-solid media with herbicide. Alternatively, plant material can be cultured in the presence of herbicide in semi-solid media followed by culturing in liquid media with herbicide.

A lethal dose of herbicide (e.g., cycloxydim) can be directly applied. Alternatively, the herbicide dose can be increased step-wise, starting with a sub-lethal dose. At least one, at least two, at least three, at least four, at least five, at least six, at least seven, at least eight, or more herbicides can be used in one step, or concurrently.

The mutational frequency can be determined by the number of mutant herbicide-tolerant clones as a fraction of the number of the individual calli used in the experiment. For example, the mutational frequency can be at least 0.03% or higher such as at least 0.03%, at least 0.05%, at least 0.10%, at least 0.15%, at least 0.20%, at least 0.25%, at least 0.30%, at least 0.35%, at least 0.40% or higher. The mutational frequencies can be at least 2 fold, at least 3 fold, at least 4 fold, at least 5 fold, at least 6 fold, at least 7 fold, at least 8 fold, at least 9 fold, at least 10 fold or higher than other methods of screening, isolating, identifying, and/or characterizing herbicide tolerant mutations in monocot plastidic ACCases.

The methods described herein may encompass identifying the herbicide tolerant mutation(s) in the ACCase. Further, the herbicide tolerant mutation(s) in monocot plant cells may be recapitulated.

In some embodiments, the invention encompasses an isolated cell or tissue said cell or tissue of plant origin having: a) a deficiency in ACCase activity derived from a host ACCase (i.e., endogenous) gene; and b) an ACCase activity from a rice plastidic ACCase gene as described herein.

### Monocot sources of ACCase

Further described herein are plastidic ACCases or portions thereof from the monocot family of plants as described herein.

Further, screening for herbicide-tolerant mutants of monocot plastidic ACCase in host plant cells is described herein.

Prepared host cells may be used to screen for herbicide-tolerant mutants of monocot plastidic ACCase. The host cell described herein may be devoid of plastidic ACCase activity. Alternatively, the host cells described herein may express a monocot plastidic ACCase which is herbicide sensitive.

The methods described herein may comprise host cells deficient in ACCase activity due to a mutation of the genomic plastidic ACCase gene which include a single point mutation, multiple point mutations, a partial deletion, a partial knockout, a complete deletion and a complete knockout. Alternatively, genomic plastidic ACCase activity is reduced or ablated using other molecular biology techniques such as RNAi, siRNA or antisense RNA. Such molecular biology techniques are well known in the art. In yet other alternative, genomic ACCase derived activity may be reduced or ablated by a metabolic inhibitor of ACCase.

The host cell can be a monocot plant host cell.

Further, a method of making a transgenic plant cell is described herein comprising: a) isolating a cell having a monocot plant origin; b) inactivating at least one copy of a genomic ACCase gene; c) providing a monocot-derived plastidic ACCase gene to said cell; d) isolating the cell comprising the monocot-derived plastidic ACCase gene; and optionally; e) inactivating at least additional copy of a genomic ACCase gene and wherein said cell is deficient in ACCase activity provided by the genomic ACCase gene.

The cycloxydim-tolerant mutational frequency may be greater than 0.03 %.

Further, a method for screening is described herein, wherein cycloxydim-tolerant plant cells or tissues are also tolerant to other ACCase inhibitors.

Further, a method for screening is described herein, wherein the cycloxydim-tolerant plant cells or tissues comprise only one mutation not present in the monocot plastidic ACCase prior to culturing in the presence of the herbicide.

Further, a method for screening is described herein, wherein the cycloxydim-tolerant plant cells or tissues comprise two or more mutations not present in the monocot plastidic ACCase prior to culturing in the presence of the herbicide.

Further, a method for screening is described herein, wherein the cycloxydim is present at a sub-lethal dose.

Further, a method for screening is described herein, wherein the culturing in the presence of cycloxydim is performed in step-wise or gradual increase in cycloxydim concentrations.

Further, a method for screening is described herein, wherein the method comprises culturing of cells on a membrane. Preferably, the method for screening comprises culturing of cells on a nylon membrane.

Further, a method for screening cycloxydim-tolerant plant cells is described herein, wherein the culturing of cells is in liquid media or semi-solid media.

Further, a method for screening is described herein, wherein the method further comprises identification of the at least one mutation not present in the exogenous monocot plastidic ACCase prior to culturing in the presence of the cycloxydim.

In the method for screening described herein said monocot may be rice.

In the method for screening described herein said exogenous monocot plastidic ACCase may be from rice.

### BRIEF DESCRIPTION OF THE DRAWINGS

Figure 1 is a bar graph showing relative growth rice calli derived from *Oryza sativa* subsp. *indica* grown in the presence of difference selection levels of herbicide. Figure 1A shows the results obtained with tepraloxydim, Figure 1B shows the results obtained with sethoxydim, and Figure 1C shows the results obtained with cycloxydim.
Figure 2 is a diagram of the selection process used to produce herbicide-tolerant rice plants.
Figure 3 shows photographs of plants taken one week after treatment with herbicide.
Figure 4 shows photographs of plants taken two weeks after treatment with herbicide.
Figure 5 provides the amino acid sequence of acetyl-coenzyme A carboxylase from *Alopecurus myosuroides* (GenBank accession number CAC84161).
Figure 6 provides the mRNA encoding acetyl-coenzyme A carboxylase from *Alopecurus myosuroides* (GenBank accession number AJ310767 region: 157..7119) (SEQ ID NO:4).
Figure 7A provides the genomic nucleotide sequence for *Oryza sativa* Indica & Japonica acetyl-Coenzyme A carboxylase gene (SEQ ID NO:5).
Figure 7B provides the nucleotide sequence encoding *Oryza sativa* Indica & Japonica acetyl-Coenzyme A carboxylase (SEQ ID NO:6).
Figure 7C provides the amino acid sequence of *Oryza sativa* Indica acetyl-Coenzyme A carboxylase (SEQ ID NO:3).
Figure 8A provides the nucleotide sequence encoding *Zea mays* acetyl-Coenzyme A carboxylase (SEQ ID NO:11).
Figure 8B provides the amino acid sequence of *Zea mays* acetyl-Coenzyme A carboxylase (SEQ ID NO:12).
Figure 9A provides the nucleotide sequence encoding *Zea mays* acetyl-Coenzyme A carboxylase (SEQ ID NO:13).
Figure 9B provides the amino acid sequence of *Zea mays* acetyl-Coenzyme A carboxylase (SEQ ID NO:14).
Figure 10A provides the nucleotide sequence encoding *Triticum aestivum* acetyl-Coenzyme A carboxylase (SEQ ID NO:15).
Figure 10B provides the amino acid sequence of *Triticum aestivum* acetyl-Coenzyme A carboxylase (SEQ ID NO:16).
Figure 11A provides the nucleotide sequence encoding *Setaria italica* acetyl-Coenzyme A carboxylase (SEQ ID NO:17).
Figure 11B provides the amino acid sequence of *Setaria italica* acetyl-Coenzyme A carboxylase (SEQ ID NO:18).
Figure 12A provides the nucleotide sequence encoding *Setaria italica* acetyl-Coenzyme A carboxylase (SEQ ID NO:19).
Figure 12B provides the amino acid sequence of *Setaria italica* acetyl-Coenzyme A carboxylase (SEQ ID NO:20).
Figure 13A provides the nucleotide sequence encoding *Setaria italica* acetyl-Coenzyme A carboxylase (SEQ ID NO:21).
Figure 13B provides the amino acid sequence of *Setaria italica* acetyl-Coenzyme A carboxylase (SEQ ID NO:22).
Figure 14A provides the nucleotide sequence encoding *Alopecurus myosuroides* acetyl-Coenzyme A carboxylase (SEQ ID NO:23).
Figure 14B provides the amino acid sequence of *Alopecurus myosuroides* acetyl-Coenzyme A carboxylase (SEQ ID NO:24).
Figure 15A provides the nucleotide sequence encoding *Aegilops tauschii* acetyl-Coenzyme A carboxylase (SEQ ID NO:25).
Figure 15B provides the amino acid sequence of *Aegilops tauschii* acetyl-Coenzyme A carboxylase (SEQ ID NO:26).
Figure 16 provides a comparison of single and double mutants.
Figure 17 provides a graph showing results for mutant rice versus various ACCase inhibitors.
Figure 18 provides *Alopecurus myosuroides* acetyl-Coenzyme A carboxylase amino acid sequence (GenBank accession no. CAC84161). Amino acids that maybe altered in the acetyl-Coenzyme A carboxylase enzymes comprised by the plants of the invention are indicated in bold double underline.
Figure 19 provides amino acid sequence of wild-type *Oryza sativa* acetyl-Coenzyme A carboxylases aligned with *Alopecurus myosuroides* acetyl-Coenzyme A carboxylase with some critical residues denoted.

### DETAILED DESCRIPTION OF THE INVENTION

### Definitions

As used herein, "tolerant" or "herbicide-tolerant" indicates a plant or portion thereof capable of growing in the presence of an amount of herbicide that normally causes growth inhibition in a non-tolerant (e.g., a wild-type) plant or portion thereof. Levels of herbicide that normally inhibit growth of a non-tolerant plant are known and readily determined by those skilled in the art. Examples include the amounts recommended by manufacturers for application. The maximum rate is an example of an amount of herbicide that would normally inhibit growth of a non-tolerant plant.

As used herein, "recombinant" refers to an organism having genetic material from different sources.

As used herein, "mutagenized" refers to an organism having an altered genetic material as compared to the genetic material of a corresponding wild-type organism, wherein the alterations in genetic material were induced and/or selected by human action. Examples of human action that can be used to produce a mutagenized organism include, but are not limited to, tissue culture of plant cells (e.g., calli) in sub-lethal concentrations of herbicides (e.g., acetyl-Coenzyme A carboxylase inhibitors such as cycloxydim or sethoxydim), treatment of plant cells with a chemical mutagen and subsequent selection with herbicides (e.g., acetyl-Coenzyme A carboxylase inhibitors such as cycloxydim or sethoxydim); or by treatment of plant cells with x-rays and subsequent selection with herbicides (e.g., acetyl-Coenzyme A carboxylase inhibitors such as cycloxydim or sethoxydim). Any method known in the art may be used to induce mutations. Methods of inducing mutations may induce mutations in random positions in the genetic material or may induce mutations in specific locations in the genetic material (i.e., may be directed mutagenesis techniques).

As used herein, a "genetically modified organism" (GMO) is an organism whose genetic characteristics have been altered by insertion of genetic material from another source organism or progeny thereof that retain the inserted genetic material. The source organism may be of a different type of organism (e.g., a GMO plant may contain bacterial genetic material) or from the same type of organism (e.g., a GMO plant may contain genetic material from another plant). As used herein, recombinant and GMO are considered synonyms and indicate the presence of genetic material from a different source whereas mutagenized indicates altered genetic material from a corresponding wild-type organism but no genetic material from another source organism.

As used herein, "wild-type" or "corresponding wild-type plant" means the typical form of an organism or its genetic material, as it normally occurs, as distinguished from mutagenized and/or recombinant forms.

For the present invention, the terms "herbicide-tolerant" and "herbicide-resistant" are used interchangeably and are intended to have an equivalent meaning and an equivalent scope. Similarly, the terms "herbicide-tolerance" and "herbicide-resistance" are used interchangeably and are intended to have an equivalent meaning and an equivalent scope. Similarly, the terms "tolerant" and "resistant" are used interchangeably and are intended to have an equivalent meaning and an equivalent scope.

As used herein in regard to herbicides useful in various embodiments hereof, terms such as auxinic herbicide, AHAS inhibitor, acetyl-Coenzyme A carboxylase (ACCase) inhibitor, PPO inhibitor, EPSPS inhibitor, imidazolinone, sulfonylurea, and the like, refer to those agronomically acceptable herbicide active ingredients (A.I.) recognized in the art. Similarly, terms such as fungicide, nematicide, pesticide, and the like, refer to other agronomically acceptable active ingredients recognized in the art.

When used in reference to a particular mutant enzyme or polypeptide, terms such as herbicide tolerant (HT) and herbicide tolerance refer to the ability of such enzyme or polypeptide to perform its physiological activity in the presence of an amount of an herbicide A.I. that would normally inactivate or inhibit the activity of the wild-type (non-mutant) version of said enzyme or polypeptide. For example, when used specifically in regard to an AHAS enzyme, or AHASL polypeptide, it refers specifically to the ability to tolerate an AHAS-inhibitor. Classes of AHAS-inhibitors include sulfonylureas, imidazolinone, triazolopyrimidines, sulfonylaminocarbonyltriazolinones, and pyrimidinyloxy[thio]henzoates.

As used herein, "descendant" refers to any generation plant.

As used herein, "progeny" refers to a first generation plant.

### Plants

Herbicide-tolerant monocotyledonous plants of the grass family Poaceae are described herein. The family Poaceae may be divided into two major clades, the clade containing the subfamilies Bambusoideae, Ehrhartoideae, and Pooideae (the BEP clade) and the clade containing the subfamilies Panicoideae, Arundinoideae, Chloridoideae, Centothecoideae, Micrairaideae, Aristidoideae, and Danthonioideae (the PACCMAD clade). The subfamily Bambusoideae includes tribe *Oryzeae.* The present invention relates to plants of the BEP clade, in particular plants of the subfamily Ehrhartoideae, namely rice plants. Plants of the invention are typically tolerant to at least one herbicide that inhibits acetyl-Coenzyme A carboxylase activity as a result of expressing an acetyl-Coenzyme A carboxylase enzyme of the invention as described below. The BET clade includes subfamilies Bambusoideae, Ehrhartoideae, and group Triticodae and no other subfamily Pooideae groups. BET crop plants are plants grown for food or forage that are members of BET subclade, for example barley, corn, etc.

Commercially important herbicide-tolerant monocots described herein include Sugarcane (Saccharum spp.), as well as Turfgrasses, e.g., Poa pratensis (Bluegrass), Agrostis spp. (Bentgrass), Lolium spp. (Ryegrasses), Festuca spp. (Fescues), Zoysia spp. (Zoysia grass), Cynodon spp. (Bermudagrass), Stenotaphrum secundatum (St. Augustine grass), Paspalum spp. (Bahiagrass), Eremochloa ophiuroides (Centipedegrass), Axonopus spp. (Carpetgrass), Bouteloua dactyloides (Buffalograss), and Bouteloua var. spp. (Grama grass).

Herbicide-tolerant plants of the Bambusoideae subfamily are described herein. Such plants are typically tolerant to one or more herbicides that inhibit acetyl-Coenzyme A carboxylase activity. Examples of herbicide-tolerant plants of the subfamily Bambusoideae include, but are not limited to, those of the genera Arundinaria, Bambusa, Chusquea, Guadua, and Shibataea.

Further, herbicide-tolerant plants of the Ehrhartoideae subfamily are described herein. Such plants are typically tolerant to one or more herbicides that inhibit acetyl-Coenzyme A carboxylase activity. Examples of herbicide-tolerant plants of the subfamily Ehrhartoideae include, but are not limited to, those of the genera *Erharta, Leersia, Microlaena, Oryza,* and *Zizania.*

Further, herbicide-tolerant plants of the Pooideae subfamily are described herein. Such plants are typically tolerant to one or more herbicides that inhibit acetyl-Coenzyme A carboxylase activity. Examples of herbicide-tolerant plants of the subfamily Ehrhartoideae include, but are not limited to, those of the genera *Triticeae, Aveneae,* and *Poeae.*

The herbicide-tolerant plants of the invention are rice plants. Two species of rice are most frequently cultivated, *Oryza sativa* and *Oryza glaberrima.* Numerous subspecies of *Oryza sativa* are commercially important including *Oryza sativa* subsp. *indica, Oryza sativa* subsp. *japonica, Oryza sativa* subsp.*javanica, Oryza sativa* subsp. *glutinosa* (glutinous rice), *Oryza sativa* Aromatica group (e.g., basmati), and *Oryza sativa* (Floating rice group). The present invention encompasses herbicide-tolerant rice plants in all of the aforementioned species and subspecies.

Further, herbicide-tolerant wheat plants are described herein. Two species of wheat are most frequently cultivated, *Triticum aestivum,* and *Triticum turgidum.* Numerous other species are commercially important including, but not limited to, *Triticum timopheevii, Triticum monococcum, Triticum zhukovskyi* and *Triticum urartu* and hybrids thereof. Examples af T. aestivum subspecies are aestivum (common wheat), compactum (club wheat), macha (macha wheat), vavilovi (vavilovi wheat), spelta and sphaecrococcum (shot wheat). Examples of T. turgidum subspecies are turgidum, carthlicum, dicoccon, durum, palcocolchictma, polonicum, turanicum and dicoccoides. Examples of T. monococcum subspecies are monococcum (einkorn) and aegilopoides. The wheat plant can be a member of the Triticum aestivum species, and more particularly, the CDC Teal cultivar.

Further, herbicide-tolerant barley plants are described herein. Two species of barley are most frequently cultivated, *Hordeum vulgare* and *Hordeum arizonicum.* Numerous other species are commercially important including, but not limited, *Hordeum bogdanii, Hordeum brachyantherum, Hordeum brevisubulatum, Hordeum bulbosum, Hordeum comosum, Hordeun depressum, Hordeum intercedes, Hordeum jubatum, Hordeum marinum, Hordeum parodii, Hordeum pusillum, Hordeum secalinum,* and *Hordeum spontaneum.*

Further, herbicide-tolerant rye plants are described herein. Commercially important species include, but are not limited to, *Secale sylvestre, Secale strictum, Secale cereale, Secale vavilovii, Secale africanum, Secale ciliatoglume, Secale ancestrale,* and *Secale montanum.*

Further, herbicide-tolerant turf plants are described herein. Numerous commercially important species of Turf grass include *Zoysia japonica, Agrostris palustris,* Poa *pratensis, Poa annua, Digitaria sanguinalis, Cyperus rotundus, Kyllinga brevifolia*, *Cyperus amuricus*, Erigeron *canadensis, Hydrocotyle sibthorpioides, Kummerowia striata, Euphorbia humifusa,* and *Viola arvensis.*

In addition to being able to tolerate herbicides that inhibit acetyl-Coenzyme A carboxylase activity, plants of the invention may also be able to tolerate herbicides that work on other physiological processes. For example, plants of the invention may be tolerant to acetyl-Coenzyme A carboxylase inhibitors and also tolerant to other herbicides, for example, enzyme inhibitors. Examples of other enzyme inhibitors to which plants of the invention may be tolerant include, but are not limited to, inhibitors of 5-enolpyruvylshikimate-3-phosphate synthase (EPSPS) such as glyphosate, inhibitors of acetohydroxyacid synthase (AHAS) such as imidazolinones, sulfonylureas and sulfonamide herbicides, and inhibitors of glutamine synthase such as glufosinate. In addition to enzyme inhibitors, plants of the invention may also be tolerant of herbicides having other modes of action, for example, auxinic herbicides such as 2,4-D or dicamba, chlorophyll/carotenoid pigment inhibitors such as hydroxyphenylpyruvate dioxygenase (HPPD) inhibitors or phytoene desaturase (PDS) inhibitors, protoporphyrinogen-IX oxidase inhibitors, cell membrane destroyers, photosynthetic inhibitors such as bromoxynil or ioxynil, cell division inhibitors, root inhibitors, shoot inhibitors, and combinations thereof. Thus, plants of the invention tolerant to acetyl-Coenzyme A carboxylase inhibitors can be made resistant to multiple classes of herbicides.

For example, plants of the invention tolerant to acetyl-Coenzyme A carboxylase inhibitors, such as "dims" (e.g., cycloxydim, sethoxydim, clethodim, or tepraloxydim), "fops" (e.g., clodinafop, diclofop, fluazifop, haloxyfop, or quizalofop), and "dens" (such as pinoxaden), in some embodiments, may be auxinic-herbicide tolerant, tolerant to EPSPS inhibitors, such as glyphosate; to PPO inhibitors, such as pyrimidinedione, such as saflufenacil, triazolinone, such as sulfentrazone, carfentrazone, flumioxazin, diphenylethers, such as acifluorfen, fomesafen, lactofen, oxyfiuorfen, N-phenylphthalamides, such as flumiclorac, CGA-248757, and/or to GS inhibitors, such as glufosinate. In addition to these classes of inhibitors, plants of the invention tolerant to acetyl-Coenzyme A carboxylase inhibitors may also be tolerant to herbicides having other modes of action, for example, chlorophyll/carotenoid pigment inhibitors, cell membrane disruptars, photosynthesis inhibitors, cell division inhibitors, root inhibitors, shoot inhibitors, and combinations thereof. Such tolerance traits may be expressed, e.g., as mutant EPSPS proteins, or mutant glutamine synthetase proteins; or as mutant native, inbred, or transgenic aryloxyalkanoate dioxygenase (AAD or DHT), haloarylnitrilase (BXN), 2,2-dichloropropionic acid dehalogenase (DEH), glyphosate-N-acetyltransferase (GAT), glyphosate decarboxylase (GDC), glyphosate oxidoreductase (GOX), glutathione-S-transferase (GST), phosphinothricin acetyltransferase (PAT or bar), or cytochrome P450 (CYP450) proteins having an herbicide-degrading activity. Plants tolerant to acetyl-Coenzyme A carboxylase inhibitors hereof can also be stacked with other traits including, but not limited to, pesticidal traits such as *Bt Cry* and other proteins having pesticidal activity toward coleopteran, lepidopteran, nematode, or other pests; nutrition or nutraceutical traits such as modified oil content or oil profile traits, high protein or high amino acid concentration traits, and other trait types known in the art.

Furthermore, plants are also covered that, in addition to being able to tolerate herbicides that inhibit acetyl-Coenzyme A carboxylase activity, are by the use of recombinant DNA techniques capable to synthesize one or more insecticidal proteins, especially those known from the bacterial genus *Bacillus,* particularly from *Bacillus thuringiensis*, such as δ-endotoxins, e.g, CryIA(b), CryIA(c), CryIF, CryIF(a2), CryIIA(b), CryIIIA, CryIIIB(b1) or Cry9c; vegetative insecticidal proteins (VIP), e. g. VIP1, VIP2, VIP3 or VIP3A; insecticidal proteins of bacteria colonizing nematodes, e. g. *Photorhabdus* spp. or *Xenorhabdus* spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxy-steroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilben synthase, bibenzyl synthase, chitinases or glucanases. In the context of the present invention these insecticidal proteins or toxins are to be understood expressly also as pre-toxins, hybrid proteins, truncated or otherwise modified proteins. Hybrid proteins are characterized by a new combination of protein domains, (see, e. g. WO 02/015701). Further examples of such toxins or genetically modified plants capable of synthesizing such toxins are disclosed, e. g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e.g. in the publications mentioned above. These insecticidal proteins contained in the genetically modified plants impart to the plants producing these proteins tolerance to harmful pests from all taxonomic groups of arthropods, especially to beetles (Coeloptera), two-winged insects (Diptera), and moths (Lepidoptera) and to nematodes (Nematoda).

Furthermore, in one embodiment, plants are also covered that are, e.g., by the use of recombinant DNA techniques and/or by breeding and/or otherwise selected for such traits, able to synthesize one or more proteins to increase the resistance or tolerance of those plants to bacterial, viral or fungal pathogens. The methods for producing such genetically modified plants are generally known to the person skilled in the art. The plants produced as described herein can also be stacked with other traits including, but not limited to, disease resistance, enhanced mineral profile, enhanced vitamin profile, enhanced oil profile (e.g., high oleic acid content), amino acid profile (e.g., high lysine corn), and other trait types known in the art.

Furthermore, in one embodiment, plants are also covered that are, e.g., by the use of recombinant DNA techniques and/or by breeding and/or by other means of selection, able to synthesize one or more proteins to increase the productivity (e.g. bio mass production, grain yield, starch content, oil content or protein content), tolerance to drought, salinity or other growth-limiting environmental factors or tolerance to pests and fungal, bacterial or viral pathogens of those plants.

Furthermore, in one embodiment, plants are also covered that contain, e.g., by the use of recombinant DNA techniques and/or by breeding and/or by other means of selection, a modified amount of substances of content or new substances of content, specifically to improve human or animal nutrition. Furthermore, plants are also covered that contain by the use of recombinant DNA techniques a modified amount of substances of content or new substances of content, specifically to improve raw material production.

Furthermore, in some embodiments, plants of the instant invention are also covered which are, *e*.*g*. by the use of recombinant DNA techniques and/or by breeding and/or otherwise selected for such traits, altered to contain increased amounts of vitamins and/or minerals, and/or improved profiles of nutraceutical compounds.

In one embodiment, plants of the invention tolerant to acetyl-Coenzyme A carboxylase inhibitors, relative to a wild-type plant, comprise an increased amount of, or an improved profile of, a compound selected from the group consisting of: glucosinolates (*e*.*g*., glucoraphanin (4-methylsulfinylbutyl-glucosinolate), sulforaphane, 3-indolylmethyl-glucosinolate (glucobrassicin), 1-methoxy-3-indolylmethyl-glucosinolate (neoglucobrassicin)); phenolics (*e.g.,* flavonoids (*e.g.,* quercetin, kaempferol), hydroxycinnamoyl derivatives (*e*.*g*., 1,2,2'-trisinapoylgentiobiose, 1,2-diferuloylgentiobiose, 1,2'-disinapoyl-2-feruloylgentiobiose, 3-O-caffeoyl-quinic (neochlorogenic acid)); and vitamins and minerals (*e*.*g*., vitamin C, vitamin E, carotene, folic acid, niacin, riboflavin, thiamine, calcium, iron, magnesium, potassium, selenium, and zinc).

In another embodiment, plants of the invention tolerant to acetyl-Coenzyme A carboxylase inhibitors, relative to a wild-type plant, comprise an increased amount of, or an improved profile of, a compound selected from the group consisting of: progoitrin; isothiocyanates; indoles (products of glucosinolate hydrolysis); glutathione; carotenoids such as beta-carotene, lycopene, and the xanthophyll carotenoids such as lutein and zeaxanthin; phenolics comprising the flavonoids such as the flavonols (*e*.*g*. quercetin, rutin), the flavans/tannins (such as the procyanidins comprising coumarin, proanthocyanidins, catechins, and anthocyanins); flavones; phytoestrogens such as coumestans, lignans, resveratrol, isoflavones *e*.*g*., genistein, daidzein, and glycitein; resorcyclic acid lactones; organosulphur compounds; phytosterols; terpenoids such as carnosol, rosmarinic acid, glycyrrhizin and saponins; chlorophyll; chlorphyllin, sugars, anthocyanins, and vanilla.

In other embodiments, plants of the invention tolerant to acetyl-Coenzyme A carboxylase inhibitors, relative to a wild-type plant, comprise an increased amount of, or an improved profile of, a compound selected from the group consisting of: vincristine, vinblastine, taxanes (*e*.*g*., taxol (paclitaxel), baccatin III, 10-desacetylbaccatin III, 10-desacetyl taxol, xylosyl taxol, 7-epitaxol, 7-epibaccatin III, 10-desacetylcephalomannine, 7-epicephalomannine, taxotere, cephalomannine, xylosyl cephalomannine, taxagifine, 8-benxoyloxy taxagifine, 9-acetyloxy taxusin, 9-hydroxy taxusin, taiwanxam, taxane Ia, taxane Ib, taxane Ic, taxane Id, GMP paclitaxel, 9-dihydro 13-acetylbaccatin III, 10-desacetyl-7-epitaxol, tetrahydrocannabinol (THC), cannabidiol (CBD), genistein, diadzein, codeine, morphine, quinine, shikonin, ajmalacine, serpentine, and the like.

The present invention also encompasses progeny of the plants of the invention as well as seeds derived from the herbicide-tolerant plants of the invention and cells derived from the herbicide-tolerant plants of the invention insofar as said progeny, seeds and cells comprise the mutagenized rice ACCase nucleic acid of the plants of the invention.

Plants hereof can be used to produce plant products. Thus, a method for preparing a descendant seed comprises planting a seed of a capable of producing a plant hereof, growing the resulting plant, and harvesting descendant seed thereof. Such a method can further comprise applying an ACCase-inhibiting herbicide composition to the resulting plant. Similarly, a method for producing a derived product from a plant hereof can comprise processing a plant part thereof to obtain a derived product. Such a method can be used to obtain a derived product that is any of, e.g., fodder, feed, seed meal, oil, or seed-treatment-coated seeds. Seeds, treated seeds, and other plant products obtained by such methods are useful products that can be commercialized.

In various embodiment, the present invention provides production of food products, consumer products, industrial products, and veterinary products from any of the plants described herein.

### Acetyl-Coenzyme A carboxylase Enzymes

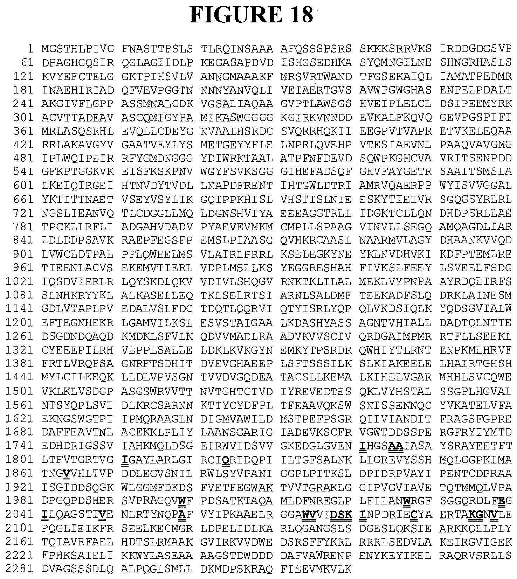

The present invention provides plants expressing acetyl-Coenzyme A carboxylase enzymes with amino acid sequences that differ from the amino acid sequence of the acetyl-Coenzyme A carboxylase enzyme found in the corresponding wild-type plant. For ease of understanding, the amino acid numbering system used herein will be the numbering system used for the acetyl-Coenzyme A carboxylase from *Alopecurus myosuroides* [Huds.] (also referred to as black grass). The mRNA sequence encoding the *A. myosuroides* acetyl-Coenzyme A carboxylase is available at GenBank accession number AJ310767 and the protein sequence is available at GenBank accession no. CAC84161. The number of the amino acid referred to will, be followed with *(Am)* to indicate the amino acid in the *Alopecurus myosuroides* sequence to which the amino acid corresponds. Figure 18 provides *Alopecurus myosuroides* acetyl-Coenzyme A carboxylase amino acid sequence (GenBank accession no. CAC84161). Amino acids that may be altered in the acetyl-Coenzyme A carboxylase enzymes of the invention are indicated in bold double underline, and Figure 19 depicts the amino acid sequence of wild-type *Oryza sativa* acetyl-Coenzyme A carboxylases aligned with *Alopecurus myosuroides* acetyl-Coenzyme A carboxylase with some critical residues denoted.

An acetyl-Coenzyme A carboxylase comprised by plants of the invention differs from the corresponding wild-type acetyl-Coenzyme A carboxylase at amino acid position 1,781*(Am)*. Wild-type *A*. *myosuroides* acetyl-Coenzyme A carboxylase has an isoleucine at position 1,781 *(Am)* (I1781). The 1,781*(Am)* ACCase mutants have a leucine at this position (I1781L).

In one embodiment, the acetyl-Coenzyme A carboxylase further differs from the corresponding wild-type acetyl-Coenzyme A carboxylase at amino acid position 1,785*(Am).* Wild-type *A. myosuroides* acetyl-Coenzyme A carboxylase has an alanine at position 1,785*(Am)* (A1785). The 1,785*(Am)* ACCase mutants will have an amino acid other than alanine at this position. Suitable examples of amino acids that may be found at this position in the acetyl-Coenzyme A carboxylase enzymes include, but are not limited to, glycine (A1785G). In one embodiment, the acetyl-Coenzyme A carboxylase enzyme will have a glycine at position 1,785*(Am)*.

In one embodiment, the acetyl-Coenzyme A carboxylase further differs from the corresponding wild-type acetyl-Coenzyme A carboxylase at amino acid position 1,786*(Am).* Wild-type *A. myosuroides* acetyl-Coenzyme A carboxylase has an alanine at position 1,786*(Am)* (A1786). The 1,786*(Am)* ACCase mutants will have an amino acid other than alanine at this position. Suitable examples of amino acids that maybe found at this position in the acetyl-Coenzyme A carboxylase enzymes include, but are not limited to, proline (A1786P). In one embodiment, the acetyl-Coenzyme A carboxylase enzyme will have a proline at position 1,786*(Am)*.

In one embodiment, the acetyl-Coenzyme A carboxylase further differs from the corresponding wild-type acetyl-Coenzyme A carboxylase at amino acid position 1,811*(Am).* Wild-type *A. myosuroides* acetyl-Coenzyme A carboxylase has an isoleucine at position 1,811 *(Am)* (I1811). The 1,811*(Am)* ACCase mutants will have an amino acid other than isoleucine at this position. Suitable examples of amino acids that may be found at this position in the acetyl-Coenzyme A carboxylase enzymes include, but are not limited to, asparagine (I1811N). In one embodiment, the acetyl-Coenzyme A carboxylase enzyme will have an asparagine at position 1,811*(Am).*

In one embodiment, the acetyl-Coenzyme A carboxylase further differs from the corresponding wild-type acetyl-Coenzyme A carboxylase at amino acid position 1,824*(am).* Wild-type *A. myosuroides* acetyl-Coenzyme A carboxylase has a glutamine at position *1,824(Am)* (Q1824). The 1,824*(Am)* ACCase mutants will have an amino acid other than glutamine at this position. Suitable examples of amino acids that may be found at this position in the acetyl-Coenzyme A carboxylase enzymes include, but are not limited to, proline (Q1824P). In one embodiment, the acetyl-Coenzyme A carboxylase enzyme will have a proline at position 1,824*(Am)*.

In one embodiment, the acetyl-Coenzyme A carboxylase further differs from the corresponding wild-type acetyl-Coenzyme A carboxylase at amino acid position 1,864*(Am).* Wild-type *A. myosuroides* acetyl-Coenzyme A carboxylase has a valine at position 1,864*(Am)* (V1864). The 1,864*(Am)* ACCase mutants will have an amino acid other than valine at this position. Suitable examples of amino acids that may be found at this position in the acetyl-Coenzyme A carboxylase enzymes include, but are not limited to, phenylalanine (V1864F). In one embodiment, the acetyl-Coenzyme A carboxylase enzyme will have a phenylalanine at position 1,864*(Am)*.

In one embodiment, the acetyl-Coenzyme A carboxylase further differs from the corresponding wild-type acetyl-Coenzyme A carboxylase at amino acid position 1,999*(Am).* Wild-type *A. myosuroides* acetyl-Coenzyme A carboxylase has a tryptophan at position 1,999*(Am)* (W1999). The 1,999*(Am)* ACCase mutants will have an amino acid other than tryptophan at this position. Suitable examples of amino acids that may be found at this position in the acetyl-Coenzyme A carboxylase enzymes include, but are not limited to, cysteine (W1999C) and glycine (W1999G). In one embodiment, the acetyl-Coenzyme A carboxylase enzyme will have a glycine at position 1,999*(Am)*.

In one embodiment, the acetyl-Coenzyme A carboxylase further differs from the corresponding wild-type acetyl-Coenzyme A carboxylase at amino acid position *2,027(Am).* Wild-type A. myosuroides acetyl-Coenzyme A carboxylase has a tryptophan at position 2,027*(Am)*(W2027). The 2,027*(Am)* ACCase mutants will have an amino acid other than tryptophan at this position. Suitable examples of amino acids that may be found at this position in the acetyl-Coenzyme A carboxylase enzymes include, but are not limited to, cysteine (W2027C) and arginine (W2027R). In one embodiment, the acetyl-Coenzyme A carboxylase enzyme will have a cysteine at position 2,027*(Am).*

In one embodiment, the acetyl-Coenzyme A carboxylase further differs from the corresponding wild-type acetyl-Coenzyme A carboxylase at amino acid position 2,039*(Am).* Wild-type *A. myosuroides* acetyl-Coenzyme A carboxylase has a glutamic acid at position 2,039*(Am)* (E2039). The 2,039*(Am)* ACCase mutants will have an amino acid other than glutamic acid at this position. Suitable examples of amino acids that may be found at this position in the acetyl-Coenzyme A carboxylase enzymes include, but are not limited to, glycine (E2039G). In one embodiment, the acetyl-Coenzyme A carboxylase enzyme will have an glycine at position 2,039*(Am)*.

In one embodiment, the acetyl-Coenzyme A carboxylase further differs from the corresponding wild-type acetyl-Coenzyme A carboxylase at amino acid position 2,041*(Am).* Wild-type *A. myosuroides* acetyl-Coenzyme A carboxylase has an isoleucine at position 2,041 *(Am)* (I2041). The 2,041 *(Am)* ACCase mutants will have an amino acid other than isoleucine at this position. Suitable examples of amino acids that may be found at this position in the acetyl-Coenzyme A carboxylase enzymes include, but are not limited to, asparagine (I2041N), or valine (I2041V). In one embodiment, the acetyl-Coenzyme A carboxylase enzyme will have an asparagine at position 2,041 *(Am).*

In one embodiment, the acetyl-Coenzyme A carboxylase further differs from the corresponding wild-type acetyl-Coenzyme A carboxylase at amino acid position 2,049*(Am)*. Wild-type *A. myosuroides* acetyl-Coenzyme A carboxylase has an valine at position 2,049*(Am)* (V2049). The 2,049*(Am)* ACCase mutants willhave an amino acid other than valine at this position. Suitable examples of amino acids that may be found at this position in the acetyl-Coenzyme A carboxylase enzymes include, but are not limited to, phenylalanine (V2049F), isoleucine (V2049I) and leucine (V2049L). In one embodiment, the acetyl-Coenzyme A carboxylase enzyme will have an phenylalanine at position 2,049*(Am)*.

In one embodiment, the acetyl-Coenzyme A carboxylase further differs from the corresponding wild-type acetyl-Coenzyme A carboxylase at amino acid position 2,059*(Am)*. Wild-type *A. myosuroides* acetyl-Coenzyme A carboxylase has an alanine at position 2,059*(Am)* (A2059). The 2,059*(Am)* ACCase mutants will have an amino acid other than an alanine at this position. Suitable examples of amino acids that may be found at this position in the acetyl-Coenzyme A carboxylase enzymes include, but are not limited to, valine (A2059V). In one embodiment, the acetyl-Coenzyme A carboxylase enzyme will have an valine at position 2,059*(Am).*

In one embodiment, the acetyl-Coenzyme A carboxylase further differs from the corresponding wild-type acetyl-Coenzyme A carboxylase at amino acid position 2,074*(Am).* Wild-type *A. myosuroides* acetyl-Coenzyme A carboxylase has a tryptophan at position 2,074*(Am)* (W2074). The 2,074*(Am)* ACCase mutants will have an amino acid other than tryptophan at this position. Suitable examples of amino acids that may be found at this position in the acetyl-Coenzyme A carboxylase enzymes include, but are not limited to, leucine (W2074L). In one embodiment, the acetyl-Coenzyme A carboxylase enzyme will have a leucine at 2,074*(Am).*

In one embodiment, the acetyl-Coenzyme A carboxylase further differs from the corresponding wild-type acetyl-Coenzyme A carboxylase at amino acid position 2,075*(Am).* Wild-type *A. myosuroides* acetyl-Coenzyme A carboxylase has a valine at position 2,075*(Am)* (V2075). The 2,075*(Am)* ACCase mutants will have an amino acid other than valine at this position. Suitable examples of amino acids that may be found at this position in the acetyl-Coenzyme A carboxylase enzymes include, but are not limited to, methionine (V2075M), leucine (V2075L) and isoleucine (V2075I). In one embodiment, the acetyl-Coenzyme A carboxylase enzyme will have a leucine at position 2,075*(Am).* In some embodiments, the acetyl-Coenzyme A carboxylase enzyme will have a valine at position 2,075*(Am)* and an additional valine immediately after position 2,075*(Am)* and before the valine at position 2,076*(Am)*, i.e., may have three consecutive valines where the wild-type enzyme has two.

In one embodiment, the acetyl-Coenzyme A carboxylase further differs from the corresponding wild-type acetyl-Coenzyme A carboxylase at amino acid position 2,078*(Am).* Wild-type *A. myosuroides* acetyl-Coenzyme A carboxylase has an aspartate at position 2,078*(Am)* (D2078). The 2,078*(Am)* ACCase mutants will have an amino acid other than aspartate at this position. Suitable examples of amino acids that may be found at this position in the acetyl-Coenzyme A carboxylase enzymes include, but are not limited to, lysine (D2078K), glycine (D2078G), or threonine (D2078T). In one embodiment, the acetyl-Coenzyme A carboxylase enzyme will have a glycine at position 2,078*(Am).*

In one embodiment, the acetyl-Coenzyme A carboxylase further differs from the corresponding wild-type acetyl-Coenzyme A carboxylase at amino acid position 2,079*(Am).* Wild-type *A. myosuroides* acetyl-Coenzyme A carboxylase has a serine at position 2,079*(Am)* (S2079). The 2,079*(Am)* ACCase mutants will have an amino acid other than serine at this position. Suitable examples of amino acids that may be found at this position in the acetyl-Coenzyme A carboxylase enzymes include, but are not limited to, phenylalanine (S2079F). In one embodiment, the acetyl-Coenzyme A carboxylase enzyme will have a phenylalanine at position 2,079*(Am).*

In one embodiment, the acetyl-Coenzyme A carboxylase further differs from the corresponding wild-type acetyl-Coenzyme A carboxylase at amino acid position 2,080*(Am)*. Wild-type *A. myosuroides* acetyl-Coenzyme A carboxylase has a lysine at position 2,080*(Am)* (K2080). The 2,080*(Am)* ACCase mutants will have an amino acid other than lysine at this position. Suitable examples of amino acids that may be found at this position in the acetyl-Coenzyme A carboxylase enzymes include, but are not limited to, glutamic acid (K2080E). In one embodiment, the acetyl-Coenzyme A carboxylase enzyme will have a glutamic acid at position 2,080*(Am)*. In another embodiment, the acetyl-Coenzyme A carboxylase enzymes will typically have a deletion of this position (Δ2080).

In one embodiment, the acetyl-Coenzyme A carboxylase further differs from the corresponding wild-type acetyl-Coenzyme A carboxylase at amino acid position 2,081 *(Am).* Wild-type *A. myosuroides* acetyl-Coenzyme A carboxylase has a isoleucine at position 2,091*(Am)* (I2081). The 2,081*(Am)* ACCase mutants will have an amino acid other than isoleucine at this position. In one embodiment, the acetyl-Coenzyme A carboxylase enzymes will typically have a deletion of this position (Δ2081).

In one embodiment, the acetyl-Coenzyme A carboxylase further differs from the corresponding wild-type acetyl-Coenzyme A carboxylase at amino acid position 2,088*(Am).* Wild-type *A. myosuroides* acetyl-Coenzyme A carboxylase has a cysteine at position 2,088*(Am)* (C2088). The 2,088*(Am)* ACCase mutants will have an amino acid other than cysteine at this position. Suitable examples of amino acids that may be found at this position in the acetyl-Coenzyme A carboxylase enzymes include, but are not limited to, arginine (C2088R), tryptophan (C2088W), phenylalanine (C2088F), glycine (C2088G), histidine (C2088H), lysine (C2088K), serine (C2088S), threonine (C2088T), leucine (C2088L) or valine (C2088V). In one embodiment, the acetyl-Coenzyme A carboxylase enzyme will have an arginine at position 2,088*(Am)*.

In one embodiment, the acetyl-Coenzyme A carboxylase further differs from the corresponding wild-type acetyl-Coenzyme A carboxylase at amino acid position 2,095 *(Am).* Wild-type *A, myosuroides* acetyl-Coenzyme A carboxylase has a lysine at position 2,095*(Am)* (K2095). The 2,095*(Am)* ACCase mutants will have an amino acid other than lysine at this position. Suitable examples of amino acids that may be found at this position in the acetyl-Coenzyme A carboxylase enzymes include, but are not limited to, glutamic acid (K2095E). In one embodiment, the acetyl-Coenzyme A carboxylase enzyme will have a glutamic acid at position 2,095*(Am)*.

In one embodiment, the acetyl-Coenzyme A carboxylase further differs from the corresponding wild-type acetyl-Coenzyme A carboxylase at amino acid position 2,096*(Am).* Wild-type *A. myosuroides* acetyl-Coenzyme A carboxylase has a glycine at position 2,096*(Am)* (G2096). The 2,096*(Am)* ACCase mutants will have an amino acid other than glycine at this position. Suitable examples of amino acids that may be found at this position. in the acetyl-Coenzyme A carboxylase enzymes include, but are not limited to, alanine (G2096A), or serine (G2096S). In one embodiment, the acetyl-Coenzyme A carboxylase enzyme will have an alanine at position 2,096*(Am)*.

In one embodiment, the acetyl-Coenzyme A carboxylase further differs from the corresponding wild-type acetyl-Coenzyme A carboxylase at amino acid position 2,098*(Am)*. Wild-type *A. myosuroides* acetyl-Coenzyme A carboxylase has a valine at position 2,098*(Am)* (V2098). The 2,098*(Am)* ACCase mutants will have an amino acid other than valine at this position. Suitable examples of amino acids that may be found at this position in the acetyl-Coenzyme A carboxylase enzymes include, but are not limited to, alanine (V2098 A), glycine (V2098G), proline (V2098P), histidine (V2098H), serine (V2098S) or cysteine (V2098C). In one embodiment, the acetyl-Coenzyme A carboxylase enzyme will have an alanine at position 2,098*(Am)*.

In one embodiment, the present invention encompasses acetyl-Coenzyme A carboxylase of an herbicide-tolerant plant of the invention which differs from the acetyl-Coenzyme A carboxylase of the corresponding wild-type plant only at position 1,781*(Am).* Further, acetyl-Coenzyme A carboxylases of herbicide-tolerant plants are described herein which differ at only one of the following positions: 1,785*(Am)*, 1,786*(Am)*, 1,811*(Am),* 1,824*(Am),* 1,864*(Am)*, 1,999*(Am)*, 2,027*(Am),* 2,039*(Am),* 2,041*(Am),* 2,049*(Am),* 2,059*(Am)*, 2,074*(Am), 2,075(Am),* 2,078*(Am),* 2,079*(Am)*, 2,080*(Am),* 2,081*(Am)*, 2,088*(Am)*, 2,095*(Am),* 2,096*(Am),* or 2,098*(Am)*.

Acetyl-Coenzyme A carboxylase enzymes described herein can have only one of the following substitutions: an isoleucine at position 2,075*(Am),* glycine at position 2,078*(Am),* arginine at position 2,088*(Am)*, glycine at position 2,039*(Am)*, valine at position 2,059*(Am)*, methionine at position 2,075*(Am),* duplication of position 2,075*(Am)* (i.e., an insertion of valine between 2,074*(Am)* and 2,075*(Am)*, or an insertion of valine between position 2,075*(Am)* and 2,076*(Am)*), deletion of amino acid position 2,080*(Am)*, glutamic acid at position 2,080*(Am)*, deletion of position 2,081*(Am)*, glutamic acid at position 2,095*(Am),* a glycine at position 1,785*(Am),* a proline at position 1,786*(Am)*, an asparagine at position 1,811*(Am),* a leucine at position 2,075*(Am),* a threonine at position 2,078*(Am),* a deletion at position 2,080*(Am)*, a tryptophan, phenylalanine, glycine, histidine, lysine, leucine, serine, threonine, or valine at position 2,088*(Am)*, a serine at position 2,096*(Am)*, an alanine at position 2,096*(Am)*, an alanine at position 2,098*(Am)*, a glycine at position 2,098(Am), an histidine at position 2,098*(Am)*, a proline at position 2,098*(Am),* a serine at position 2,098*(Am)*, a leucine at position 1,781 *(Am),* a threonine at position 1,781*(Am)*, a valine at position 1,781*(Am),* an alanine at position 1,781*(Am),* a glycine at position 1,999*(Am)*, a cysteine or arginine at position 2,027*(Am),* an arginine at position 2,027*(Am),* an asparagine at position 2,041*(Am),* or a valine at position 2,041*(Am)*

Nucleic acids encoding acetyl-Coenzyme A carboxylase polypeptide having only one of the following substitutions: isoleucine at position 2,075*(Am),* glycine at position *2,078(Am),* or arginine at position 2,088*(Am)* can be used transgenically. A monocot plant cell can be transformed with an expression vector construct comprising the nucleic acid encoding acetyl-Coenzyme A carboxylase polypeptide having only one of the following substitutions: isoleucine at position 2,078(*Am*), glycine at position 2,078(*Am*), or arginine at position 2,088*(Am).*

BEP clade plants are described herein comprising nucleic acids encoding acetyl-Coenzyme A carboxylase polypeptides having a substitution at only one amino acid position as described above.

Further, BET subclade plants are described herein comprising nucleic acids encoding acetyl-Coenzyme A carboxylase polypeptides having a substitution at only one amino acid position as described above.

Further, BET crop plants are described herein comprising nucleic acids encoding acetyl-Coenzyme A carboxylase polypeptides having a substitution at only one amino acid position as described above.

Further, monocot plants are described herein comprising nucleic acids encoding acetyl-Coenzyme A carboxylase polypeptides having a substitution at only one amino acid position as described above.

Further, monocot plants are described herein comprising nucleic acids encoding acetyl-Coenzyme A carboxylase polypeptides having a substitution at amino acid position 1,781*(Am)*, wherein the amino acid at position 1,781*(Am)* differs from that of wild type and is not leucine.

Further, monocot plants are described herein comprising nucleic acids encoding acetyl-Coenzyme A carboxylase polypeptides having a substitution at amino acid position 1,999*(Am)*, wherein the amino acid at position 1,999*(Am)* differs from that of wild type and is not cysteine.

Further, monocot plants are described herein comprising nucleic acids encoding acetyl-Coenzyme A carboxylase polypeptides having a substitution at amino acid position 2,027*(Am)*, wherein the amino acid at position 2,027*(Am)* differs from that of wild type and is not cysteine.

Further, monocot plants are described herein comprising nucleic acids encoding acetyl-Coenzyme A carboxylase polypeptides having a substitution at amino acid position 2,041 *(Am),* wherein the amino acid at position 2,041 *(Am)* differs from that of wild type and is not valine or asparagine.

Further, monocot plants are described herein comprising nucleic acids encoding acetyl-Coenzyme A carboxylase polypeptides having a substitution at amino acid position 2,096*(Am)*, wherein the amino acid at position 2,096*(Am)* differs from that of wild type and is not alanine.

The acetyl-Coenzyme A carboxylase enzymes comprised by the plants of the present invention may have an amino acid sequence that differs in more than one amino acid position from that of the acetyl-Coenzyme A carboxylase enzyme found in the corresponding wild-type plant. For example, an acetyl-Coenzyme A carboxylase may differ in 2, 3, 4, 5, 6, or 7 positions from that of the acetyl-Coenzyme A carboxylase enzyme found in the corresponding wild-type plant.

In one embodiment, the acetyl-Coenzyme A carboxylase differs from the corresponding wild-type acetyl-Coenzyme A carboxylase at amino acid position 1,781*(Am)* and at one or more additional amino acid positions. The mutagenized nucleic acid of the plants of the present invention encodes a rice plastidic acetyl-Coenzyme A carboxylase enzyme having a leucine at position 1,781*(Am).* In addition, the enzyme may also comprise one or more of a glycine at position 1,785*(Am)*, a proline at position 1,786*(Am)*, an asparagine at position 1,811 *(Am),* a proline at position 1,824*(Am),* a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041 *(Am),* a phenylalanine, isoleucine or leucine at position 2,049*(Am)*, a valine at position 2,059*(Am),* a leucine at position 2,074*(Am)*, a leucine, isoleucine, methionine, or an additional valine at position *2,075(Am),* a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am),* an arginine tryptophan, phenylalanine, glycine, histidine, lysine, serine, threonine, or valine at position 2,088*(Am)*, a glutamic acid at position 2,095*(Am)*, an alanine or serine at position 2,096*(Am),* and an alanine, glycine, proline, histidine, cysteine, or serine at position 2,098*(Am)*. In one embodiment, the acetyl-Coenzyme A carboxylase will have a leucine at position 1,781 *(Am)* and a glycine at position 1,785*(Am).* In one embodiment, the acetyl-Coenzyme A carboxylase will have a leucine at position 1,781*(Am)* and a proline at position 1,786*(Am)*. In one embodiment, the acetyl-Coenzyme A carboxylase will have a leucine at position 1,781*(Am)* and an asparagine at position 1,811*(Am).* In one embodiment, the acetyl-Coenzyme A carboxylase will have a leucine at position 1,781*(Am)* and a proline at position *1,824(Am).* In one embodiment, the acetyl-Coenzyme A carboxylase will have a leucine at position 1,781*(Am)* and a phenylalanine at position 1,864*(Am)*. In one embodiment, the acetyl-Coenzyme A carboxylase will have a leucine at position 1,781 *(Am)* and a cysteine or glycine at position 1,999*(Am)*. In one embodiment, the acetyl-Coenzyme A carboxylase will have a leucine at position 1,781*(Am)* and a cysteine or an arginine at position 2,027*(Am).* In one embodiment, the acetyl-Coenzyme A carboxylase will have a leucine at position 1,781*(Am)* and a glycine at position 2,039*(Am)*. In one embodiment, the acetyl-Coenzyme A carboxylase will have a leucine at position 1,781*(Am)* and an asparagine at position 2,041*(Am).* In one embodiment, the acetyl-Coenzyme A carboxylase will have a leucine at position 1,781*(Am)* and a phenylalanine, leucine or isoleucine at position 2,049*(Am)*. In one embodiment, the acetyl-Coenzyme A carboxylase will have a leucine at position 1,781*(Am)* and a valine at position 2,059*(Am)*. In one embodiment, the acetyl-Coenzyme A carboxylase will have a leucine at position 1,781 *(Am)* and a leucine at position *2,074(Am).* In one embodiment, the acetyl-Coenzyme A carboxylase will have a leucine at position 1,781 *(Am)* and a leucine, isoleucine methionine, or additional valine at position 2,075*(Am).* In one embodiment, the acetyl-Coenzyme A carboxylase will have a leucine at position 1,781*(Am)* and a glycine or threonine at position 2,078*(Am).* In one embodiment, the acetyl-Coenzyme A carboxylase will have a leucine at position 1,781 *(Am)* and a phenylalanine at position 2,079*(Am).* In one embodiment, the acetyl-Coenzyme A carboxylase will have a leucine at position 1,781*(Am)* and a glutamic acid or a deletion at position 2,080*(Am*). In one embodiment, the acetyl-Coenzyme A carboxylase will have a leucine at position 1,781*(Am)* and a deletion at position 2,081 *(Am).* In one embodiment, the acetyl-Coenzyme A carboxylase will have a leucine at position 1,781 *(Am)* and an arginine, tryptophan, phenylalanine, glycine, histidine, lysine, serine, threonine, or valine at position 2,088*(Am)*. In one embodiment, the acetyl-Coenzyme A carboxylase will have a leucine at position 1,781*(Am)* and a glutamic acid at position 2,095*(Am)*. In one embodiment, the acetyl-Coenzyme A carboxylase will have a leucine at position 1,781 *(Am)* and an alanine or serine at position *2,096(Am).* In one embodiment, the acetyl-Coenzyme A carboxylase will have a leucine at position 1,781*(Am)* and an alanine, glycine, proline, histidine, cysteine, or serine at position 2,098*(Am)*. In one embodiment, the acetyl-Coenzyme A carboxylase will have a leucine at position 1,781*(Am)*, a cysteine or arginine at position 2,027*(Am),* and an asparagine at position 2,041 *(Am).* In one embodiment, the acetyl-Coenzyme A carboxylase will have a leucine at position 1,781*(Am),* a cysteine or arginine at position 2,027*(Am)*, an asparagine at position 2,041*(Am),* and an alanine at position 2,096*(Am)*.

In a preferred embodiment, the acetyl-Coenzyme A carboxylase has a leucine at position 1,781*(Am)* and a proline at position 1,824*(Am)*; the acetyl-Coenzyme A carboxylase has a leucine at position 1,781*(Am)* and an arginine at position 2,027*(Am)*; or the acetyl-Coenzyme A carboxylase has a glycine at position *2,078(Am)* and a proline at position 1,824*(Am).*

In a more preferred embodiment, the acetyl-Coenzyme A carboxylase has a leucine at position 1,781*(Am)* and a phenylalanine at position 2,049*(Am)*.

In a most preferred embodiment, the acetyl-Coenzyme A carboxylase has a leucine at position 1,781 *(Am)* and a asparagine at position 2,041 *(Am);* the acetyl-Coenzyme A carboxylase has a leucine at position 1,781 *(Am)* and a cysteine at position 2,027*(Am)*; the acetyl-Coenzyme A carboxylase has a leucine at position 1,781 *(Am)* and a leucine at position 2,075*(Am)*; the acetyl-Coenzyme A carboxylase has a leucine at position *1,781(Am)* and a phenylalanine at position 1,864*(Am)*; the acetyl-Coenzyme A carboxylase has a leucine at position 1,781*(Am)* and an alanine at position 2,098*(Am)*; the acetyl-Coenzyme A carboxylase has a leucine at position 1,781*(Am)* and a glycine at position 2,098*(Am)*; or the acetyl-Coenzyme A carboxylase has a leucine at position 1,781*(Am)* and a duplication 2,075*(Am).*

### Nucleic acid molecules :

Further described herein are nucleic acid molecules that encode all or a portion of the acetyl-Coenzyme A carboxylase enzymes described above. Nucleic acid molecules of the invention may comprise a nucleic acid sequence encoding an amino acid sequence comprising a modified version of one or both of SEQ ID NOs: 2 and 3, wherein the sequence is modified such that the encoded protein comprises an isoleucine-to-leucine substitution at position 1,781*(Am)* and optionally one or more of the following: the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine or arginine; the amino acid at position 2,039*(Am)* is glycine; the amino acid at position 2,041*(Am)* is asparagine; the amino acid at position 2,049*(Am)* is phenylalanine, isoleucine or leucine; the amino acid at position 2,059*(Am)* is valine; the amino acid at position 2,074*(Am)* is leucine; the amino acid at position 2,075*(Am)* is leucine, isoleucine , methionine or additional valine; the amino acid at position 2,078*(Am)* is glycine, or threonine; the amino acid at position 2,079*(Am)* is phenylalanine; the amino acid at position 2,080*(Am)* is glutamic acid; the amino acid at position 2,080*(Am)* is deleted; the amino acid at position 2,081*(Am)* is deleted; the amino acid at position 2,088*(Am)* is arginine, tryptophan, phenylalanine, glycine, histidine, lysine, leucine, serine, threonine, or valine; the amino acid at position 2,095*(Am)* is glutamic acid; the amino acid at position 2,096*(Am)* is alanine, or serine; or the amino acid at position 2,098*(Am)* is alanine, glycine, proline, histidine, or serine. Further, nucleic acid molecules complementary to all or a portion of the coding sequences are described. In some embodiments, a nucleic acid molecule of the invention may encode an acetyl-Coenzyme A carboxylase having multiple differences from the wild type acetyl-Coenzyme A carboxylase as described above.

In one embodiment, the present invention encompasses a nucleic acid molecule encoding an acetyl-Coenzyme A carboxylase which differs from the acetyl-Coenzyme A carboxylase of the corresponding wild-type plant only at position 1,781*(Am).* Further, nucleic acid molecules are described herein encoding an acetyl-Coenzyme A carboxylase which differs from the acetyl-Coenzyme A carboxylase of the corresponding wild-type plant at only one of the following positions: 1,785*(Am)*, 1,786*(Am)*, 1,811*(Am)*, 1,824*(Am)*, 1,864*(Am)*, 1,999*(Am)*, 2,027*(Am)*, 2,039*(Am)*, *2,041(Am)*, 2,049*(Am)*, 2,059*(Am)*, *2,074(Am),* 2,075*(Am)*, 2,078*(Am)*, 2,079*(Am)*, 2,080*(Am)*, 2,081*(Am)*, 2,088*(Am)*, 2,095*(Am)*, *2,096(Am),* or 2,098*(Am).*

A nucleic acid molecule described herein can encode an acetyl-Coenzyme A carboxylase having only one of the following substitutions: isoleucine at position 2,075*(Am)*, glycine at position 2,078*(Am)*, arginine at position 2,088*(Am)*, glycine at position 2,039*(Am)*, valine at position 2,059*(Am)*, methionine, at position 2,075*(Am)*, duplication of position 2,075*(Am)* (i.e., an insertion of valine between 2,074*(Am)* and 2,075*(Am)*, or an insertion of valine between position 2,075*(Am)* and 2,076*(Am)*, deletion of amino acid position 2,088*(Am)*, glutamic acid at position 2,080*(Am)*, deletion of position 2,088*(Am)*, glutamic acid at position 2,095*(Am)*, a glycine at position 1,785*(Am)*, a proline at position 1,786*(Am)*, an asparagine at position 1,811*(Am)*, a leucine at position 2,075*(Am)*, a methionine at position 2,075*(Am)*, a threonine at position 2,078*(Am)*, a deletion at position 2,080*(Am)*, a deletion at position 2,081*(Am),* a tryptophan at position 2,088*(Am)*, a serine at position 2,096*(Am)*, an alanine at position 2,096*(Am)*, an alanine at position 2,098*(Am)*, a glycine at position 2,098*(Am)*, an histidine at position 2,098*(Am)*, a proline at position 2,098*(Am)*, or a serine at position 2,098*(Am)*, a leucine at pasition 1,781*(Am)*, a threonine at position 1,781*(Am)*, a valine at position *1,781(Am),* an alanine at position 1,781*(Am)*, a glycine at position 1,999*(Am)*, a cysteine at position 2,027*(Am)*, an arginine at position 2,027*(Am)*, an asparagine at position 2,041*(Am),* or a valine at position 2,041 *(Am).*

In one embodiment, a nucleic acid molecule of the invention may encode an acetyl-Coenzyme A carboxylase comprising a leucine at position 1,781*(Am)* and a cysteine or glycine at position 1,999*(Am).* In one embodiment, a nucleic acid molecule of the invention may encode an acetyl-Coenzyme A carboxylase comprising a leucine at position 1,781*(Am)* and a cysteine or arginine at position 2,027*(Am).* In one embodiment, a nucleic acid molecule of the invention may encode an acetyl-Coenzyme A carboxylase comprising a leucine at position 1,781 *(Am)* and an asparagine at position 2,041*(Am).* In one embodiment, a nucleic acid molecule of the invention may encode an acetyl-Coenzyme A carboxylase comprising a leucine at position 1,781*(Am)* and a phenylalanine, isoleucine or leucine at position 2,049*(Am)*. In one embodiment, a nucleic acid molecule of the invention may encode an acetyl-Coenzyme A carboxylase comprising a leucine at position 1,781*(Am)* and a leucine or isoleucine at position 2,075*(Am).* In one embodiment, a nucleic acid molecule of the invention may encode an acetyl-Coenzyme A carboxylase comprising a leucine at position 1,781*(Am)* and a glycine at position 2,078*(Am).* In one embodiment, a nucleic acid molecule of the invention may encode an acetyl-Coenzyme A carboxylase comprising a leucine at position 1,781*(Am)* and an arginine at position 2,088*(Am)*. In one embodiment, a nucleic acid molecule of the invention may encode an acetyl-Coenzyme A carboxylase comprising a leucine at position 1,781*(Am)* and an alanine at position 2,096*(Am)*. In one embodiment, a nucleic acid molecule of the invention may encode an acetyl-Coenzyme A carboxylase comprising a leucine at position 1,781*(Am)* and an alanine at position 2,098*(Am)*. In one embodiment, a nucleic acid molecule of the invention may encode an acetyl-Coenzyme A carboxylase comprising a leucine at position 1,781 *(Am),* a cysteine at position 2,027*(Am)*, and an asparagine at position 2,041 *(Am).* In one embodiment, a nucleic acid molecule of the invention may encode an acetyl-Coenzyme A carboxylase comprising a leucine at position 1,781*(Am),* a cysteine at position 2,027*(Am)*, an asparagine at position 2,041*(Am),* and an alanine at position 2,096*(Am).*

In a preferred embodiment, the invention includes a nucleic acid molecule encoding an acetyl-Coenzyme A carboxylase having a leucine at position 1,781*(Am)* and a proline at position 1,824*(Am)*; and a nucleic acid molecule encoding an acetyl-Coenzyme A carboxylase having a leucine at position 1,781*(Am)* and an arginine at position 2,027*(Am).*

In a more preferred embodiment, the invention includes a nucleic acid molecule encoding an acetyl-Coenzyme A carboxylase having a leucine at position 1,781*(Am)* and a phenylalanine at position 2,049*(Am).*

In a most preferred embodiment, the invention includes, a nucleic acid molecule encoding an acetyl-Coenzyme A carboxylase having a leucine at position 1,781*(Am)* and a asparagine at position 2,041*(Am)*; a nucleic acid molecule encoding an acetyl-Coenzyme A carboxylase having a leucine at position 1,781*(Am)* and a cysteine at position 2,027*(Am)*; a nucleic acid molecule encoding an acetyl-Coenzyme A carboxylase having a leucine at position 1,781*(Am)* and a leucine at position 2,075*(Am)*; a nucleic acid molecule encoding an acetyl-Coenzyme A carboxylase having a leucine at position 1,781 *(Am)* and a phenylalanine at position 1,864*(Am)*; a nucleic acid molecule encoding an acetyl-Coenzyme A carboxylase having a leucine at position 1,781*(Am)* and an alanine at position 2,098*(Am)*; anucleic acid molecule encoding an acetyl-Coenzyme A carboxylase having a leucine at position 1,788*(Am)* and a glycine at position 2,098*(Am)*; and a nucleic acid molecule encoding an acetyl-Coenzyme A carboxylase having a leucine at position 1,781 *(Am)* and a duplication 2,075*(Am).*

BEP clade plants are described herein comprising nucleic acids encoding acetyl-Coenzyme A carboxylase polypeptide having one or more substitutions as described above.

Further, BET subclade plant are described herein comprising nucleic acids encoding acetyl-Coenzyme A carboxylase polypeptide having one or more substitutions as described above.

Further, BET crop plants are described herein comprising nucleic acids encoding acetyl-Coenzyme A carboxylase polypeptide having one or more substitutions as described above.

Further, monocot plants are described herein comprising nucleic acids encoding acetyl-Coenzyme A carboxylase polypeptide having one or more substitutions as described above.

A nucleic acid molecule of the invention may be DNA, derived from genomic DNA or cDNA, or RNA. A nucleic acid molecule of the invention may be naturally occurring or may be synthetic. A nucleic acid molecule of the invention may be recombinant.

A nucleic acid molecule of the invention encodes an acetyl-Coenzyme A carboxylase enzyme in which the amino acid at position 1,781*(Am)* is leucine. Further, a nucleic acid molecule is described herein that is complementary to such a nucleic acid molecule of the invention. Such nucleic acid molecules include, but are not limited to, genomic DNA that serves as a template for a primary RNA transcription, a plasmid molecule encoding the acetyl-Coenzyme A carboxylase, as well as an mRNA encoding such an acetyl-Coenzyme A carboxylase.

Nucleic acid molecules of the invention may comprise non-coding sequences, which may or may not be transcribed. Non-coding sequences that may be included in the nucleic acid molecules of the invention include, but are not limited to, 5' and 3' UTRs, polyadenylation signals and regulatory sequences that control gene expression (e.g., promoters). Nucleic acid molecules of the invention may also comprise sequences encoding transit peptides, protease cleavage sites, covalent modification sites and the like. In one embodiment, nucleic acid molecules of the invention encode a chloroplast transit peptide sequence in addition to a sequence encoding an acetyl-Coenzyme A carboxylase enzyme.

In another embodiment, nucleic acid molecules of the invention may encode an acetyl-Coenzyme A carboxylase enzyme having at least 50%, 60%, 70%, 75%, 80%, 85%, 90%, 95% or more sequence identity to a modified version of one or both of SEQ ID NOs: 2 and 3, wherein the sequence is modified such that the encoded protein comprises an isoleucine-to-leucine substitution at position 1,781*(Am)* and optionally one or more of the following: the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine or arginine; the amino acid at position 2,039*(Am)* is glycine; the amino acid at position 2,041*(Am)* is asparagine; the amino acid at position 2,049*(Am)* is phenylalanine, leucine or isoleucine; the amino acid at position 2,059*(Am)* is valine; the amino acid at position 2,074*(Am)* is leucine; the amino acid at position 2,075*(Am)* is leucine, isoleucine or methionine or an additional valine; the amino acid at position 2,078*(Am)* is glycine, or threonine; the amino acid at position 2,079*(Am)* is phenylalanine; the amino acid at position 2,080*(Am)* is glutamic acid; the amino acid at position 2,080*(Am)* is deleted; the amino acid at position 2,081*(Am)* is deleted; the amino acid at position 2,088*(Am)* is arginine, tryptophan, phenylalanine, glycine, histidine, lysine, leucine, serine, threonine, or valine; the amino acid at position 2,095*(Am)* is glutamic acid; the amino acid at position 2,096*(Am)* is alanine, or serine; or the amino acid at position 2,098*(Am)* is alanine, glycine, proline, histidine, or serine. Further, nucleic acid molecules complementary to all or a portion of the coding sequences are described herein.

As used herein, "percent (%) sequence identity" is defined as the percentage of nucleotides or amino acids in the candidate derivative sequence identical with the nucleotides or amino acids in the subject sequence (or specified portion thereof), after aligning the sequences and introducing gaps, if necessary to achieve the maximum percent sequence identity, as generated by the program BLAST available at http:/ blast.ncbi.nlm.nih.gov/Blast.cgi with search parameters set to default values.

Further, nucleic acid molecules are described herein that hybridize to nucleic acid molecules encoding acetyl-Coenzyme A carboxylase of the invention as well as nucleic acid molecules that hybridize to the reverse complement of nucleic acid molecules encoding an acetyl-Coenzyme A carboxylase of the invention. Said nucleic acid molecules may hybridize to a nucleic acid molecule encoding one or more of a modified version of one or both of SEQ ID NOs: 2 and 3, wherein the sequence is modified such that the encoded protein comprises an isoleucine-to-leucine substitution at position 1,781*(Am)* and optionally one or more of the following: the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine or arginine; the amino acid at position 2,039*(Am)* is glycine; the amino acid at position 2,041 *(Am)* is asparagine; the amino acid at position 2,049*(Am)* is phenylalanine, isoleucine or leucine; the amino acid at position 2,059*(Am)* is valine; the amino acid at position 2,074*(Am)* is leucine; the amino acid at position 2,075*(Am)* is leucine, isoleucine or methionine or an additional valine; the amino acid at position 2,078*(Am)* is glycine, or threonine; the amino acid at position 2,079*(Am)* is phenylalanine; the amino acid at position 2,080*(Am)* is glutamic acid; the amino acid at position 2,080*(Am)* is deleted; the amino acid at position 2,081 *(Am)* is deleted; the amino acid at position 2,088*(Am)* is arginine, tryptophan, phenylalanine, glycine, histidine, lysine, leucine, serine, threonine, or valine; the amino acid at position 2,095*(Am)* is glutamic acid; the amino acid at position 2,096*(Am)* is alanine, or serine; or the amino acid at position 2,098*(Am)* is alanine, glycine, proline, histidine, or serine, as well as nucleic acid molecules complementary to all or a portion of the coding sequences, or the reverse complement of such nucleic acid molecules under stringent conditions. The stringency of hybridization can be controlled by temperature, ionic strength, pH, and the presence of denaturing agents such as formamide during hybridization and washing. Stringent conditions that may be used include those defined in Current Protocols in Molecular Biology, Vol. 1, Chap. 2.10, John Wiley & Sons, Publishers (1994) and Sambrook et al., Molecular Cloning, Cold Spring Harbor (1989) which relate to teaching stringent conditions.

Any of the mutants described above in a plasmid with a combination of the gene of interest can be used in transformation.

In one embodiment, the present invention provides expression vectors comprising nucleic acid molecules encoding any of the ACCase mutants described above.

Mutant ACCase nucleic acids and proteins encoded by such mutant ACCase nucleic acids as described above can be used as selectable markers.

Further, oligonucleotides are described herein that may be used as hybridization probes, sequencing primers, and/or PCR primers. Such oligonucleotides may be used, for example, to determine a codon sequence at a particular position in a nucleic acid molecule encoding an acetyl-Coenzyme A carboxylase, for example, by allele specific PCR. Such oligonucleotides maybe from about 15 to about 30, from about 20 to about 30, or from about 20-25 nucleotides in length.

### Test for double mutant ACCase genes "DBLM Assay":

(1) In a test population (of, e.g., at least 12 and preferably at least 20) whole rice plants containing 1 or 2 copies of a transgenic ACCase gene encoding an at-least-double-mutant ACCase (i.e. 1 min. and 2 max. chromosomal insertions of the transgenic ACCase gene to be tested),
   wherein the rice plants are TO ("T-zero") regenerants
   and in parallel with a control population of such plants to be used as untreated check plants;
(2) Application to the test population at 200 L / ha spray volume of a composition comprising Tepraloxydim (AI) and 1% Crop Oil Concentrate (COC), to provide an AI application rate equivalent to 50 g ha of Tepraloxydim (AI);
(3) Determining a phytotoxicity score for each test and check plant, based on a traditional plant injury rating system (e.g., evaluating visual evidence of herbicide burn, leaf morphology changes, wilt, yellowing, and other morphological characteristics, preferably according to a typical, at least-5-level injury rating scale);
(4) Analyzing the collected data to determine whether at least 75% of the plants in the test population exhibit an average phytotoxicity, i.e. increase in injury relative to check plants, of less than 10%; and
(5) Identifying a positive result so determined as demonstrating that the double-mutant ACCase provides an acceptable AIT.

### Herbicides

The present invention provides rice plants that are tolerant of concentrations of herbicide that normally inhibit the growth of wild-type plants. The plants are typically resistant to herbicides that interfere with acetyl-Coenzyme A carboxylase activity. Any herbicide that inhibits acetyl-Coenzyme A carboxylase activity can be used in conjunction with the plants of the invention. Suitable examples include, but are not limited to, cyclohexanedione herbicides, aryloxyphenoxy propionate herbicides, and phenylpyrazole herbicides. In some methods of controlling weeds and/or growing herbicide-tolerant plants, at least one herbicide is selected from the group consisting of sethoxydim, cycloxydim, tepraloxydim, haloxyfop, haloxyfop-P or a derivative of any of these herbicides.

Table 1 provides a list of cyclohexanedione herbicides (DIMs, also referred to as: cyclohexene oxime cyclohexanedione oxime; and CHD) that interfere with acetyl-Coenzyme A carboxylase activity and may be used in conjunction with the herbicide-tolerant plants of the invention. One skilled in the art will recognize that other herbicides in this class exist and maybe used in conjunction with the herbicide-tolerant plants of the invention. Also included in Table 1 is a list of aryloxyphenoxy propionate herbicides (also referred to as aryloxyphenoxy propanoate; aryloxyphenoxyalkanoate; oxyphenoxy; APP; AOPP; APA; APPA; FOP, note that these are sometime written with the suffix '-oic') that interfere with acetyl-Coenzyme A carboxylase activity and may be used in conjunction with the herbicide-tolerant plants of the invention. One skilled in the art will recognize that other herbicides in this class exist and may be used in conjunction with the herbicide-tolerant plants of the invention.

**Table 1**

| **ACCase Inhibitor** | **Class** | **Company** | **Examples of Synonyms and Trade Names** |
|---|---|---|---|
| alloxydim | DIM | BASF | Fervin, Kusagard, NP-48Na, BAS 9021 H, Carbodimedon, Zizalon |
| butroxydim | DIM | Syngenta | Falcon, ICI-A0500, Butroxydim |
| clethodim | DIM | Valent | Select, Prism, Centurion, RE-45601, Motsa |
| Clodinafop-propargyl | FOP | Syngenta | Discover, Topik, CGA 184 927 |
| clofop | FOP | | Fenofibric Acid, Alopex |
| cloproxydim | FOP | | |
| chlorazifop | FOP | | |
| cycloxydim | DIM | BASF | Focus, Laser, Stratos, BAS 517H |
| cyhalofop-butyl | FOP | Dow | Clincher, XDE 537, DEH 112, Barnstorm |
| diclofop-methyl | FOP | Bayer | Hoegrass, Hoelon, Illoxan, HOE 23408, Dichlorfop, Illoxan |
| fenoxaprop-P-ethyl | FOP | Bayer | Super Whip, Option Super, Exel Super, HOE-46360, Aclaim, Puma S, Fusion |
| fenthiaprop | FOP | | Taifun; Joker |
| fluazifop-P-butyl | FOP | Syngenta | Fusilade, Fusilade 2000, Fusilade DX, ICI-A 0009, ICI-A 0005, SL-236, IH-773B, TF-1169, Fusion |
| haloxyfop-etotyl | FOP | Dow | Gallant, DOWCO 453EE |
| haloxyfop-methyl | FOP | Dow | Verdict, DOWCO 453ME |
| haloxyfop-P-methyl | FOP | Dow | Edge, DE 535 |
| isoxapyrifop | FOP | | |
| Metamifop | FOP | Dongbu | NA |
| pinoxaden | DEN | Syngenta | Axial |
| profoxydim | DIM | BASF | Aura, Tetris, BAS 625H, Clefoxydim |
| propaquizafop | FOP | Syngenta | Agil, Shogun, Ro 17-3664, Correct |
| quizalofop-P-ethyl | FOP | DuPont | Assure, Assure II, DPX-Y6202-3, Targa Super, NC-302, Quizafop |
| quizalofop-P-tefuryl | FOP | Uniroyal | Pantera, UBI C4874 |
| sethoxydim | DIM | BASF | Poast, Poast Plus, NABU, Fervinal, NP-55, Sertin, BAS 562H, Cyethoxydim, Rezult |
| tepraloxydim | DIM | BASF | BAS 620H, Aramo, Caloxydim |
| tralkoxydim | DIM | Syngenta | Achieve, Splendor, ICI-A0604, Tralkoxydime, Tralkoxidym |
| trifop | FOP | | |

In addition to the herbicides listed above, other ACCAse-inhibitors can be used in conjunction with the herbicide-tolerant plants of the invention. For example, ACCase-inhibiting herbicides of the phenylpyrazole class, also known as DENs, can be used. An exemplary DEN is pinoxaden, which is a phenylpyrazoline-type member of this class. Herbicide compositions containing pinoxaden are sold under the brands Axial and Traxos.

The herbicidal compositions hereof comprising one or more acetyl-Coenzyme A carboxylase-inhibiting herbicides, and optionally other agronomic A.I.(s), *e*.*g*., one or more sulfonylureas (SUs) selected from the group consisting of amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, or one or more imidazolinones selected from the group of imazamox, imazethapyr, imazapyr, imazapic, combinations thereof, and their agriculturally suitable salts and esters, can be used in any agronomically acceptable format. For example, these can be formulated as ready-to-spray aqueous solutions, powders, suspensions; as concentrated or highly concentrated aqueous, oily or other solutions, suspensions or dispersions; as emulsions, oil dispersions, pastes, dusts, granules, or other broadcastable formats. The herbicide compositions can be applied by any means known in the art, including, for example, spraying, atomizing, dusting, spreading, watering, seed treatment, or co-planting in admixture with the seed. The use forms depend on the intended purpose; in any case, they should ensure the finest possible distribution of the active ingredients.

In other embodiments, where the optional A.I. includes an herbicide from a different class to which the plant(s) hereof would normally be susceptible, the plant to be used is selected from among those that further comprise a trait of tolerance to such herbicide. Such further tolerance traits can be provided to the plant by any method known in the art, e.g., including techniques of traditional breeding to obtain a tolerance trait gene by hybridization or introgression, of mutagenesis, and/or of transformation. Such plants can be described as having "stacked" traits.

In addition, any of the above acetyl-Coenzyme A carboxylase-inhibiting herbicides can be combined with one or more herbicides of another class, for example, any of the acetohydroxyacid synthase-inhibiting herbicides, EPSP synthase-inhibiting herbicides, glutamine synthase-inhibiting herbicides, lipid-or pigment-biosynthesis inhibitor herbicides, cell-membrane disrupter herbicides, photosynthesis or respiration inhibitor herbicides, or growth regulator or growth inhibitor herbicides known in the art. Non-limiting examples include those recited in Weed Science Society of America's Herbicide Handbook, 9th Edition edited by S.A. Senseman, copyright 2007. An herbicidal composition herein can contain one or more agricultural active ingredient(s) selected from the agriculturally-acceptable fungicides, strobilurin fungicides, insecticides (including nematicides), miticides, and molluscicides. Non-limiting examples include those recited in 2009 Crop Protection Reference (www.greenbook.net), Vance Publications.

In one embodiment of the invention, any of the above acetyl-Coenzyme A carboxylase-inhihiting herbicides are combined with herbicides which exhibit low damage to rice, whereby the rice tolerance to such herbicides may optionally be a result of genetic modifications of the crop plants. Examples of such herbicides are the acetohydroxyacid synthase-inhibiting herbicides imazamethabenz, imazamox, imazapic, imazapyr, imazaquin, imazethapyr, azimsulfuron, bensulfuron, chlorimuron, cyclosulfamuron, ethoxysulfuron, flucetosulfuron, halosulfuron, imazosulfuron, metsulfuron, orthosulfamuron, propyrisulfuron, pyrazosulfuron, bispyribac, pyrimisulfan or penoxsulam, the EPSP synthase-inhibiting herbicides glyphosate or sulfosate, the glutamine synthase-inhibiting herbicides glufosinate, glufosinate-P or bialaphos, the lipid biosynthesis inhibitor herbicides benfuresate, molinate or thiobencarb, the photosynthesis inhibitor herbicides bentazon, paraquat, prometryn or propanil, the bleacher herbicides benzobicyclone, clomazone or tefuryltrione, the auxin herbicides 2,4-D, fluroxypyr, MCPA, quinclorac, quinmerac or triclopyr, the microtubule inhibitor herbicide pendimethalin, the VLCFA inhibitor herbicides anilofos, butachlor, fentrazamide, ipfencarbazone, mefenacet, pretilachlor, acetochlor, metolachlor or S-metolachlor or the protoporphyrinogen-IX-oxidase inhibitor herbicides carfentrazone, oxadiazon, oxyfluorfen, pyraclonil or saflufenacil.

In one embodiment of the invention, any of the above acetyl-Coenzyme A carboxylase-inhibiting herbicides are combined with herbicides which exhibit low damage to cereals such as wheat, barley or rye, whereby the cereals tolerance to such herbicides may optionally be a result of genetic modifications of the crop plants. Examples of such herbicides are the acetohydroxyacid synthase-inhibiting herbicides imazamethabenz, imazamox, imazapic, imazapyr, imazaquin, imazethapyr, amidosulfuron, chlorsulfuron, flucetosulfuron, flupyrsulfuron, iodosulfuron, mesosulfuron, metsulfuron, sulfosulfuron, thifensulfuron, triasulfuron, tribenuron, tritosulfuron, florasulam, pyroxsulam, pyrimisulfan, flucarbazone, propoxycarbazone or thiencarbazone, the EPSP synthase-inhibiting herbicides glyphosate or sulfosate, the glutamine synthase-inhibiting herbicides glufosinate, glufosinate-P or bialaphos, the lipid biosynthesis inhibitor herbicides prosulfocarb, the photosynthesis inhibitor herbicides bentazon, chlorotoluron, isoproturon, ioxynil, bromoxynil, the bleacher herbicides diflufenican, flurtamone, picolinafen or pyrasulfotole, the auxin herbicides aminocyclopyrachlor, aminopyralid, 2,4-D, dicamba, fluroxypyr, MCPA, clopyralid, MCPP, or MCPP-P, the microtubule inhibitor herbicides pendimethalin or trifluralin, the VLCFA inhibitor herbicide flufenacet, or the protoporphyrinogen-IX-oxidase inhibitor herbicides bencarbazone, carfentrazone or saflufenacil, or the herbicide difenzoquat.

In one embodiment of the invention, any of the above acetyl-Coenzyme A carboxylase-inhibiting herbicides are combined with herbicides which exhibit low damage to turf, whereby the turf tolerance to such herbicides may optionally be a result of genetic modifications of the crop plants. Examples of such herbicides are the acetohydroxyacid synthase-inhibiting herbicides imazamethabenz, imazamox, imazapic, imazapyr, imazaquin, imazethapyr, flazasulfuron, foramsulfuron, halosulfuron, trifloxysulfuron, bispyribac or thiencarbazone, the EPSP synthase-inhibiting herbicides glyphosate or sulfosate, the glutamine synthase-inhibiting herbicides glufosinate, glufosinate-P or bialaphos, the photosynthesis inhibitor herbicides atrazine or bentazon, the bleacher herbicides mesotrione, picolinafen, pyrasulfotole or topramezone, the auxin herbicides aminocyclopyrachlor, aminopyralid, 2,4-D, 2,4-DB, clopyralid, dicamba, dichlorprop, dichlorprop-P, fluroxypyr, MCPA, MCPB, MCPP, MCPP-P, quinclorac, quinmerac or trichlopyr, the microtubule inhibitor herbicide pendimethalin, the VLCFA inhibitor herbicides dimethenamide, dimethenamide-P or ipfencarbazone, the protoporphyrinogen-IX-oxidase inhibitor herbicides saflufenacil or sulfentrazone, or the herbicide indaziflam.

Furthermore, any of the above acetyl-Coenzyme A carboxylase-inhibiting herbicides can be combined with safeners. Safeners are chemical compounds which prevent or reduce damage on useful plants without having a major impact on the herbicidal action of the herbicides towards unwanted plants. They can be applied either before sowings (e. g. on seed treatments, shoots or seedlings) or in the pre-emergence application or post-emergence application of the useful plant. The safeners and the aforementioned herbicides can be applied simultaneously or in succession. Suitable safeners are e. g. (quinolin-8-oxy)acetic acids, 1-phenyl-5-haloalkyl-1H-1,2,4-triazol-3-carboxylic acids, 1-phenyl-4,5-dihydro-5-alkyl-1H-pyrazol-3,5-dicarboxylic acids, 4,5-dihydro-5,5-diaryl-3-isoxazol carboxylic acids, dichloroacetamides, alpha-oximinophenylacetonitriles, acetophenonoximes, 4,6-dihalo-2-phenylpyrimidines, N-[[4-(aminocarbonyl)phenyl]sulfonyl]-2-benzoic amides, 1,8-naphthalic anhydride, 2-halo-4-(haloalkyl)-5-thiazol carboxylic acids, phosphorthiolates and N-alkyl-O-phenylcarbamates. Examples of saferners are benoxacor, cloquintocet, cyometrinil, cyprosulfamide, dichlormid, dicyclonon, dietholate, fenchlorazole, fenclorim, flurazole, fluxofenim, furilazole, isoxadifen, mefenpyr, mephenate, naphthalic anhydride, oxabetrinil, 4-(dichloroacetyl)-1-oxa-4-azaspiro[4.5-]decane (MON4660, CAS 71526-07-3) and 2,2,5-trimethyl-3-(dichloroacetyl)-1,3-oxazolidine (R-29148, CAS 52836-31-4).

In some embodiments, an herbicidal composition hereof can comprise, e.g., a combination of: auxinic herbicide(s), e.g., dicamba; AHAS-inhibitor(s), e.g., imidazolinone(s) and/or sulfonylurea(s); ACCase-inhibitor(s); EPSPS inhibitors), e.g., glyphosate; glutamine synthetase inhibitor(s), e.g.., glufosinate; protoporphyrinogen-IX oxidase (PPO) inhibitors), e.g., saflufenacil; fungicide(s), e.g., strobilurin fungicide(s) such as pyraclostrobin; and the like. In some embodiments, an herbicidal composition hereof can comprise, e.g., a combination of auxinic herbicide(s), e.g., dicamba; a microtubule inhibitor herbicide, e.g., pendimethalin and strobilurin fungicide(s) such as pyraclostrobin(s). An herbicidal composition will be selected according to the tolerances of a plant hereof, and the plant can be selected from among those having stacked tolerance traits.

The herbicides individually and/or in combination as described herein can be used as pre-mixes or tank mixes. Such herbicides can also be incorporated into an agronomically acceptable compositions.

Those skilled in the art will recognize that some of the above mentioned herbicides and/or safeners are capable of forming geometrical isomers, for example E/Z isomers. It is possible to use both, the pure isomers and mixtures thereof, in the compositions used according to the invention. Furthermore, some of the above mentioned herbicides and/or safeners have one or more centers of chirality and, as a consequence, are present as enantiomers or diastereomers. It is possible to use both, the pure enantiomers and diastereomers and their mixtures, in the compositions used according to the invention. In particular, some of the aryloxyphenoxy propionate herbicides are chiral, and some of them are commonly used in enantiomerically enriched or enantiopure form, e. g. clodinafop, cyhalofop, fenoxaprop-P, fluazifop-P, haloxyfop-P, metamifop, propaquizafop or quizalofop-P. As a further example, glufosinate, may be used in enantiomerically enriched or enantiopure form, also known as glufosinate-P.

Those skilled in the art will recognize that any derivative of the above mentioned herbicides and/or safeners can be used in the practice of the invention, for example agriculturally suitable salts and esters.

The herbicides and or safeners, or the herbicidal compositions comprising them, can be used, for example, in the form of ready-to-spray aqueous solutions, powders, suspensions, also highly concentrated aqueous, oily or other suspensions or dispersions, emulsions, oil dispersions, pastes, dusts, materials for broadcasting, or granules, by means of spraying, atomizing, dusting, spreading, watering or treatment of the seed or mixing with the seed. The use forms depend on the intended purpose; in any case, they should ensure the finest possible distribution of the active ingredients used according to the invention.

The herbicidal compositions comprise an herbicidal effective amount of at least one of the acetyl-Coenzyme A carboxylase-inhibiting herbicides and potentially other herbicides and/or safeners and auxiliaries which are customary for the formulation of crop protection agents.

Examples of auxiliaries customary for the formulation of crop protection agents are inert auxiliaries, solid carriers, surfactants (such as dispersants, protective colloids, emulsifiers, wetting agents and tackifiers), organic and inorganic thickeners, bactericides, antifreeze agents, antifoams, optionally colorants and, for seed formulations, adhesives. The person skilled in the art is sufficiently familiar with the recipes for such formulations.

Examples of thickeners (i.e. compounds which impart to the formulation modified flow properties, i.e. high viscosity in the state of rest and low viscosity in motion) are polysaccharides, such as xanthan gum (Kelzan® from Kelco), Rhodopol® 23 (Rhone Poulenc) or Veegum® (from R.T. Vanderbilt), and also organic and inorganic sheet minerals, such as Attaclay® (from Engelhardt).

Examples of antifoams are silicone emulsions (such as, for example, Silikon^{®} SRE, Wacker or Rhodorsil® from Rhodia), long-chain alcohols, fatty acids, salts of fatty acids, organofluorine compounds and mixtures thereof.

Bactericides can be added for stabilizing the aqueous herbicidal formulations. Examples of bactericides are bactericides based on diclorophen and benzyl alcohol hemiformal (Proxel® from ICI or Acticide® RS from Thor Chemie and Kathon® MK from Rohm & Haas), and also isothiazolinone derivates, such as alkylisothiazolinones and benzisothiazolinones (Acticide MBS from Thor Chemie).

Examples of antifreeze agents are ethylene glycol, propylene glycol, urea or glycerol.

Examples of colorants are both sparingly water-soluble pigments and water-soluble dyes. Examples which may be mentioned are the dyes known under the names Rhodamin B, C.I. Pigment Red 112 and C.I. Solvent Red 1, and also pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

Examples of adhesives are polyvinylpyrrolidone, polyvinyl acetate, polyvinyl alcohol and tylose.

Suitable inert auxiliaries are, for example, the following: mineral oil fractions of medium to high boiling point, such as kerosene and diesel oil, furthermore coal tar oils and oils of vegetable or animal origin, aliphatic, cyclic and aromatic hydrocarbons, for example paraffin, tetrahydronaphthalene, alkylated naphthalenes and their derivatives, alkylated benzenes and their derivatives, alcohols such as methanol, ethanol, propanol, butanol and cyclohexanol, ketones such as cyclohexanone or strongly polar solvents, for example amines such as N-methylpyrrolidone, and water.

Suitable carriers include liquid and solid carriers. Liquid carriers include e.g. non-aqeuos solvents such as cyclic and aromatic hydrocarbons, e.g. paraffins, tetrahydronaphthalene, alkylated naphthalenes and their derivatives, alkylated benzenes and their derivatives, alcohols such as methanol, ethanol, propanol, butanol and cyclohexanol, ketones such as cyclohexanone, strongly polar solvents, e.g. amines such as N-methylpyrrolidone, and water as well as mixtures thereof. Solid carriers include e.g. mineral earths such as silicas, silica gels, silicates, talc, kaolin, limestone, lime, chalk, bole, loess, clay, dolomite, diatomaceous earth, calcium sulfate, magnesium sulfate and magnesium oxide, ground synthetic materials, fertilizers such as ammonium sulfate, ammonium phosphate, ammonium nitrate and ureas, and products of vegetable origin, such as cereal meal, tree bark meal, wood meal and nutshell meal, cellulose powders, or other solid carriers.

Suitable surfactants (adjuvants, wetting agents, tackifiers, dispersants and also emulsifiers) are the alkali metal salts, alkaline earth metal salts and ammonium salts of aromatic sulfonic acids, for example lignosulfonic acids (e.g. Borrespers-types, Borregaard), phenolsulfonic acids, naphthalenesulfonic acids (Morwet types, Akzo Nobel) and dibutylnaphthalenesulfonic acid (Nekal types, BASF AG), and of fatty acids, alkyl- and alkylarylsulfonates, alkyl sulfates, lauryl ether sulfates and fatty alcohol sulfates, and salts of sulfated hexa-, hepta- and octadecanols, and also of fatty alcohol glycol ethers, condensates of sulfonated naphthalene and its derivatives with formaldehyde, condensates of naphthalene or of the naphthalenesulfonic acids with phenol and formaldehyde, polyoxyethylene octylphenol ether, ethoxylated isooctyl-, octyl- or nonylphenol, alkylphenyl or tributylphenyl polyglycol ether, alkylaryl polyether alcohols, isotridecyl alcohol, fatty alcohol/ethylene oxide condensates, ethoxylated castor oil, polyoxyethylene alkyl ethers or polyoxypropylene alkyl ethers, lauryl alcohol polyglycol ether acetate, sorbitol esters, lignosulfite waste liquors and proteins, denaturated proteins, polysaccharides (e.g. methylcellulose), hydrophobically modified starches, polyvinyl alcohol (Mowiol types Clariant), polycarboxylates (BASF AG, Sokalan types), polyalkoxylates, polyvinylamine (BASF AG, Lupamine types), polyethyleneimine (BASF AG, Lupasol types), polyvinylpyrrolidone and copolymers thereof.

Powders, materials for broadcasting and dusts can be prepared by mixing or concomitant grinding the active ingredients together with a solid carrier.

Granules, for example coated granules, impregnated granules and homogeneous granules, can be prepared by binding the active ingredients to solid carriers.

Aqueous use forms can be prepared from emulsion concentrates, suspensions, pastes, wettable powders or water-dispersible granules by adding water. To prepare emulsions, pastes or oil dispersions, the herbicidal compositions, either as such or dissolved in an oil or solvent, can be homogenized in water by means of a wetting agent, tackifier, dispersant or emulsifier. Alternatively, it is also possible to prepare concentrates comprising active compound, wetting agent, tackifier, dispersant or emulsifier and, if desired, solvent or oil, which are suitable for dilution with water.

### Methods of controlling weeds

Herbicide-tolerant plants of the invention may be used in conjunction with an herbicide to which they are tolerant. Herbicides may be applied to the plants of the invention using any techniques known to those skilled in the art. Herbicides may be applied at any point in the plant cultivation process. For example, herbicides may be applied pre-planting, at planting, pre-emergence, post-emergence or combinations thereof.

Herbicide compositions hereof can be applied, e.g., as foliar treatments, soil treatments, seed treatments, or soil drenches. Application can be made, e.g., by spraying, dusting, broadcasting, or any other mode known useful in the art.

In one embodiment, herbicides may be used to control the growth of weeds that may be found growing in the vicinity of the herbicide-tolerant plants invention. In embodiments of this type, an herbicide may be applied to a plot in which herbicide-tolerant plants of the invention are growing in vicinity to weeds. An herbicide to which the herbicide-tolerant plant of the invention is tolerant may then be applied to the plot at a concentration sufficient to kill or inhibit the growth of the weed. Concentrations of herbicide sufficient to kill or inhibit the growth of weeds are known in the art.

Having now described the present invention in detail, the same will be more clearly understood by reference to the following examples, which are included herewith for purposes of illustration only and are not intended to be limiting of the invention.

### Use of Tissue Culture for Selection of Herbicide

Herbicide tolerant crops offer farmers additional options for weed management. Currently, there are genetically modified (GMO) solutions available in some crop systems. Additional, mutational techniques have been used to select for altered enzyme, activities or structures that confer herbicide resistance such as the current CLEARFIELD° solutions from BASF. In the US, CLEARFIELD Rice is the premier tool for managing red rice in infested areas (USDA-ARS, 2006); however, gene flow between red rice and CLEARFIELD Rice represents a considerable risk for the AHAS tolerance since out-crossing, has been reported at up to 170 F1 hybrids/ha (Shivrain et al, 2007). Stewardship guidelines including, amongst many other aspects, alternation non CLEARFIELD Rice can limit CLEARFIELD Rice market penetration. The generation of cultivated rice with tolerance to a different mode of action (MOA) graminicides would reduce these risks and provide more tools for weed management.

One enzyme that is already a target for many different graminaceous herbicides is acetyl CoA carboxylase (ACCase, EC 6.4.1.2), which catalyzes the first committed step in fatty acid (FA) biosynthesis. Aryloxyphenoxypropionate (APP or FOP) and cyclohexanedione (CHD or DIM) type herbicides are used post-emergence in dicot crops, with the exception of cyhalofop-butyl which is selective in rice to control grass weeds. Furthermore, most of these herbicides have relatively low persistence in soil and provide growers with flexibility for weed control and crop rotation. Mutations in this enzyme are known that confer tolerance to specific sets of FOPS and/or DIMS (Liu et al, 2007; Delye et al, 2003, 2005).

Tissue culture offers an alternative approach in that single clumps of callus represent hundreds or even thousands of cells, each of which can be selected for a novel trait such as herbicide resistance (Jain, 2001). Mutations arising spontaneously in tissue culture or upon some kind of induction can be directly selected in culture and mutated events selected.

The exploitation of somaclonal variation that is inherent to *in vitro* tissue culture techniques has been a successful approach to selectively generate mutations that confer DIM and FOP tolerance in corn (Somers, 1996; Somers et al., 1994; Marshal et al., 1992; Parker et al., 1990) and in seashore paspalum (Heckart et al, 2009). In the case of maize, the efficiencies of producing regenerable events can be calculated. In Somers et al, 1994, sethoxydim resistant maize plants were obtained using tissue culture selection. They utilized 100 g of callus and obtained 2 tolerant lines following stepwise selection at 0.5, 1.0, 2.0, 5.0 and 10 µM sethoxydim. A calculated mutation rate in their protocol would be 2 lines/100 g of callus or 0.02 lines/g.

In the case of seashore paspalum, Heckert directly utilized a high level of sethoxydim and recovered 3 regenerable lines in approx 10,000 callus pieces or, essentially, a 0.03 % rate. While not comparable, these numbers will be later used for comparison with rice tissue culture mutagenesis. In the maize work, calli were constantly culled at each selection stage with only growing callus being transferred; however, in the case of seashore paspalum, all calli were transferred at each subculture. ACCase genes as selectable markers:

Plant transformation involves the use of selectable marker genes to identify the few transformed cells or individuals from the larger group of non-transformed cells or individuals. Selectable marker genes exist, but they are limited in number and availability. Alternative marker genes are required for stacking traits. In addition, the use of a selectable marker gene that confers an agronomic trait (i.e. herbicide resistance) is often desirable. ACCase genes can be used as selectable markers that can be added to the current limited suite of available selectable marker genes. Any of the mutants described herein can be introduced into a plasmid with a gene of interest and transformed into the whole plant, plant tissue or plant cell for use as selectable markers. A detailed method is outlined in example 7 below. The selectable markers described herein maybe utilized to produce events that confer field tolerance to a given group of herbicides and other where cross protection has been shown (i.e., FOP's).

Modern, high throughput plant transformation systems require an effective selectable marker system; however, there is a limited number available that are acceptable in the market. Therefore, selection systems which also convey a commercial trait are always valuable. The system described herein is an effective selection system in/for plant cells which also encode for an herbicide tolerance trait suitable for use in any monocotyledonous crop.

A method for selecting a transformed plant is described herein comprising introducing a nucleic acid molecule encoding a gene of interest into a plant cell, wherein the nucleic acid molecule further encodes a mutant acetyl-Coenzyme A carboxylase (ACCase) in which the amino acid sequence differs from an amino acid sequence of an ACCase of a corresponding wild-type rice plant at one amino acid position; and contacting the plant cells with an ACCase inhibitor to obtain the transformed plant, wherein said mutant ACCase confers upon the transformed plant increased herbicide tolerance as compared to the corresponding wild-type variety of the plant when expressed therein.

Further, a method of marker-assisted breeding is described herein, the method comprising breeding any plant of the invention with a second plant; and contacting progeny of the breeding step with an ACCase inhibitor to obtain the progeny comprising said mutant ACCase; wherein said mutant ACCase confers upon the progeny plant increased herbicide tolerance as compared to the second plant.

A single ACCase gene can be linked to a single gene of interest. The ACCase gene may be linked upstream or downstream of the gene of interest.

ACCase nucleic acid and protein as described above can be used in diagnostic assays. The diagnostic uses for selectable markers described herein can be employed to identify ACCase gene. Diagnostic methods can include PCR methodologies, proteins assays, labeled probes, and any other standard diagnostic methods known in the art.

### EXAMPLES

### EXAMPLE 1

### Tissue culture conditions

An *in vitro* tissue culture mutagenesis assay has been developed to isolate and characterize plant tissue (e.g., rice tissue) that is tolerant to acetyl-Coenzyme A carboxylase inhibiting herbicides, e.g., tepraloxydim, cycloxydim, and sethoxydim. The assay utilizes the somaclonal variation that is found in *in vitro* tissue culture. Spontaneous mutations derived from somaclonal variation can be enhanced by chemical mutagenesis and subsequent selection in a stepwise manner, on increasing concentrations of herbicide.

Tissue culture conditions for encouraging growth of friable, embryogenic rice callus that is regenerable are described herein, Calli were initiated from 4 different rice cultivars encompassing both Japonica (Taipei 309, Nipponbare, Koshihikari) and Indica (Indica 1) varieties. Dehusked seed were surface sterilized in 70% ethanol for approximately 1 min followed by 20% commercial Clorox bleach for 20 minutes. Seeds were rinsed with sterile water and plated on callus induction media. Various callus induction media were tested. The ingredient lists for the media tested are presented in Table 2.

**Table 2**

| **Ingredient** | **Supplier** | **R001M** | **R025M** | **R026M** | **R327M** | **R008M** | **MS711 R** |
|---|---|---|---|---|---|---|---|
| B5 Vitamins | Sigma | | | | | 1.0 X | |
| MS salts | Sigma | | | 1.0 X | 1.0 X | 1.0 X | 1.0 X |
| MS Vitamins | Sigma | | | 1.0 X | 1.0 X | | |
| N6 salts | Phytotech | 4.0 g/L | 4.0g/L | | | | |
| N6 vitamins | Phytotech | 1.0 X | 1.0 X | | | | |
| L-Proline | Sigma | 2.9 g/L | 0.5 g/L | | | | 1.2 g/L |
| Casamino Acids | BD | 0.3 g/L | 0.3 g/L | 2 g/L | | | |
| Casein Hydrolysate | Sigma | | | | | | 1.0 g/L |
| L-Asp Monohydrate | Phytotech | | | | | | 150 mg/L |
| Nicotinic Acid | Sigma | | | | | | 0.5 mg/L |
| Pyridoxine HCl | Sigma | | | | | | 0.5 mg/L |
| Thiamine HCl | Sigma | | | | | | 1.0 mg/L |
| Myo-inositol | Sigma | | | | | | 100 mg/L |
| MES | Sigma | 500 mg/L | 500 mg/L | 500 mg/L | 500 mg/L | 500 mg/L | 500 mg/L |
| Maltose | VWR | 30 g/L | 30 g/L | 30 g/L | 30 g/L | | |
| Sorbitol | Duchefa | | | 30 g/L | | | |
| Sucrose | VWR | | | | | 10 g/L | 30 g/L |
| NAA | Duchefa | | | | | 50 µg/L | |
| 2,4-D | Sigma | 2.0 mg/l | | | | | 1.0 mg/L |
| MgCl₂·6H₂O | VWR | | | | | 750 mg/L | |
| →pH | | 5.8 | 5.8 | 5.8 | 5.8 | 5.8 | 5.7 |
| Gelrite | Duchefa | 4.0 g/L | | | | 2.5 g/L | |
| Agarose Type1 | Sigma | | 7.0 g/L | 10 g/L | 10 g/L | | |
| →Autoclave | | 15 min | 15 min | 15 min | 15 min | 15 min | 20 min |
| Kinetin | Sigma | | 2.0 mg/L | 2.0 mg/L | | | |
| NAA | Duchefa | | 1.0 mg/L | 1.0 mg/L | | | |
| ABA | Sigma | | 5.0 mq/L | | | | |
| Cefotaxime | Duchefa | | 0.1 g/L | 0.1 g/L | 0.1 g/L | | |
| Vancomycin | Duchefa | | 0.1 g/L | 0.1 g/L | 0.1 g/L | | |
| G418 Disulfate | Sigma | | 20 mg/L | 20 mg/L | 20 mg/L | | |

R001M callus induction media was selected after testing numerous variations. Cultures were kept in the dark at 30°C. Embryogenic callus was subcultured to fresh media after 10-14 days.

### EXAMPLE 2

### Selection of herbicide-tolerant calli

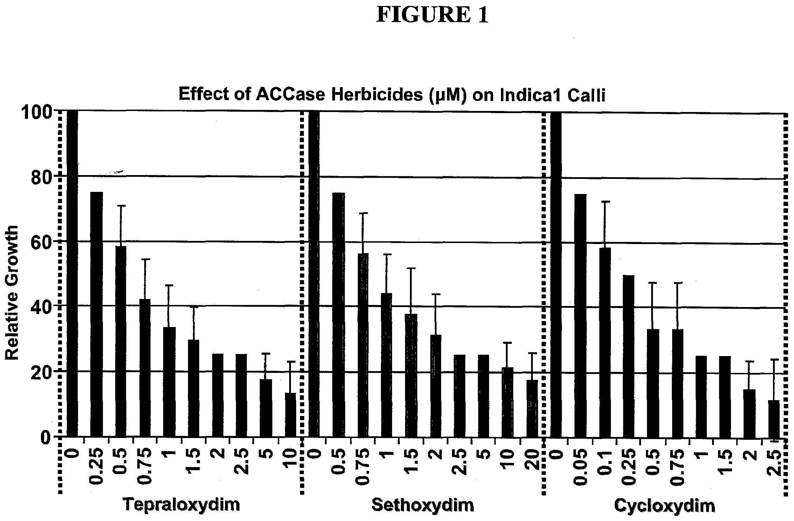

Once tissue culture conditions were determined, further establishment of selection conditions were established through the analysis of tissue survival in kill curves with cycloxydim, tepraloxydim, sethoxydim (Figure 1) or haloxyfop (not shown). Careful consideration of accumulation of the herbicide in the tissue, as well as its persistence and stability in the cells and the culture media was performed. Through these experiments, a sublethal dose has been established for the initial selection of mutated material.

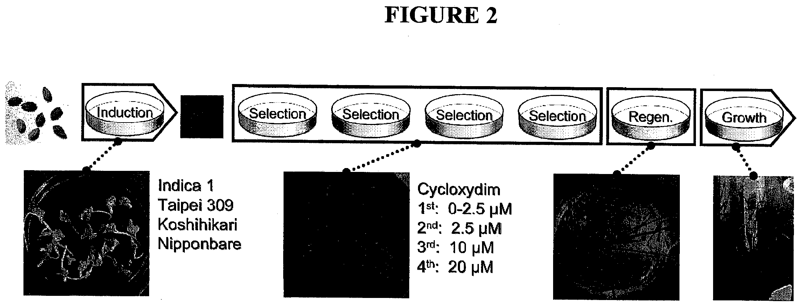

After the establishment of the starting dose of sethoxydim, cycloxydim, tepraloxydim, and haloxyfop in selection media, the tissues were selected in a step-wise fashion by increasing the concentration of the ACCase inhibitor with each transfer until cells are recovered that grew vigorously in the presence of toxic doses (see Figure 2). The resulting calli were further subcultured every 3-4 weeks to R001M with selective agent. Over 26,000 calli were subjected to selection for 4-5 subcultures until the selective pressure was above toxic levels as determined by kill curves and observations of continued culture. Toxic levels were determined to be 50 µM sethoxydim, 20 µM cycloxydim, 2.5 µM tepraloxydim (Figure 1) and 10 µM haloxyfop (not shown).

Alternatively, liquid cultures initiated from calli in MS711R (Table 2) with slow shaking and weekly subcultures. Once liquid cultures were established, selection agent was added directly to the flask at each subculture. Following 2-4 rounds of liquid selection, cultures were transferred to filters on solid R001M media for further growth.

### EXAMPLE 3

### Regeneration of plants

Tolerant tissue was regenerated and characterized molecularly for ACCase gene sequence mutations and/or biochemically for altered ACCase activity in the presence of the selective agent.

Following herbicide selection, calli were regenerated using a media regime of R025M for 10 - 14 days, R026M for ca. 2 weeks, R327M until well formed shoots were developed, and R008S until shoots were well rooted for transfer to the greenhouse (Table 2). Regeneration was carried out in the light. No selection agent was included during regeneration.

Once strong roots were established, M0 regenerants were transplant to the greenhouse in 4" square pots in a mixture of sand, NC Sandhills loamy soil, and Redi-earth (2:4:6) supplemented with gypsum. Transplants were maintained under a clear plastic cup until they were adapted to greenhouse conditions (ca. 1 week). The greenhouse was set to a day/night cycle of 27°C/21°C (80°F/70°F) with 600W high pressure sodium lights supplementing light to maintain a 14 hour day length. Plants were watered 2-3 times a day depending in the weather and fertilized daily. Rice plants selected for seed increase were transplanted into one gallon pots. As plants approached maturity and prepared to bolt, the pots were placed in small flood flats to better maintain water and nutrient delivery. Plants were monitored for insects and plant health and managed under standard Integrated Pest Management practices.

### EXAMPLE 4

### Sequence analysis

Leaf tissue was collected from clonal plants separated for transplanting and analyzed as individuals. Genomic DNA was extracted using a Wizard® 96 Magnetic DNA Plant System kit (Promega, US Patent Nos. 6,027,945 & 6,368,800) as directed by the manufacturer. Isolated DNA was PCR amplified using one forward and one reverse primer.

### Forward Primers:

OsACCpU5142: 5'-GCAAATGATATTACGTTCAGAGCTG-3' (SEQ ID NO:7)
OsACCpU5245: 5'-GTTACCAACCTAGCCTGTGAGAAG-3' (SEQ ID NO: 8)

### Reverse Primers:

OsACCpL7100: 5'-GATTTCTTCAACAAGTTGAGCTCTTC-3' (SEQ ID NO: 9)
OsACCpL7054: 5'-AGTAACATGGAAAGACCCTGTGGC-3' (SEQ ID NO: 10)

PCR amplification was performed using Hotstar Taq DNA Polymerase (Qiagen) using touchdown thermocycling program as follows: 96°C for 15 min, followed by 35 cycles (96°C, 30 sec; 58°C - 0.2 °C per cycle, 30 sec; 72°C, 3 min and 30 sec), 10 min at 72°C.

PCR products were verified for concentration and fragment size via agarose gel electrophoresis. Dephosphorylated PCR products were analyzed by direct sequence using the PCR primers (DNA Landmarks). Chromatogram trace files (.scf) were analyzed for mutation relative to Os05g0295300 using Vector NTI Advance 10™ (Invitrogen). Based on sequence information, two mutations were identified in several individuals. I1,781(*Am*)L and D2,078(*Am*)G were present in the heterozygous state. Sequence analysis was performed on the representative chromatograms and corresponding AlignX alignment with default settings and edited to call secondary peaks.

Samples inconsistent with an ACCase mutation were spray tested for tolerance and discarded as escapes. Surprisingly, most of the recovered lines were heterozygous for the 11,781(*Am*)L mutation and resistant events were generated in all tested genotypes using cycloxydim or sethoxydim: Indical (≥18 lines), Taipei 309 (≥14 lines), Nipponbare (≥3 lines), and Koshihikare (≥6 lines). One line was heterozygous for a D2,078(*Am*)G mutation. The D2,078(*Am*)G heterozygote line appeared stunted with narrow leaves, while the I1,781(*Am*)L heterozygotes varied in appearance, but most looked normal relative to their parental genotype. Several escapes were recovered and confirmed by sequencing and spray testing; however, sequencing results of the herbicide sensitive region of ACCase revealed that most tolerant mutants were heterozygous for an 11,781(*Am*)L, A to T mutation (See Table 3). One line, OsARWI010, was heterozygous for a D2,078(*Am*)G, A to G mutation. To date, all recovered plants lacking an ACCase mutation have been sensitive to herbicide application in the greenhouse.

**Table 3: Genotype of Rice Lines Recovered via Tissue Culture Selection**

| **Line** | **Parental Genotype** | **Rice Type** | **Mutation Identified** | **ATCC® Patent Deposit Designation** |
|---|---|---|---|---|
| OsARWI1 | Indica 1 | *indica* | I1781(*Am*)L | PTA-10568 |
| OsARWI3 | Indica 1 | *indica* | I1781(*Am*)L | PTA-10569 |
| OsARWI8 | Indica 1 | *indica* | I1781(*Am*)L | PTA-10570 |
| OsARWI10 | Indica 1 | *indica* | D2078(*Am*)G | NA, sterile |
| OSARWI315 | Indica 1 | *indica* | I1781(*Am*)L | NA |
| OsHPHI2 | Indica 1 | *indica* | I1781(*Am*)L | PTA-10267 |
| OsHPHI3 | Indica 1 | *indica* | I1781(*Am*)L | NA |
| OsHPHI4 | Indica 1 | *indica* | I1781(*Am*)L | NA |
| OsHPHKl | Koshihikari | *Japonica* | I1781(*Am*)L | NA |
| OsHPHK2 | Koshihikari | *Japonica* | I1781(*Am*)L | NA |
| OsHPHK3 | Koshihikari | *japonica* | I1781(*Am*)L | NA |
| OsHPHK4 | Koshihikari | *japonica* | I1781(*Am*)L | NA |
| OsHPHK6 | Koshihikari | *japonica* | I1781(*Am*)L | NA |
| OsHPHN1 | Nipponbare | *japonica* | I1781(*Am*)L | PTA-10571 |
| OsHPHT1 | Taipei 309 | *japonica* | I1781(*Am*)L | NA |
| OsHPHT4 | Taipei 309 | *japonica* | I1781(*Am*)L | NA |
| OsHPHT6 | Taipei 309 | *japonica* | I1781(*Am*)L | NA |

### EXAMPLE 5

### Demonstration of herbicide-tolerance

Selected mutants and escapes were transferred to small pots. Wild-type cultivars and 3 biovars of red rice were germinated from seed to serve as controls.

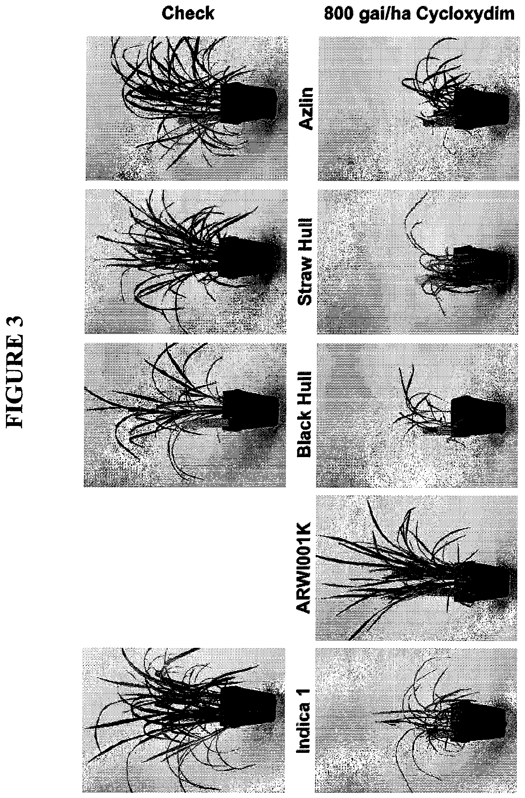

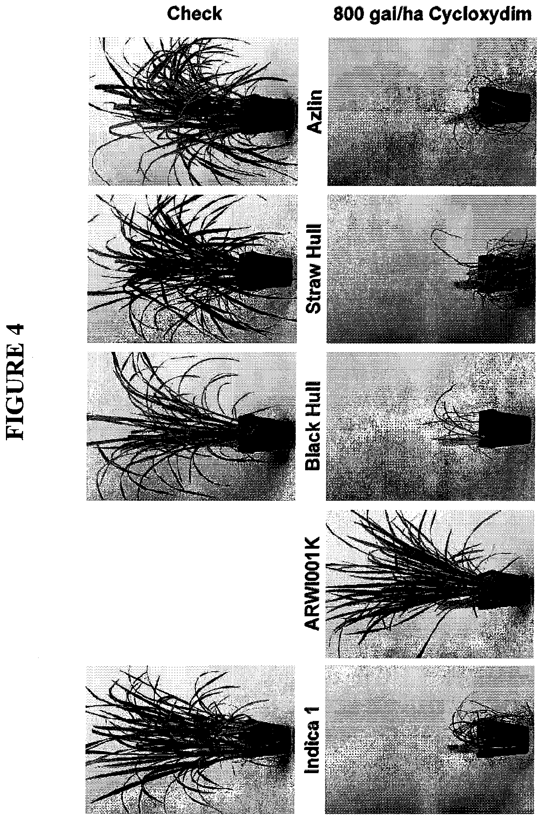

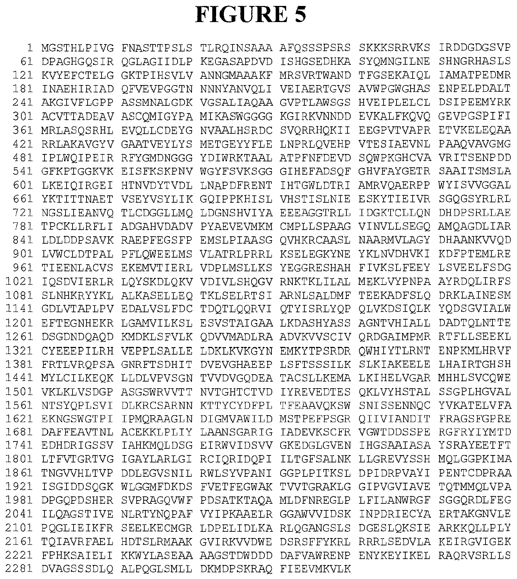

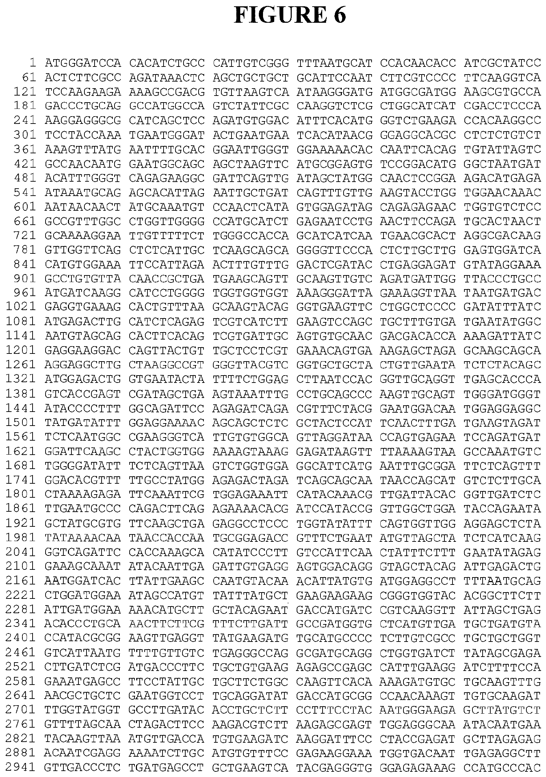

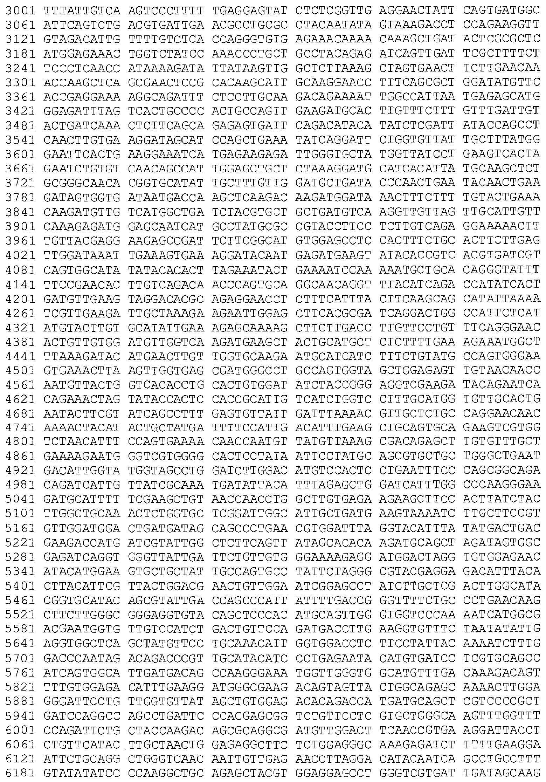

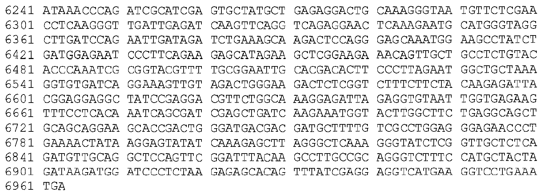

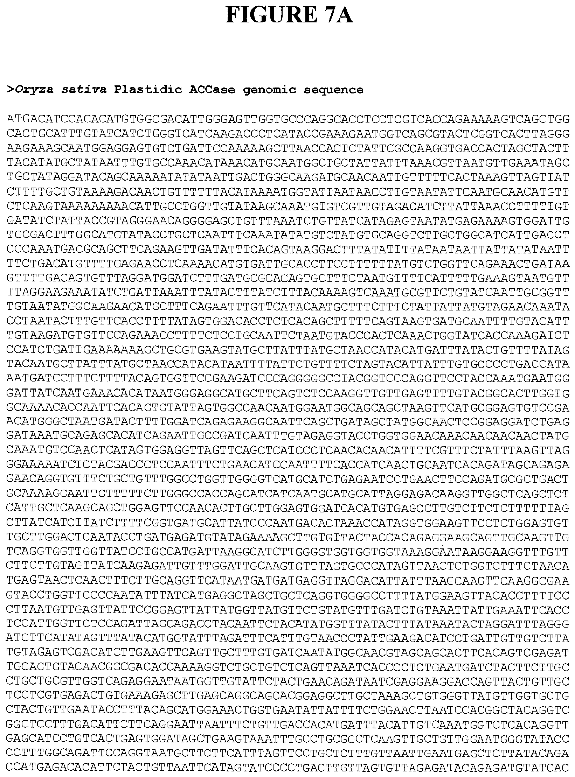

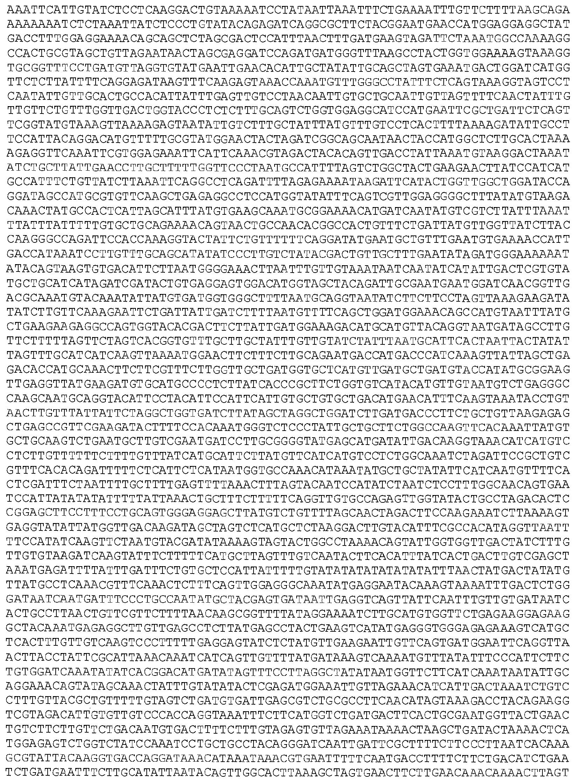

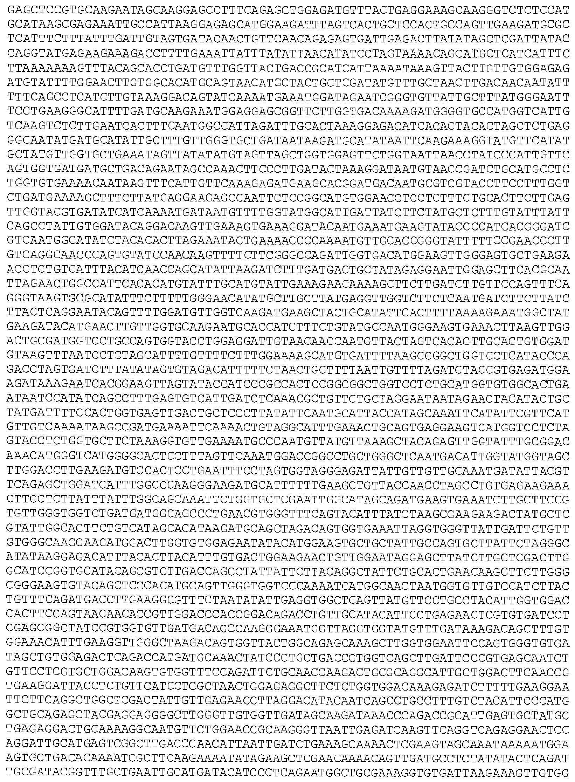

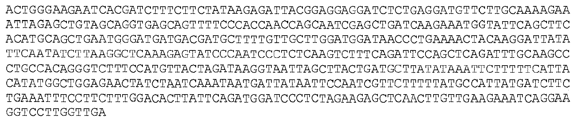

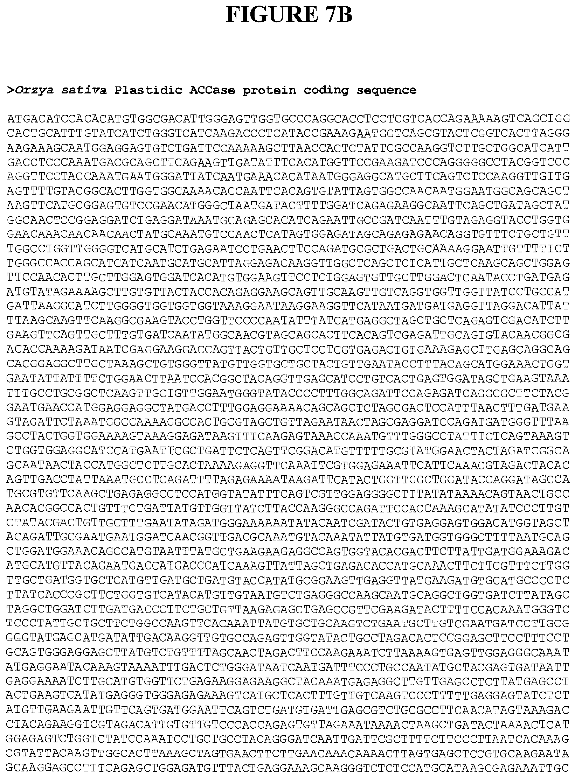

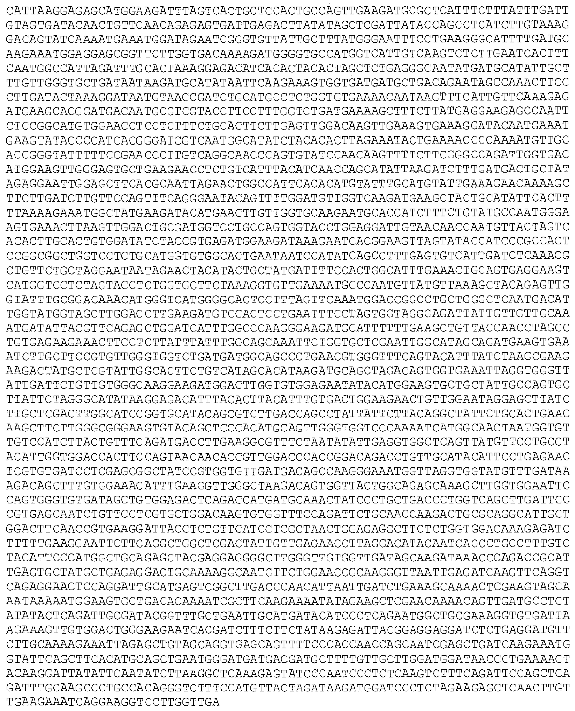

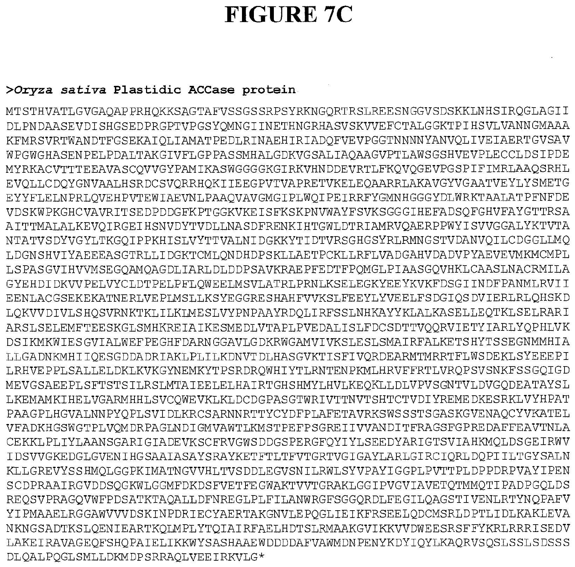

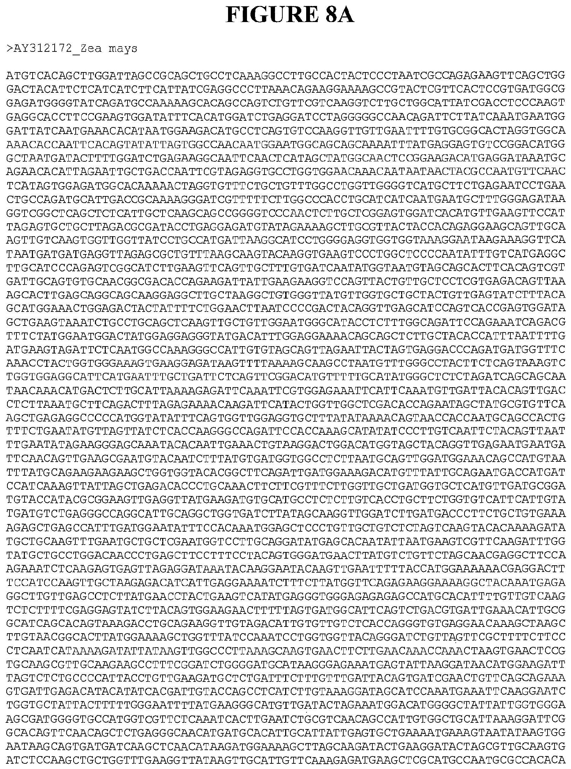

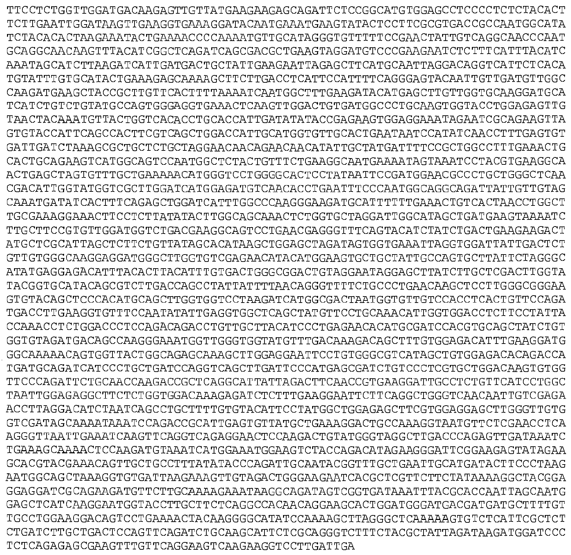

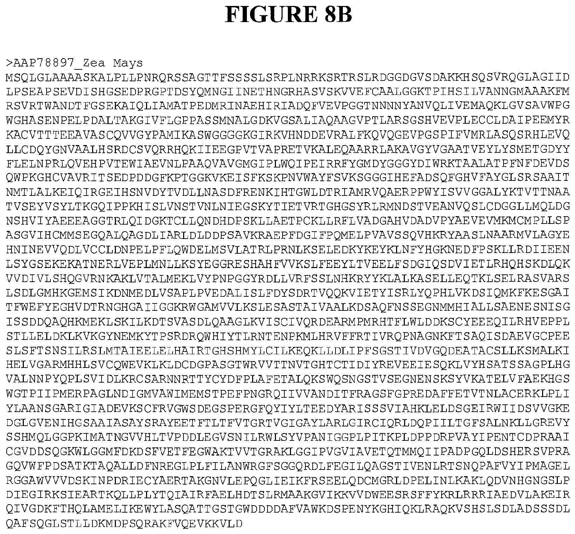

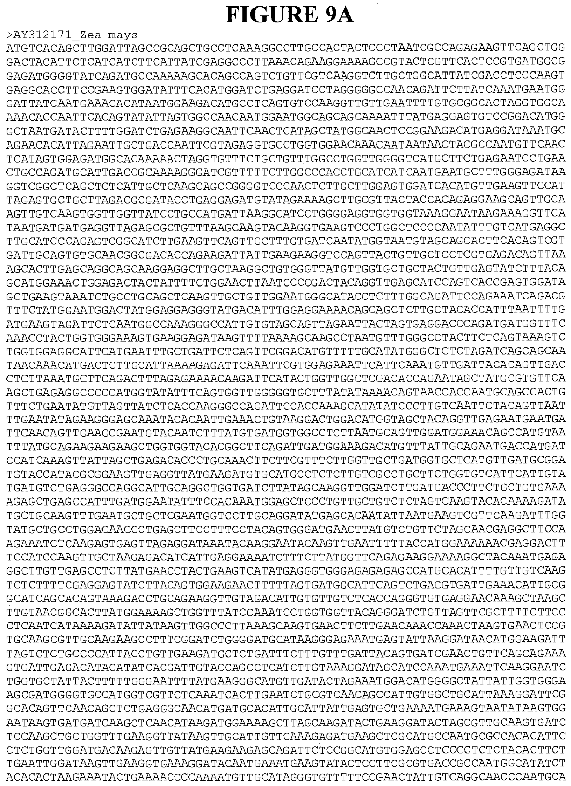

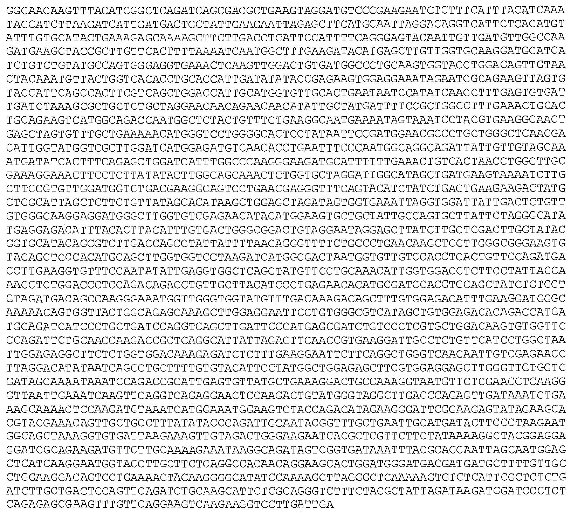

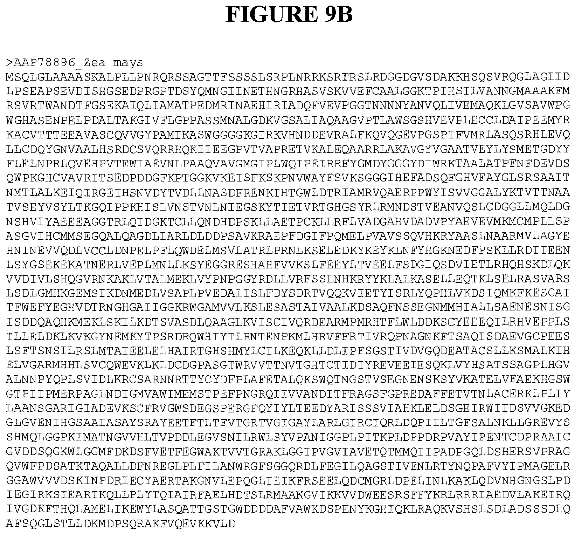

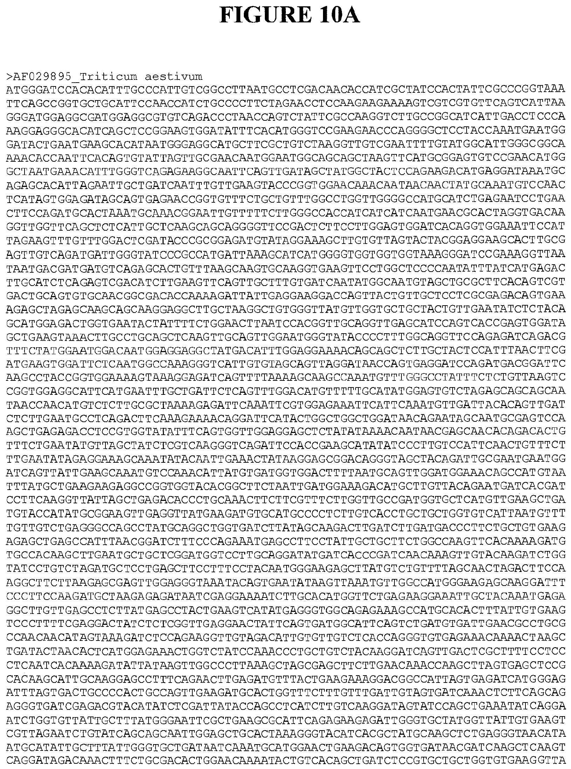

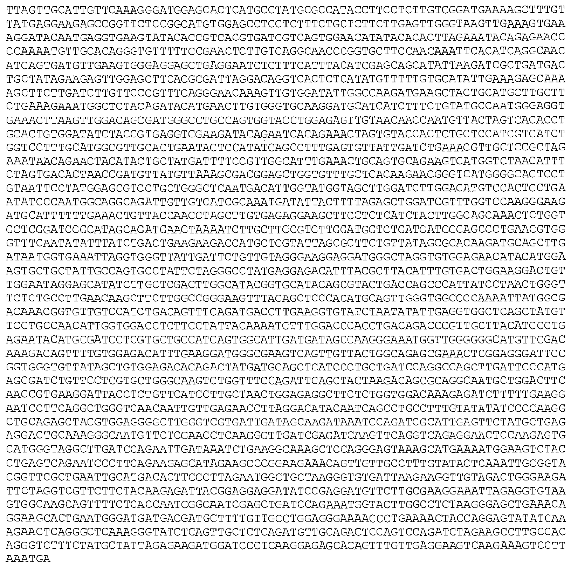

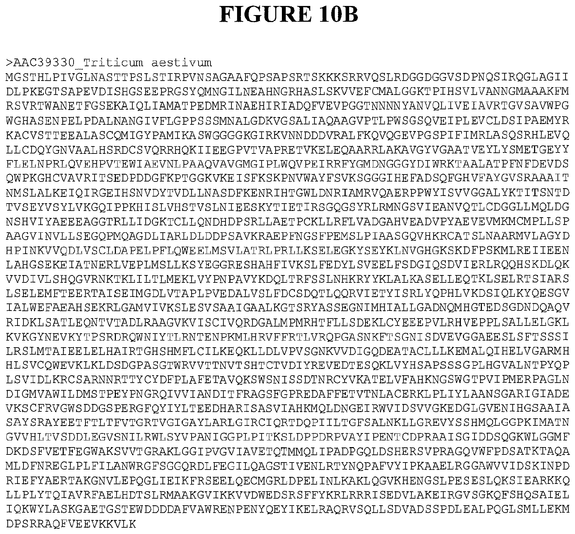

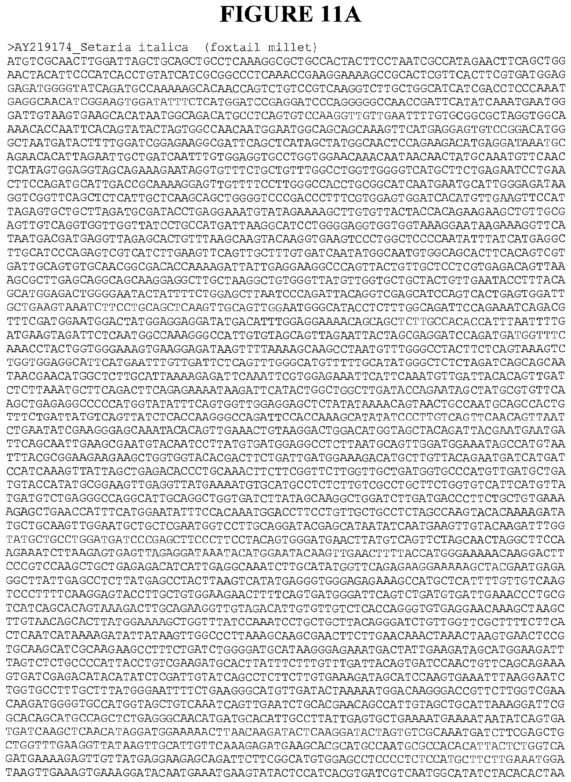

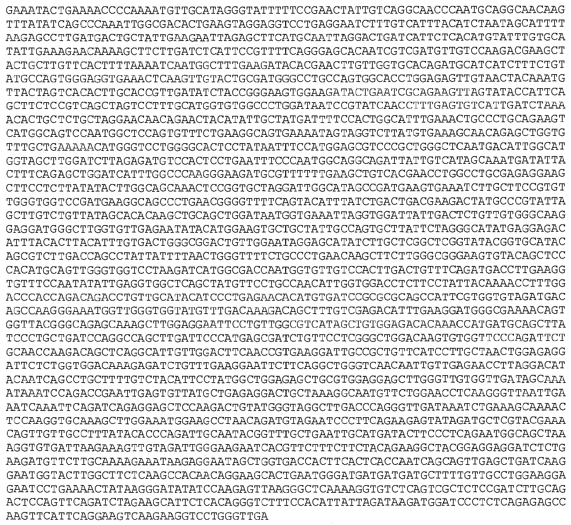

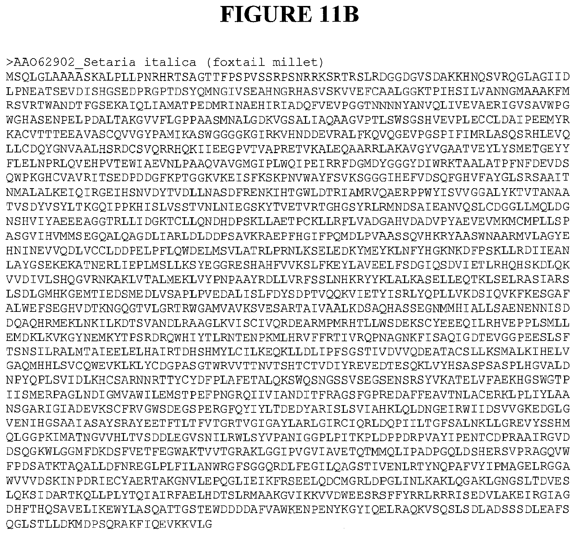

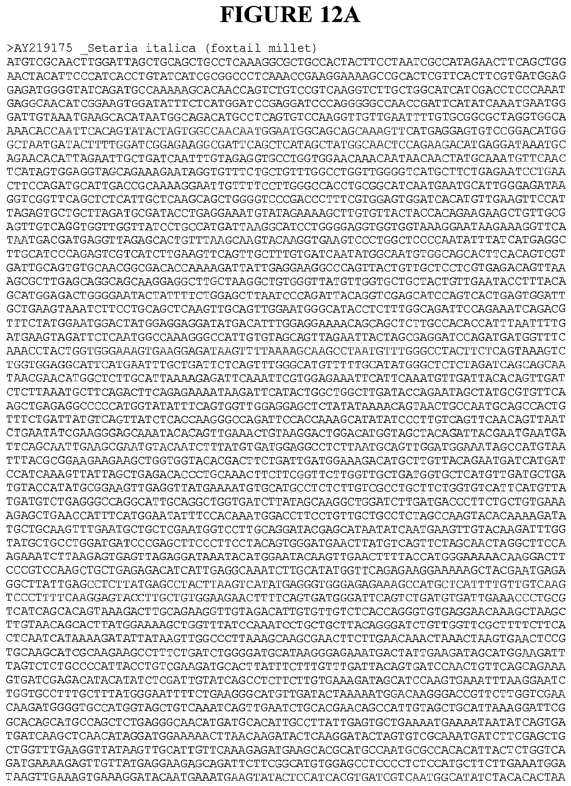

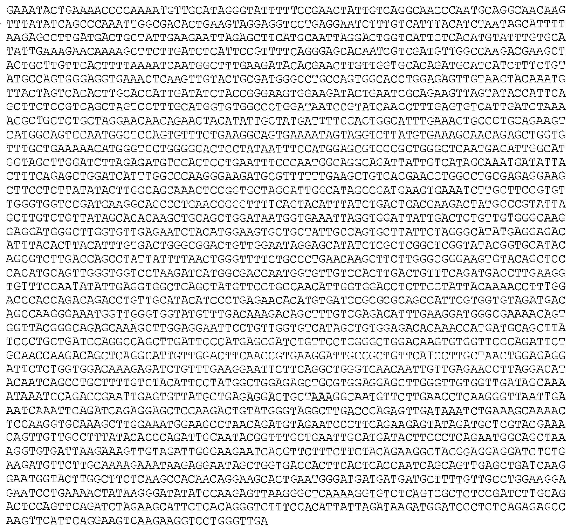

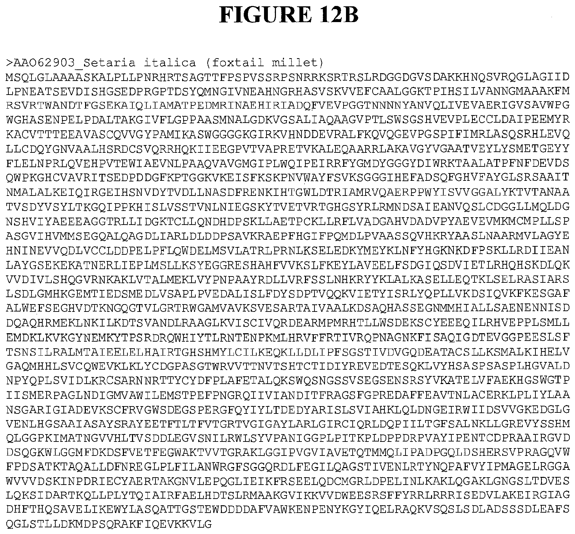

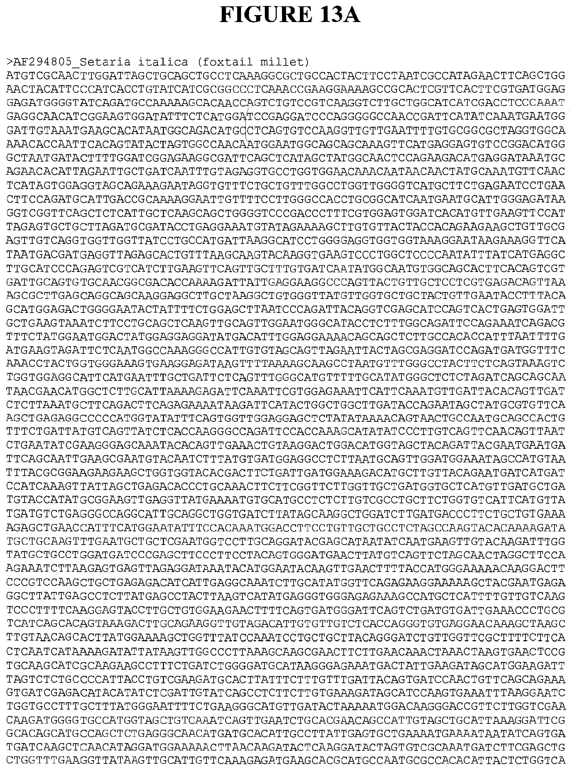

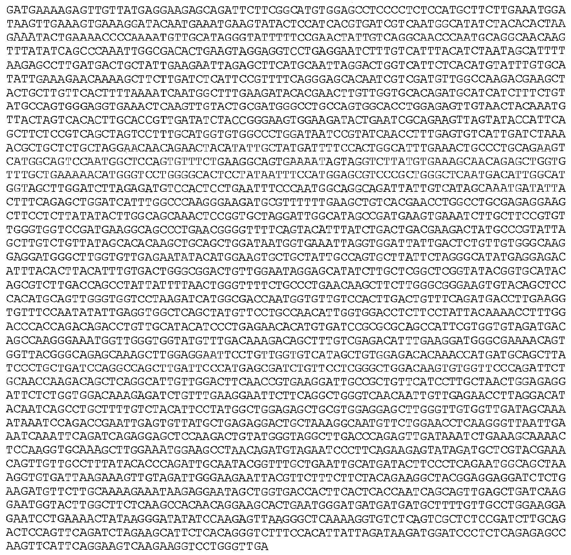

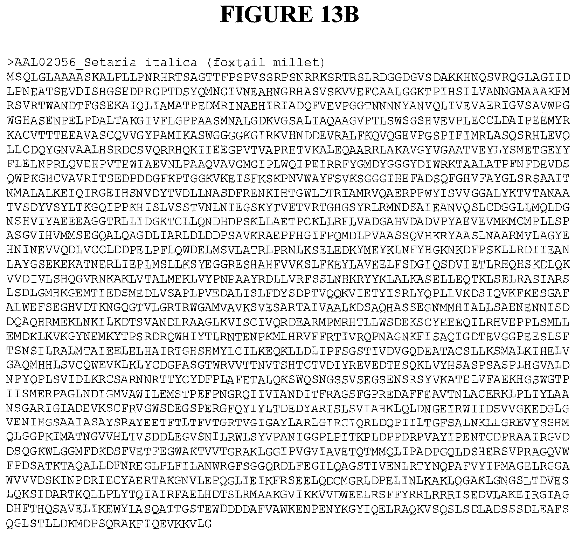

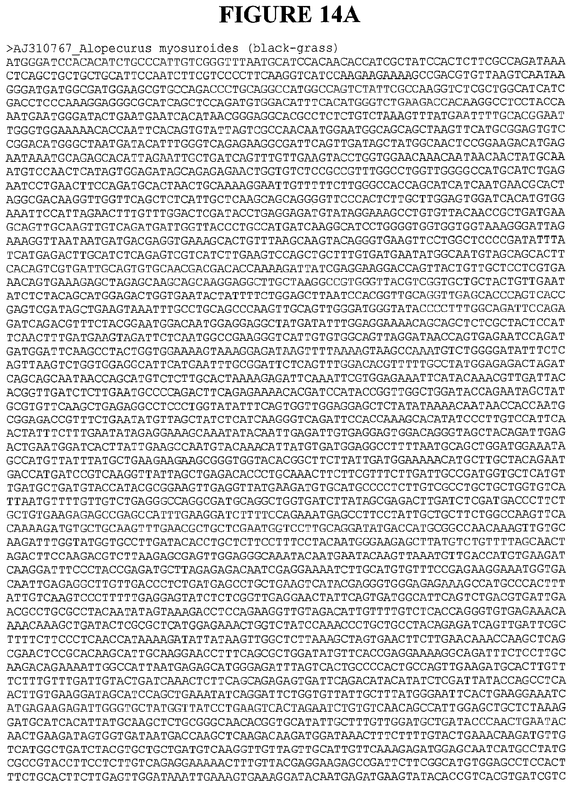

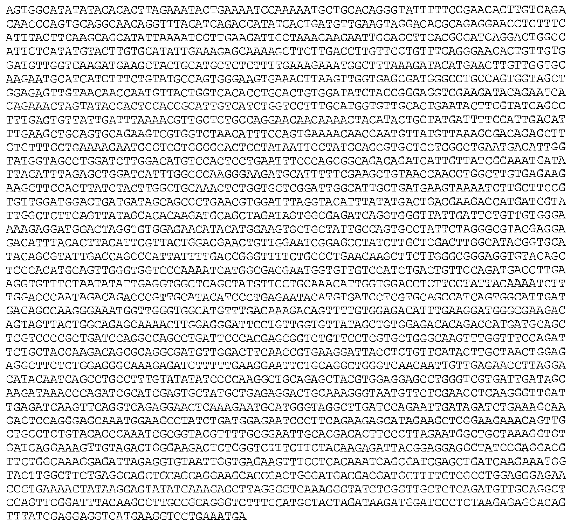

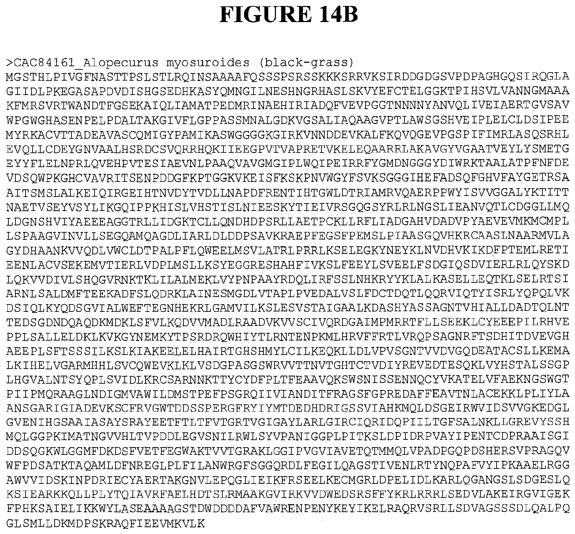

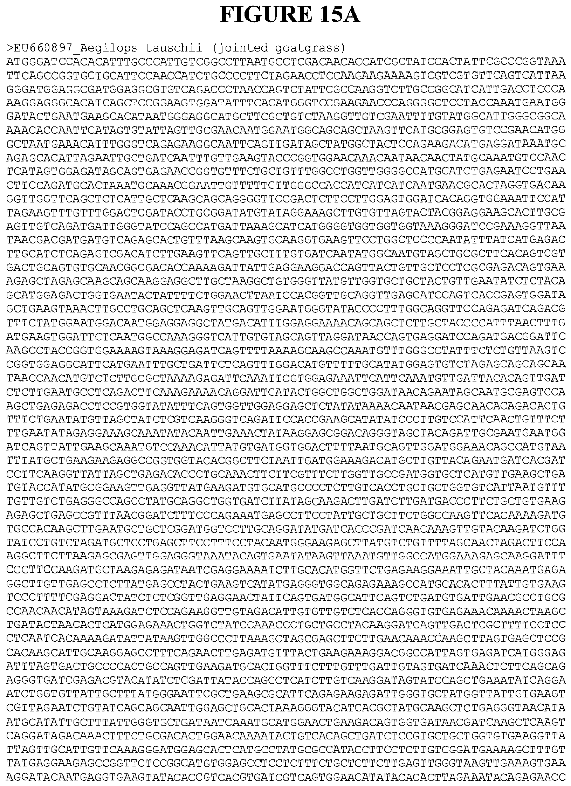

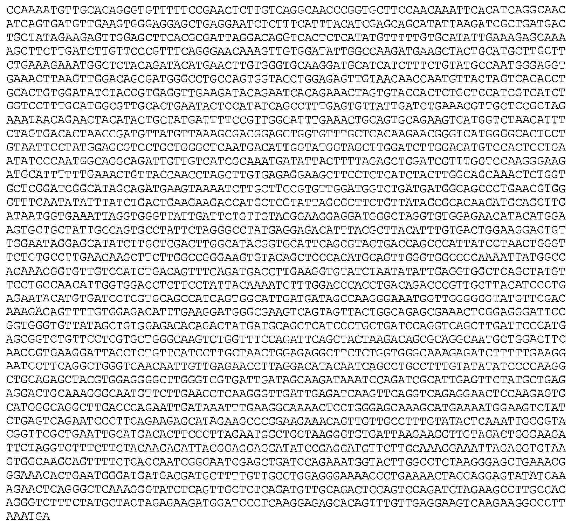

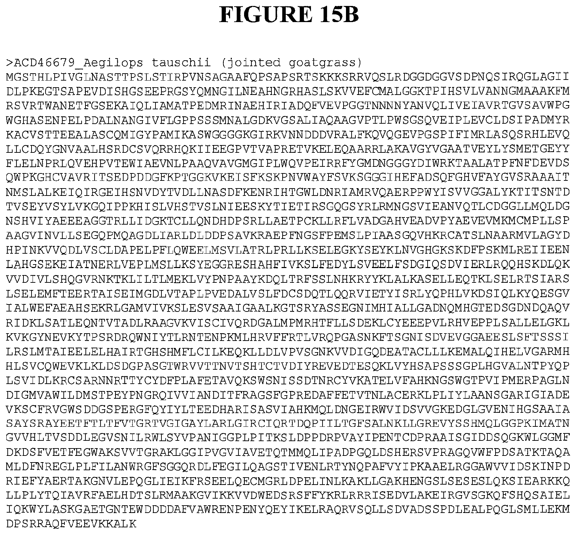

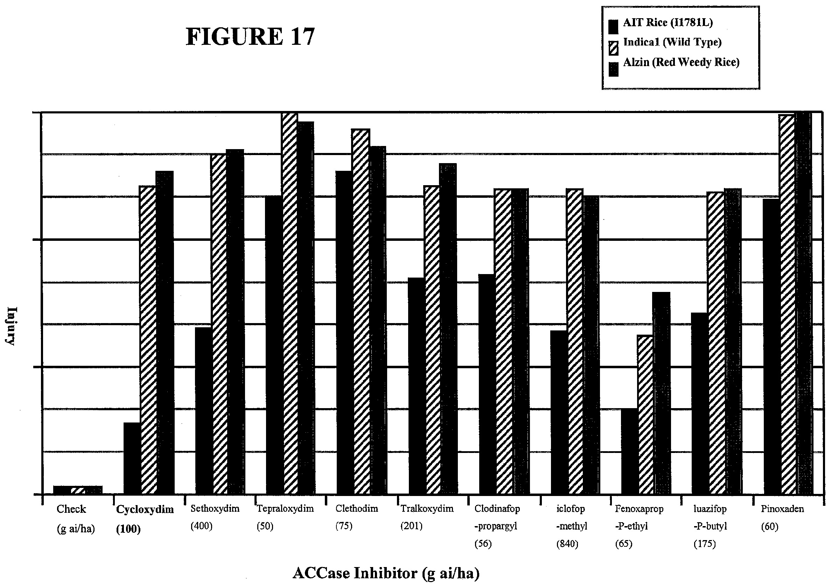

After ca. 3 weeks post-transplant, MO regenerants were sprayed using a track sprayer with 400-1600 g ai/ha cycloxydim (BAS 517H) supplemented with 0.1% methylated seed oil. After the plants had adapted to greenhouse conditions, a subset were sprayed with 800 g ai/ha cycloxydim. Once sprayed, plants were kept on drought conditions for 24 hours before being watered and fertilized again. Sprayed plants were photographed and rated for herbicide injury at 1 (Figure 3) and 2 weeks after treatment (Figure 4). No injury was observed on plants containing the 11,781(*Am*)L heterozygous mutation while control plants and tissue culture escapes (regenerated plants negative for the sequenced mutations) were heavily damaged after treatment (Figures 3 & 4). Figures 5-15 provide nucleic acid and/or amino acid sequences of acetyl-Coenzyme A carboxylase enzymes from various plants. Figure 17 provides a graph showing results for mutant rice versus various ACCase inhibitors.

### EXAMPLE 6

### Herbicide selection using tissue Culture

Media was selected for use and kill curves developed as specified above. For selection, different techniques were utilized. Either a step wise selection was applied, or an immediate lethal level of herbicide was applied. In either case, all of the calli were transferred for each new round of selection. Selection was 4-5 cycles of culture with 3-5 weeks for each cycle. Cali were placed onto nylon membranes to: facilitate transfer (200 micron pore sheets, Biodesign, Saco, Maine). Membranes were cut to fit 100x20 mm Petri dishes and were autoclaved prior to use 25-35 calli (average weight/calli being 22mg) were utilized in every plate. In addition, one set of calli were subjected to selection in liquid culture media with weekly subcultures followed by further selection on semi-solid media.

Mutant lines were selected using cycloxydim or sethoxydim in 4 different rice genotypes. Efficiencies of obtaining mutants was high either based on a percentage of calli that gave rise to a regenerable, mutant line or the number of lines as determined by the gram of tissue utilized. Overall, the mutation frequency compared to seashore paspalum is 5 fold and compared to maize is 2 fold. In some cases, this difference is much higher (>10 fold) as shown in Table 4 below.

**Table 4**

| Genotype | # Calli | Selection | Mutants | Rate | Weight (g) | # / gm callus |
|---|---|---|---|---|---|---|
| Indica 1 | 1865 | Cycloxidim | 3 | 0.161% | 41.04 | 0.07 |
| Indica 1 | 2640 | Sethoxydim | 3 | 0.114% | 58.08 | 0.05 |
| Koshi | 1800 | Cycloxidim | 6 | 0.333% | 39.6 | 0.15 |
| NB | 3400 | Cycloxidim | 1 | 0.029% | 74.8 | 0.01 |
| NB | 725 | Sethoxydim | 0 | 0.000% | 15.95 | 0.00 |
| T309 | 1800 | Cycloxidim | 8 | 0.444% | 36.9 | 0.20 |
| T309 | 1015 | Sethoxydim | 0 | 0.000% | 22.33 | 0.00 |
| Total | 13245 | | 21 | 0.159% | 291.39 | 0.07 |

If the data is analyzed using the criteria of selection, it is possible to see that cylcoxydim selection contributes to a higher rate of mutants isolated than sethoxydim, as shown in Table 5.

**Table 5**

| Genotype | # Calli | Selection | Mutants | Rate | Weight (g) | # / gm callus |
|---|---|---|---|---|---|---|
| Indica 1 | 1865 | Cycloxidim | 3 | 0.161% | 41.03 | 0.07 |
| Koshi | 1800 | Cycloxidim | 6 | 0.333% | 39.6 | 0.15 |
| NB | 3400 | Cycloxidim | 1 | 0.029% | 74.8 | 0.01 |
| T309 | 1800 | Cycloxidim | 8 | 0.444% | 39.6 | 0.20 |
| Total | 8865 | | 18 | 0.203% | 195.03 | 0.09 |
| Indica 1 | 2640 | Sethoxydim | 3 | 0.114% | 58.08 | 0.05 |
| NB | 725 | Sethoxydim | 0 | 0.000% | 15.95 | 0.00 |
| T309 | 1015 | Sethoxydim | 0 | 0.000% | 22.33 | 0.00 |
| Total | 4380 | | 3 | 0.068% | 96.36 | 0.03 |

Using this analysis, the rate for cycloxydim is almost 10 fold higher than either of the previous reports using sethoxydim selection, whereas rates using sethoxydirn selection are similar to those previously reported. Further, 68% of the lines were confirmed as mutants when selection was on cycloxydim compared to 21% of the lines when selection was on sethoxydim. Increases seem to come from using cycloxydim instead of sethoxydim as a selection agent. Further, the use of membranes made transfer of callus significantly easier than moving each piece individually during subcultures. Over 20 mutants were obtained. Fertility appears to be high with the exception of one mutant that has a mutation known to cause a fitness penalty (D2,078(*Am*)G).

### EXAMPLE 7

### Use of mutant ACCase genes as selectable markers in plant transformation

### Methods:

Indical and Nipponbare rice callus transformation was carried out essentially as described in Hiei and Komari (2008) with the exception of media substitutions as specified (see attached media table for details). Callus was induced on R001M media for 4-8 weeks prior to use in transformation. Agrobacterium utilized was LBA4404(pSB1) (Ishida et al. 1996) transformed with RLM185 (L. Mankin, unpublished: contains DsRed and a mutant AHAS for selection), ACC gene containing I1781(*Am*)L, ACC gene containing I1781(*Am*)L and W2027C, ACC gene containing I1781(*Am*)L and I2041(*Am*)N, or ACC gene containing I1781(*Am*)A or wild type which also contains a mutant AHAS gene for selection. Agrobacterium grown for 1-3 days on solid media was suspended in M-LS-002 medium and the OD₆₆₀ adjusted to approximately 0.1. Callus was immersed in the Agrobacterium solution for approximately 30 minutes. Liquid was removed, and then callus was moved to filter paper for co-culture on semi-solid rice cc media. Co-culture was for 3 days in the dark at 24°C. Filters containing rice callus were directly transferred to R001M media containing Timentin for 1-2 weeks for recovery and cultured in the dark at 30°C. Callus was subdivided onto fresh R001M media with Timentin and supplemented with 100µM Imazethapyr, 10µM Cycloxydim or 2.5µM Tepraloxydim. After 3-4 weeks, callus was transferred to fresh selection media. Following another 3-4 weeks, growing callus was transferred to fresh media and allowed to grow prior to Taqman analysis. Taqman analysis was for the Nos terminator and was conducted to provide for a molecular confirmation of the transgenic nature of the selected calli. Growth of transgenic calli was measured with various selection agents by subculturing calli on media containing either 10µM Cycloxydim or Haloxyfop, 2.5µM Tepraloxydim or 100µM Imazethapry. Calli size was measured from scanned images following initial subculture and then after approximately 1 month of growth.

Transformation of maize immature embryos was carried out essentially as described by Lai et al (submitted). Briefly, immature embryos were co-cultured with the same *Agrobacterium* strains utilized for rice transformation suspended in M-LS-002 medium to an OD₆₆₀ of 1.0. Co-culture was on Maize CC medium for 3 days in the dark at 22°C. Embryos were removed from co-culture and transferred to M-MS-101 medium for 4-7 days at 27°C. Responding embryos were transferred to M-LS-202 medium for Imazethapyr selection or M-LS-213 media supplemented with either 1µM Cycloxydim or 0.75µM Tepraloxydim. Embryos were cultured for 2 weeks and growing callus was transferred to a second round of selection using the same media as previous except that Cycloxydim selection was increased to 5µM. Selected calli were transferred to M-LS-504 or M-LS-513 media supplemented with either 5µM Cycloxydim or 0.75µM of Tepraloxydim for and moved to the light (16hr/8hr day/night) for regeneration. Shoots appeared between 2-3 weeks and were transferred to plantcon boxes containing either M-LS-618 or M-LS-613 supplemented with either 5µM Cycloxydim or 0.75µM of Tepraloxydim for further shoot development and rooting. Leaf samples were submitted for Taqman analysis. Positive plants were transferred to soil for growth and seed generation. In the second set of experiments, conditions were identical except that Tepraloxydim selection was decreased to 0.5µM during regeneration and shoot and root formation. In the third set of experiments, Haloxyfop was also tested as a selection agent. In these experiments, 1M was used throughout for selection

### Results and Discussion:

Transgenic calli were obtained from Indical rice transformation experiments using ACC gene containing I1781(*Am*)L and W2027(*Am*)C, and ACC gene containing I1781(*Am*)L and I2041(*Am*)N. One callus was obtained from ACC gene containing I1781(*Am*)L and W2027(*Am*)C following Tepraloxydim selection and 3 calli were obtained from ACC gene containing I1781(*Am*)L and I2041(*Am*)N. One callus was obtained from ACC gene containing I1781(*Am*)L and I2041(*Am*)N using Cycloxydim selection. Nos Taqman showed that all of these calli were transgenic. Calli were screened for growth under various selection agents including Imazethapry (Pursuit - P) for the mutant AHAS selectable marker.

As can be observed in Table 6, the double mutant constructs allowed for growth on both Cycloxydim and Tepraloxydim in addition to Haloxyfop. The levels utilized in these growth experiments are inhibitory for wild type material.

**Table 6: Growth of transgenic Indical callus on various selection media. Growth was measured as a % change in size following 1 month of culture on the selection media.**

| **Selection µM** | | | | |
|---|---|---|---|---|
| **Construct** | **H10** | **C10** | **T2.5** | **P100** |
| **I1781(*Am*)L, W2027(*Am*)C** | 1669% | 867% | 1416% | 739% |
| **I1781(*Am*)L, I2041(*Am*)N** | 1613% | 884% | 1360% | 634% |

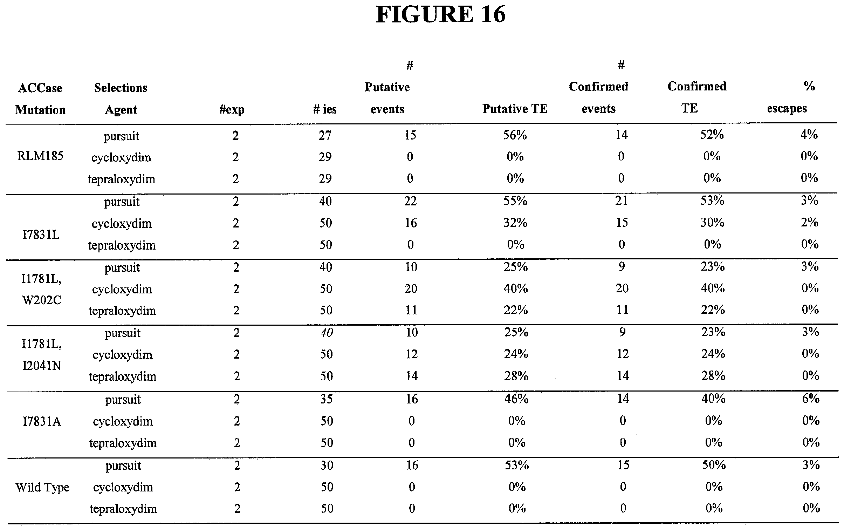

Results from the first set of maize experiments reveal that both the single of the double mutant can be used to select for Cycloxydim resistance or both Cylcoxydim or Tepraloxydim resistance at a relatively high efficiency (Figure 16).

Efficiencies between selection agents was relatively comparable in these experiments with maybe a slight decrease in the overall efficiency with the single mutant on Cycloxydim compared to Pursuit selection. However, the double mutant may have a slight increased efficiency. The escape rate - the percentage of non-confirmed putative events - was lower for Cycloxydim or Tepraloxydim. Further, under the conditions described, it was possible to differentiate between the single and double mutants using Tepraloxydim selection.

Similar results have been obtained in the second set of experiments (not shown). In the third set of experiments, Haloxyfop is also an efficient selectable marker for use in transformation with either the single or the double mutant (not shown).

The single mutant is useful for high efficiency transformation using Cycloxydim or Haloxyfop selection. It should also be useful for other related compounds such as Sethoxydim. The double mutant is useful for these selection agents with the addition that Tepraloxydim can be used. The single and the double mutant can be used in a two stage transformation in that the single mutant can be differentiated from the double with Tepraloxydim selection. In combination with other current BASF selection markers, these give two more options for high efficiency transformations of monocots and maize in particular.

Herbicide tolerance phenotypes as described herein have also been exhibited by ACCase-inhibitor tolerant rice plants hereof, in the field under 600 g/ha cycloxydim treatment (data not shown).

## Claims

1. A rice plant comprising a mutagenized rice plastidic acetyl-Coenzyme A carboxylase (ACCase) nucleic acid having a sequence obtained by an induced, random mutagenesis method and encoding a rice plastidic ACCase having, as a result of said mutagenesis, an isoleucine-to-leucine substitution at the amino acid position corresponding to position 1,781 of the *Alopecurus myosuroides* (*Am*) plastidic ACCase, wherein the rice plastidic ACCase is a protein comprising a modified version of SEQ ID NO:2 or SEQ ID NO:3, and confers to the rice plant tolerance to 100 g ai/ha cycloxydim.

2. The rice plant of claim 1, wherein said plastidic ACCase further comprises: an amino acid substitution at position 1,785; 1,786; 1,811; 1,864; 2,027; 2,039; 2,041; 2,049; 2,074; 2,075; 2,088; 2,080; 2,088; 2,095; 2,096; or 2,098 corresponding to the position of the *Alopecurus myosuroides* (*Am*) plastidic ACCase; a duplication of valine at position 2,075 *(Am);* or a deletion of the amino acid at position 2,080(*Am*).

3. The rice plant of claim 1 further comprising:
a. a glycine substitution at position 1,785(*Am*);
b. a proline substitution at position 1,786(*Am*);
c. an asparagine substitution at position 1,811(*Am*);
d. a glycine substitution at position 2,039(*Am*);
e. an isoleucine, leucine, or phenylalanine substitution at position 2,049(*Am*);
f. a leucine substitution at position 2*,*074(*Am*);
g. a leucine, isoleucine, or methionine substitution at position 2,075(*Am*);
h. a duplication of the valine at position 2,075(*Am*);
i. a threonine, or glycine substitution at position 2,078(*Am*);
j. a glutamic acid substitution at position 2,080(*Am*);
k. an arginine, tryptophan, phenylalanine, glycine, histidine, lysine, leucine, serine, threonine, or valine substitution at position 2,088(*Am*);
l. a glutamic acid substitution at position 2,095(*Am*); or
m. an alanine, histidine, proline, serine, or glycine substitution at position 2,098(*Am*).

4. The rice plant of claim 1, wherein said plant is a plant of any one of lines: OsHPHI2, OsARWI1, OSARWI3, OsARWI8, or OsHPHN1, a representative sample of seed of each line having been deposited with American Type Culture Collection (ATCC) under Patent Deposit Designation Number PTA-10267, PTA-10568, PTA-10569, PTA-10570, or PTA-10571, respectively; or is a hybrid, descendant, or genetically engineered derivative of a plant of any one of lines OsHPHI2, OsARWI1, OsARWI3, OsARWI8, or OSHPHN1.

5. The rice plant of claim 1, wherein said plant is tolerant to at least one ACCase-inhibiting herbicide selected from: alloxydim, butroxydim, clethodim, cloproxydim, cycloxydim, sethoxydim, tepraloxydim, tralkoxydim, chlorazifop, clodinafop, clofop, diclofop, fenoxaprop, fenoxaprop-P, fenthiaprop, fluazifop, fluazifop-P, haloxyfop, haloxyfop-P, isoxapyrifop, propaquizafop, quizalofop, quizalofop-P, quizalofop-P-ethyl, quizalofop-P-tefuryl, trifop, pinoxaden, and agronomically acceptable salts or esters thereof.

6. A rice seed or rice cell comprising a mutagenized acetyl-Coenzyme A carboxylase (ACCase) nucleic acid having a sequence obtained by an induced, random mutagenesis method and encoding a rice plastidic ACCase having, as a result of said mutagenesis, an isoleucine-to-leucine substitution at an amino acid position corresponding to position 1,781 of the *Alopecurus myosuroides* (*Am*) plastidic ACCase, wherein the rice plastidic ACCase is a protein comprising a modified version of SEQ ID NO:2 or SEQ ID NO:3, and confers to a rice plant tolerance to 100 g ai/ha cycloxydim.

7. The rice seed or rice cell of claim 6, wherein said plastidic ACCase further comprises: an amino acid substitution at position 1,785; 1,786; 1,811; 1,864; 2,027; 2,039; 2,041; 2,049; 2,074; 2,075; 2,088; 2,080; 2,088; 2,095; 2,096; or 2,098 corresponding to the position of the *Alopecurus myosuroides* (*Am*) plastidic ACCase; a duplication of valine at position 2,075 (*Am*); or a deletion of the amino acid at position 2,080(*Am*).

8. The rice seed or rice cell of claim 6, further comprising:
a. a glycine substitution at position 1,785(*Am*);
b. a proline substitution at position 1,786(*Am*);
c. an asparagine substitution at position 1,811(*Am*);
d. a glycine substitution at position 2,039(*Am*);
e. an isoleucine, leucine, or phenylalanine substitution at position 2,049(*Am*);
f. a leucine substitution at position 2,074(*Am*);
g. a leucine, isoleucine, or methionine substitution at position 2,075(*Am*);
h. a duplication of the valine at position 2,075(*Am*);
i. a threonine, or glycine substitution at position 2,078(*Am*);
j. a glutamic acid substitution at position 2,080(*Am*);
k. an arginine, tryptophan, phenylalanine, glycine, histidine, lysine, leucine, serine, threonine, or valine substitution at position 2,088(*Am*);
l. a glutamic acid substitution at position 2,095(*Am*); or
m. an alanine, histidine, proline, serine, or glycine substitution at position 2,098(*Am*).

9. The seed of any one of claims 6-8, wherein the seed further comprises a seed treatment comprising an ACCase-inhibiting herbicide.

10. The seed of claim 9, wherein said ACCase-inhibiting herbicide comprises at least one of: alloxydim, butroxydim, clethodim, cloproxydim, cycloxydim, sethoxydim, tepraloxydim, tralkoxydim, chlorazifop, clodinafop, clofop, diclofop, fenoxaprop, fenoxaprop-P, fenthiaprop, fluazifop, fluazifop-P, haloxyfop, haloxyfop-P, isoxapyrifop, propaquizafop, quizalofop, quizalofop-P, quizalofop-P-ethyl, quizalofop-P-tefuryl, trifop, pinoxaden, or an agronomically acceptable salt or ester thereof.

11. A rice plastidic ACCase as described in any one of claims 1-10, wherein said rice plastidic ACCase is non-transgenic.

12. The plant or seed of any one of claims 1-10, wherein said plant or seed is non-transgenic.

13. A method for growing a herbicide-tolerance rice plant, said method comprising:
a. growing the plant of any one of claims 1-5 or 12, or growing a plant from the rice seed of any one of claims 6-10 or 12 by allowing the seed to sprout; and
b. applying to the plant and the vicinity thereof an acetyl-Coenzyme A carboxylase-inhibiting herbicide in an amount that inhibits growth of a corresponding wild type plant.

14. The method of claim 13, wherein said ACCase-inhibiting herbicide comprises at least one of: alloxydim, butroxydim, clethodim, cloproxydim, cycloxydim, sethoxydim, tepraloxydim, tralkoxydim, chlorazifop, clodinafop, clofop, diclofop, fenoxaprop, fenoxaprop-P, fenthiaprop, fluazifop, fluazifop-P, haloxyfop, haloxyfop-P, isoxapyrifop, propaquizafop, quizalofop, quizalofop-P, quizalofop-P-ethyl, quizalofop-P-tefuryl, trifop, pinoxaden, or an agronomically acceptable salt or ester thereof.

15. Use of a plant of any one of claims 1-5 or a seed of any one of claims 6-10 for producing a food product, a consumer product, an industrial product or a veterinary product.

16. An isolated nucleic acid comprising a nucleic acid sequence as described in any one of claims 1-12.

17. A method for identifying a plant of any one of claims 1-5 or12 or a seed of any one of claims 6-10 or 12, comprising:
a. providing biological material from a plant or a seed;
b. performing PCR or hybridization testing of the ACCase genes in said biological material to determine if the biological material comprises an ACCase nucleic acid as described in any one of claims 1-12; and
c. identifying, based on the results of step (b), that the plant of step (a) comprises the nucleic acid as described in any one of claims 1-12.

## Patentansprüche

1. Reispflanze umfassend eine mutagenisierte Reis-Plastiden-Acetyl-Coenzym-A-Carboxylase-(ACCase-)Nukleinsäure, welche eine Sequenz aufweist, die durch ein induziertes, ungerichtetes Mutageneseverfahren erhalten wurde und für eine Reis-Plastiden-ACCase kodiert, die als Ergebnis dieser Mutagenese eine Isoleucin-zu-Leucin-Substitution an der Aminosäureposition aufweist, welche der Position 1.781 der *Alopecurus myosuroides*-(*Am*-)Plastiden-ACCase entspricht, wobei die Reis-Plastiden-ACCase ein Protein ist, das eine modifizierte Version von SEQ ID NR:2 oder SEQ ID NR:3 umfasst und der Reispflanze Toleranz gegenüber 100 g Wirkstoff/ha Cycloxydim verleiht.

2. Reispflanze nach Anspruch 1, wobei die Plastiden-ACCase ferner folgendes umfasst: eine Aminosäuresubstitution an Position 1.785, 1.786, 1.811, 1.864, 2.027, 2.039, 2.041, 2.049, 2.074, 2.075, 2.088, 2.080, 2.088, 2.095, 2.096 oder 2.098, entsprechend der Position der *Alopecurus* myosuroides-(*Am*-)Plastiden-ACCase; eine Duplikation des Valins an Position 2.075(Am); oder eine Deletion der Aminosäure an Position 2.080(Am).

3. Reispflanze nach Anspruch 1 ferner umfassend:
a. eine Glycin-Substitution an Position 1.785(Am);
b. eine Prolin-Substitution an Position 1.786(*Am*);
c. eine Asparagin-Substitution an Position 1.811 *(Am);*
d. eine Glycin-Substitution an Position 2.039(Am);
e. eine Isoleucin-, Leucin- oder Phenylalanin-Substitution an Position 2.049(Am);
f. eine Leucin-Substitution an Position 2.074(Am);
g. eine Leucin-, Isoleucin oder Methionin-Substitution an Position 2.075(Am);
h. eine Duplikation des Valins an Position 2.075(Am);
i. eine Threonin- oder Glycin-Substitution an Position 2.078(Am);
j. eine Glutaminsäure-Substitution an Position 2.080(Am);
k. eine Arginin-, Tryptophan-, Phenylalanin-, Glycin-, Histidin-, Lysin-, Leucin-, Serin-, Threonin- oder Valin-Substitution an Position 2.088(Am);
l. eine Glutaminsäure-Substitution an Position 2.095(Am); oder
m. eine Alanin-, Histidin-, Prolin-, Serin- oder Glycin-Substitution an Position 2.098(Am).

4. Reispflanze nach Anspruch 1, wobei die Pflanze eine Pflanze einer der folgenden Linien ist: OsHPHI2, OsARWI1 , OsARWI3, OsARWI8 oder OsHPHN1, wobei eine repräsentative Saatgutprobe jeder Linie unter der Patent-Hinterlegungsnummer PTA-10267, PTA-10568, PTA-10569, PTA-10570 bzw. PTA-10571 bei der American Type Culture Collection (ATCC) hinterlegt wurde, oder ein Hybrid, Nachkomme oder genetechnisch veränderter Abkömmling einer Pflanze einer der Linien OsHPHI2, OsARWI1, OsARWI3, OsARWI8 oder OsHPHN1 ist.

5. Reispflanze nach Anspruch 1, wobei die Pflanze gegenüber mindestens einem ACCase-inhibierenden Herbizid tolerant ist, das ausgewählt ist unter: Alloxydim, Butroxydim, Clethodim, Cloproxydim, Cycloxydim, Sethoxydim, Tepraloxydim, Tralkoxydim, Chlorazifop, Clodinafop, Clofop, Diclofop, Fenoxaprop, Fenoxaprop-P, Fenthiaprop, Fluazifop, Fluazifop-P, Haloxyfop, Haloxyfop-P, Isoxapyrifop, Propaquizafop, Quizalofop, Quizalofop-P, Quizalofop-P-ethyl, Quizalofop-P-tefuryl, Trifop, Pinoxaden, und landwirtschaftlich verträglichen Salzen oder Estern davon.

6. Reissaatkorn oder Reiszelle umfassend eine mutagenisierte Plastiden-Acetyl-Coenzym-A-Carboxylase-(ACCase-)Nukleinsäure, welche eine Sequenz aufweist, die durch ein induziertes, ungerichtetes Mutageneseverfahren erhalten wurde und für eine Reis-Plastiden-ACCase kodiert, die als Ergebnis dieser Mutagenese eine Isoleucin-zu-Leucin-Substitution an einer Aminosäureposition aufweist, welche der Position 1.781 der *Alopecurus myosuroides*-(*Am*-)Plastiden-ACCase entspricht, wobei die Reis-Plastiden-ACCase ein Protein ist, das eine modifizierte Version von SEQ ID NR:2 oder SEQ ID NR:3 umfasst und einer Reispflanze Toleranz gegenüber 100 g Wirkstoff/ha Cycloxydim verleiht

7. Reissaatkorn oder Reiszelle nach Anspruch 6, wobei die Plastiden-ACCase ferner folgendes umfasst: eine Aminosäuresubstitution an Position 1.785, 1.786, 1.811, 1.864, 2.027, 2.039, 2.041, 2.049, 2.074, 2.075, 2.088, 2.080, 2.088, 2.095, 2.096 oder 2.098, entsprechend der Position der *Alopecurus myosuroides*-(*Am*-)Plastiden-ACCase; eine Duplikation des Valins an Position 2.075(*Am*); oder eine Deletion der Aminosäure an Position 2.080(Am).

8. Reissaatkorn oder Reiszelle nach Anspruch 6 ferner umfassend:
a. eine Glycin-Substitution an Position 1.785(*Am*);
b. eine Prolin-Substitution an Position 1.786(*Am*);
c. eine Asparagin-Substitution an Position 1.811 (*Am*);
d. eine Glycin-Substitution an Position 2.039(*Am*);
e. eine Isoleucin-, Leucin- oder Phenylalanin-Substitution an Position 2.049(*Am*);
f. eine Leucin-Substitution an Position 2.074(*Am*);
g. eine Leucin-, Isoleucin oder Methionin-Substitution an Position 2.075(*Am*);
h. eine Duplikation des Valins an Position 2.075(*Am*);
i. eine Threonin- oder Glycin-Substitution an Position 2.078(*Am*);
j. eine Glutaminsäure-Substitution an Position 2.080(*Am*);
k. eine Arginin-, Tryptophan-, Phenylalanin-, Glycin-, Histidin-, Lysin-, Leucin-, Serin-, Threonin- oder Valin-Substitution an Position 2.088(*Am*);
l. eine Glutaminsäure-Substitution an Position 2.095(*Am*); oder
m. eine Alanin-, Histidin-, Prolin-, Serin- oder Glycin-Substitution an Position 2.098(*Am*).

9. Reissaatkorn nach einem der Ansprüche 6-8, wobei das Saatkorn ferner eine Saatgutbehandlung umfasst, welche ein ACCase-inhibierendes Herbizid umfasst.

10. Reissaatkorn nach Anspruch 9, wobei das ACCase-inhibierende Herbizid mindestens eines der folgenden umfasst: Alloxydim, Butroxydim, Clethodim, Cloproxydim, Cycloxydim, Sethoxydim, Tepraloxydim, Tralkoxydim, Chlorazifop, Clodinafop, Clofop, Diclofop, Fenoxaprop, Fenoxaprop-P, Fenthiaprop, Fluazifop, Fluazifop-P, Haloxyfop, Haloxyfop-P, Isoxapyrifop, Propaquizafop, Quizalofop, Quizalofop-P, Quizalofop-P-ethyl, Quizalofop-P-tefuryl, Trifop, Pinoxaden, oder ein landwirtschaftlich verträgliches Salz oder einen landwirtschaftlich verträglichen Ester davon.

11. Reis-Plastiden-ACCase wie in einem der Ansprüche 1-10 beschrieben, wobei die Reis-Plastiden-ACCase nicht-transgen ist.

12. Pflanze oder Saatkorn nach einem der Ansprüche 1-10, wobei die Pflanze oder das Saatkorn nicht-transgen ist.

13. Verfahren zum Anbau einer herbizidtoleranten Reispflanze, wobei das Verfahren folgendes umfasst:
a. den Anbau der Pflanze eines der Ansprüche 1-5 oder 12 oder den Anbau einer Pflanze von einem Reissaatkorn eines der Ansprüche 6-10 oder 12, indem man das Saatkorn keimen lässt, und
b. das Aufbringen eines Acetyl-Coenzym-A-Carboxylase inhibierenden Herbizids auf die Pflanze und die Umgebung derselben in einer Menge, die das Wachstum einer entsprechenden Wildtyppflanze inhibiert.

14. Verfahren nach Anspruch 13, wobei das ACCase-inhibierende Herbizid mindestens eines der folgenden umfasst: Alloxydim, Butroxydim, Clethodim, Cloproxydim, Cycloxydim, Sethoxydim, Tepraloxydim, Tralkoxydim, Chlorazifop, Clodinafop, Clofop, Diclofop, Fenoxaprop, Fenoxaprop-P, Fenthiaprop, Fluazifop, Fluazifop-P, Haloxyfop, Haloxyfop-P, Isoxapyrifop, Propaquizafop, Quizalofop, Quizalofop-P, Quizalofop-P-ethyl, Quizalofop-P-tefuryl, Trifop, Pinoxaden, oder ein landwirtschaftlich verträgliches Salz oder einen landwirtschaftlich verträglichen Ester davon.

15. Verwendung einer Pflanze eines der Ansprüche 1-5 oder eines Saatkorns eines der Ansprüche 6-10 zur Herstellung eines Nahrungsmittelprodukts, Verbraucherprodukts, Industrieprodukts oder Veterinärprodukts.

16. Isolierte Nukleinsäure umfassend eine Nukleinsäuresequenz, wie sie in einem der Ansprüche 1-12 beschrieben ist.

17. Verfahren zum Identifizieren einer Pflanze eines der Ansprüche 1-5 oder 12 oder eines Saatkorns eines der Ansprüche 6-10 oder 12, umfassend:
a. die Bereitstellung von biologischem Material einer Pflanze oder eines Saatkorns;
b. das Durchführen einer PCR- oder Hybridisierungsprüfung von ACCase-Genen in diesem biologischen Material um zu bestimmen, ob das biologische Material eine ACCase-Nukleinsäure umfasst, wie sie in einem der Ansprüche 1-12 beschrieben ist; und
c. die auf den Ergebnissen von Schritt (b) basierte Feststellung, dass die Pflanze von Schritt (a) die Nukleinsäure enthält, wie sie in einem der Ansprüchen 1-12 beschrieben ist.

## Revendications

1. Plante de riz comprenant un acide nucléique d'acétyl-coenzyme A carboxylase (ACCase) plastidique de riz mutagénisé ayant une séquence obtenue par un procédé de mutagenèse aléatoire induite et codant pour une ACCase plastidique de riz selon l'un quelconque de SEQ ID NO: 2 ou 3 ayant, en conséquence de ladite mutagenèse,
une substitution d'isoleucine en leucine à la position d'acide aminé correspondant à la position 1 781 de l'ACCase plastidique d'*Alopecurus myosuroides* (*Am*), l'ACCase plastidique de riz conférant à la plante de riz une tolérance à 100 g ai/ha de cycloxydime.

2. Plante de riz de la revendication 1, dans laquelle ladite ACCase plastidique comprend en outre : une substitution d'acide aminé à la position 1 785 ; 1 786 ; 1 811 ; 1 864 ; 2 027 ; 2 039 ; 2 041 ; 2 049 ; 2 074 ; 2 075 ; 2 088 ; 2 080 ; 2 088 ; 2 095 ; 2 096 ; ou 2 098 correspondant à la position de l'ACCase plastidique d'*Alopecurus myosuroides* (*Am*) ; une duplication de valine à la position 2 075 (*Am*) ; ou une délétion de l'acide aminé à la position 2 080 (*Am*)*.*

3. Plante de riz de la revendication 1 comprenant en outre :
a. une substitution par la glycine à la position 1 785 (*Am*) ;
b. une substitution par la proline à la position 1 786 (*Am*) ;
c. une substitution par l'asparagine à la position 1 811 (*Am*) ;
d. une substitution par la glycine à la position 2 039 (*Am*) ;
e. une substitution par l'isoleucine, la leucine ou la phénylalanine à la position 2 049 (*Am*) ;
f. une substitution par la leucine à la position 2 074 (*Am*) ;
g. une substitution par la leucine, l'isoleucine ou la méthionine à la position 2 075 (*Am*) ;
h. une duplication de la valine à la position 2 075 (*Am*) ;
i. une substitution par la thréonine ou la glycine à la position 2 078 (*Am*) ;
j. une substitution par l'acide glutamique à la position 2 080 (*Am*) ;
k. une substitution par l'arginine, le tryptophane, la phénylalanine, la glycine, l'histidine, la lysine, la leucine, la sérine, la thréonine ou la valine à la position 2 088 (*Am*) ;
l. une substitution par l'acide glutamique à la position 2 095 (*Am*) ; ou
m. une substitution par l'alanine, l'histidine, la proline, la sérine ou la glycine à la position 2 098 (*Am*).

4. Plante de riz de la revendication 1, ladite plante étant une plante de l'une quelconque des lignées : OsHPHI2, OsARWI1, OsARWI3, OsARWI8 ou OsHPHN1, un échantillon représentatif de graines de chaque lignée ayant été déposé auprès de l'American Type Culture Collection (ATCC) sous le numéro de désignation de dépôt de brevet PTA-10267, PTA-10568, PTA-10569, PTA-10570 ou PTA-10571, respectivement ; ou est un hybride, descendant ou dérivé génétiquement modifié d'une plante de l'une quelconque des lignées OsHPHI2, OsARWI1, OsARWI3, OsARWI8 ou OsHPHN1.

5. Plante de riz de la revendication 1, ladite plante étant tolérante à au moins un herbicide inhibiteur d'ACCase choisi parmi : alloxydime, butroxydime, cléthodime, cloproxydime, cycloxydime, séthoxydime, tépraloxydime, tralcoxydime, chlorazifop, clodinafop, clofop, diclofop, fénoxaprop, fénoxaprop-P, fenthiaprop, fluazifop, fluazifop-P, haloxyfop, haloxyfop-P, isoxapyrifop, propaquizafop, quizalofop, quizalofop-P, quizalofop-P-éthyl, quizalofop-P-téfuryl, trifop, pinoxadène, et des sels ou esters acceptables en agronomie de ceux-ci.

6. Graine de riz ou cellule de riz comprenant un acide nucléique d'acétyl-coenzyme A carboxylase (ACCase) mutagénisé ayant une séquence obtenue par un procédé de mutagenèse aléatoire induite et codant pour une ACCase plastidique de riz selon l'un quelconque de SEQ ID NO: 2 ou 3 ayant, en conséquence de ladite mutagenèse, une substitution d'isoleucine en leucine à une position d'acide aminé correspondant à la position 1 781 de l'ACCase plastidique d'*Alopecurus myosuroides* (*Am*), l'ACCase plastidique de riz conférant à la plante de riz une tolérance à 100 g ai/ha de cycloxydime.

7. Graine de riz ou cellule de riz de la revendication 6, ladite ACCase plastidique comprenant en outre : une substitution d'acide aminé à la position 1 785 ; 1 786 ; 1 811 ; 1 864 ; 2 027 ; 2 039 ; 2 041 ; 2 049 ; 2 074 ; 2 075 ; 2 088 ; 2 080 ; 2 088 ; 2 095 ; 2 096 ; ou 2 098 correspondant à la position de l'ACCase plastidique d'*Alopecurus myosuroides* (*Am*) ; une duplication de valine à la position 2 075 *(Am) ;* ou une délétion de l'acide aminé à la position 2 080 (*Am*).

8. Graine de riz ou cellule de riz de la revendication 6, comprenant en outre :
a. une substitution par la glycine à la position 1 785 (*Am*) ;
b. une substitution par la proline à la position 1 786 (*Am*) ;
c. une substitution par l'asparagine à la position 1 811 (*Am*) ;
d. une substitution par la glycine à la position 2 039 (*Am*) ;
e. une substitution par l'isoleucine, la leucine ou la phénylalanine à la position 2 049 (*Am*) ;
f. une substitution par la leucine à la position 2 074 (*Am*) ;
g. une substitution par la leucine, l'isoleucine ou la méthionine à la position 2 075 (*Am*) ;
h. une duplication de la valine à la position 2 075 (*Am*) ;
i. une substitution par la thréonine ou la glycine à la position 2 078 (*Am*) ;
j. une substitution par l'acide glutamique à la position 2 080 (*Am*) ;
k. une substitution par l'arginine, le tryptophane, la phénylalanine, la glycine, l'histidine, la lysine, la leucine, la sérine, la thréonine ou la valine à la position 2 088 (*Am*) ;
l. une substitution par l'acide glutamique à la position 2 095 (*Am*) ; ou
m. une substitution par l'alanine, l'histidine, la proline, la sérine ou la glycine à la position 2 098 (*Am*).

9. Graine de l'une quelconque des revendications 6 à 8, la graine comprenant en outre un traitement de graine comprenant un herbicide inhibiteur d'ACCase.

10. Graine de la revendication 9, ledit herbicide inhibiteur d'ACCase comprenant au moins l'un des : alloxydime, butroxydime, cléthodime, cloproxydime, cycloxydime, séthoxydime, tépraloxydime, tralcoxydime, chlorazifop, clodinafop, clofop, diclofop, fénoxaprop, fénoxaprop-P, fenthiaprop, fluazifop, fluazifop-P, haloxyfop, haloxyfop-P, isoxapyrifop, propaquizafop, quizalofop, quizalofop-P, quizalofop-P-éthyl, quizalofop- P-téfuryl, trifop, pinoxadène, ou un sel ou ester acceptable en agronomie de ceux-ci.

11. ACCase plastidique de riz telle que décrite dans l'une quelconque des revendications 1 à 10, ladite ACCase plastidique de riz étant non transgénique.

12. Plante ou graine de l'une quelconque des revendications 1 à 10, ladite plante ou graine étant non transgénique.

13. Procédé de culture d'une plante de riz tolérante aux herbicides, ledit procédé comprenant :
a. la culture de la plante de l'une quelconque des revendications 1 à 5 ou 12, ou la culture d'une plante à partir de la graine de riz de l'une quelconque des revendications 6 à 10 ou 12 en laissant la graine germer ; et
b. l'application sur la plante et le voisinage de celle-ci d'un herbicide inhibiteur d'acétyl-coenzyme A carboxylase en une quantité qui inhibe la croissance d'une plante de type sauvage correspondante.

14. Procédé de la revendication 13, dans lequel ledit herbicide inhibiteur d'ACCase comprend au moins l'un des : alloxydime, butroxydime, cléthodime, cloproxydime, cycloxydime, séthoxydime, tépraloxydime, tralcoxydime, chlorazifop, clodinafop, clofop, diclofop, fénoxaprop, fénoxaprop-P, fenthiaprop, fluazifop, fluazifop-P, haloxyfop, haloxyfop-P, isoxapyrifop, propaquizafop, quizalofop, quizalofop-P, quizalofop-P-éthyl, quizalofop-P-téfuryl, trifop, pinoxadène, ou un sel ou ester acceptable en agronomie de ceux-ci.

15. Utilisation d'une plante de l'une quelconque des revendications 1 à 5 ou d'une graine de l'une quelconque des revendications 6 à 10 pour produire un produit alimentaire, un produit de consommation, un produit industriel ou un produit vétérinaire.

16. Acide nucléique isolé comprenant une séquence d'acide nucléique telle que décrite dans l'une quelconque des revendications 1 à 12.

17. Procédé d'identification d'une plante de l'une quelconque des revendications 1 à 5 ou 12 ou d'une graine de l'une quelconque des revendications 6 à 10 ou 12, comprenant :
a. la fourniture d'un matériau biologique provenant d'une plante ou d'une graine ;
b. la conduite d'un essai de PCR ou d'hybridation des gènes d'ACCase dans ledit matériau biologique pour déterminer si le matériau biologique comprend un acide nucléique d'ACCase tel que décrit dans l'une quelconque des revendications 1 à 12 ; et
c. l'identification, sur la base des résultats de l'étape (b), du fait que la plante de l'étape (a) comprend l'acide nucléique tel que décrit dans l'une quelconque des revendications 1 à 12.
